# EUROPÄISCHE PATENTSCHRIFT

(11) **EP 1 436 327 B1**
(45) Veröffentlichungstag und Bekanntmachung des Hinweises auf die Patenterteilung: **03.03.2010**
(21) Anmeldenummer: 02801900.8
(22) Anmeldetag: 18.10.2002
(51) Int. Cl.: C07K 14/47, C12N 15/12, C07K 16/18, C12Q 1/68, A61K 31/7088, A01K 67/00

(54) **EE3-PROTEINFAMILIE UND ZUGRUNDELIEGENDE DNA-SEQUENZEN**
EE3-PROTEIN FAMILY AND CORRESPONDING DNA SEQUENCES
FAMILLE DE PROTEINES EE3 ET SEQUENCES D'ADN CORRESPONDANTES

(30) Priorität: 18.10.2001 DE 10151511
(43) Veröffentlichungstag der Anmeldung: 14.07.2004
(73) Patentinhaber: Sygnis Bioscience GmbH & Co. KG, 69120 Heidelberg (DE)
(72) Erfinder: SCHNEIDER, Armin, 69118 Heidelberg (DE); MAURER, Martin, 69121 Heidelberg (DE); KUSCHINSKY, Wolfgang, 69118 Heidelberg (DE); GRÜNEWALD, Sylvia, 69121 Heidelberg (DE); GASSLER, Nikolaus, 69120 Heidelberg (DE)
(74) Vertreter: Isenbruck, Günter
(86) Internationale Anmeldenummer: PCT/EP2002/011698
(87) Internationale Veröffentlichungsnummer: WO 2003/035684

(56) Entgegenhaltungen:
- WAGNER K.F. ET AL.: "Chronic inborn erythrocytosis leads to cardiac disfunction and premature death in mice overexpressing erythropoietin" RED CELLS, Bd. 97, Nr. 2, 15. Januar 2001 (2001-01-15), Seiten 536-542, XP002235701 in der Anmeldung erwähnt
- DATABASE EMBL [Online] 2. August 2001 (2001-08-02) HYSEQ INC: retrieved from EBI Database accession no. AAM23512 XP002235703
- DATABASE EMBL [Online] 10. September 2001 (2001-09-10) NIH: retrieved from EBI Database accession no. BC013793 XP002235704
- DATABASE SWALL [Online] 1. Oktober 2002 (2002-10-01) SHAW M.A. ET AL.: retrieved from EBI Database accession no. Q8NFB2 XP002235705
- DATABASE EMBL [Online] 14. Januar 2001 (2001-01-14) NIH: retrieved from EBI Database accession no. BF784459 XP002235706
- DATABASE EMBL [Online] 20. September 2001 (2001-09-20) NIH: retrieved from EBI Database accession no. BI691709 XP002235707
- DATABASE EMBL [Online] 30. August 2001 (2001-08-30) INCYTE GENOMICS INC: retrieved from EBI Database accession no. AAU69452 XP002235708
- DATABASE EMBL [Online] 30. August 2001 (2001-08-30) INCYTE GENOMICS INC: retrieved from EBI Database accession no. AAS63615 XP002235709
- DATABASE EMBL [Online] 9. August 2001 (2001-08-09) HYSEQ INC: retrieved from EBI Database accession no. AAM79063 XP002235710
- DATABASE EMBL [Online] 9. August 2001 (2001-08-09) HYSEQ INC: retrieved from EBI Database accession no. AAM80047 XP002235711
- DATABASE SWALL [Online] 1. März 2001 (2001-03-01) KAWAKAMI T. ET AL.: retrieved from EBI Database accession no. Q9HF7F4 XP002235712
- DATABASE EMBL [Online] 4. Dezember 2000 (2000-12-04) HUMAN GENOME SCI INC: retrieved from EBI Database accession no. AAB25744 XP002235713
- DATABASE EMBL [Online] 4. April 2002 (2002-04-04) INCYTE GENOMICS INC: retrieved from EBI Database accession no. ABP43503 XP002235714
- DATABASE EMBL [Online] 4. April 2002 (2002-04-04) INCYTE GENOMICS INC: retrieved from EBI Database accession no. ABN99386 XP002235715
- DATABASE EMBL [Online] 1. November 2000 (2000-11-01) NIH: retrieved from EBI Database accession no. BF167734 XP002235716
- DATABASE EMBL [Online] 28. Juni 2001 (2001-06-28) NIH: retrieved from EBI Database accession no. BI07823 XP002235717
- DATABASE EMBL [Online] 27. Juli 2000 (2000-07-27) HUMAN GENOME SCI INC: retrieved from EBI Database accession no. AAB25681 XP002235718
- DATABASE EMBL [Online] 4. Januar 2002 (2002-01-04) NIH: retrieved from EBI Database accession no. BC020408 XP002235719
- DATABASE EMBL [Online] 10. September 2001 (2001-09-10) NIH: retrieved from EBI Database accession no. BC013793 XP002235720
- DATABASE EMBL [Online] 20. September 2001 (2001-09-20) NIH: retrieved from EBI Database accession no. BI691709 XP002235721
- DATABASE EMBL [Online] 26. Oktober 1999 (1999-10-26) retrieved from EBI Database accession no. AC012363 XP002235722
- DATABASE EMBL [Online] 27. Dezember 2001 (2001-12-27) INCYTE GENOMICS INC: retrieved from EBI Database accession no. ABK28664 XP002235723
- DATABASE EMBL [Online] 27. Dezember 2001 (2001-12-27) INCYTE GENOMICS INC: retrieved from EBI Database accession no. AAU82005 XP002235724
- DATABASE EMBL [Online] 27. April 2001 (2001-04-27) WISTOW G.: retrieved from EBI Database accession no. AF353675 XP002235725
- DATABASE EMBL [Online] 8. Januar 2001 (2001-01-08) WISTOW: retrieved from EBI Database accession no. BF724625 XP002235726
- DATABASE EMBL [Online] 13. September 2001 (2001-09-13) retrieved from EBI Database accession no. BI659959 XP002235727
- DATABASE EMBL [Online] 25. Januar 1999 (1999-01-25) ZAO ET AL.: retrieved from EBI Database accession no. AQ355091 XP002235728
- DATABASE EMBL [Online] 18. November 1997 (1997-11-18) NCI: retrieved from EBI Database accession no. AA659178 XP002235729
- MAURER M.H. ET AL.: "Analysis of brain mRNA of EPO overexpressing mice" PFLUEGERS ARCHIV EUROPEAN JOURNAL OF PHYSIOLOGY, Bd. 441, Nr. 6S, März 2001 (2001-03), Seite R174 XP002235702

## Beschreibung

Die vorliegende Erfindung betrifft (i) DNA-Sequenzen, (ii) Expressionsvektoren, die erfindungsgemäße DNA-Sequenzen enthalten, (iv) Wirtszellen, die erfindungsgemäße Expressionsvektoren aufweisen, (v) Genprodukte, die durch erfindungsgemäße Sequenzen codiert werden, (vi) hinsichtlich erfindungsgemäßer Sequenzen veränderte transgene Tiere, (vii) gegen erfindungsgemäße Genprodukte gerichtete Antikörper, (viii) Verfahren zur Expression bzw. Isolierung von erfindungsgemäßen Genprodukten, (ix) die Verwendung von erfindungsgemäßen DNA-Sequenzen bzw. Genprodukten als Arzneimittel, (x) und (xi) nicht-therapeutische Verwendungen erfindungsgemäßer DNA-Sequenzen bzw. Genprodukte.

Aus dem Stand der Technik sind zahlreiche Proteine bekannt, die zur Klasse der der G-Protein-gekoppelten Rezeptoren (GPCRs) gehören. Es handelt sich dabei um die größte Familie von Oberflächenmolekülen, die in die Signaltransduktion involviert sind. Sie werden von einer großen Vielfalt an Liganden und anderen Stimuli aktiviert, z.B. Licht (Rhodopsin), Gerüchen (Odorant-Rezeptoren), Calcium, Aminosäuren oder biogenen Aminen, Nukleotiden, Peptiden, Fettsäuren- und Derivaten, und verschiedenen Polypeptiden. In Säugetieren geht man davon aus, daß ca. 1500 verschiedene Proteine der Klasse der GPCRs existieren, wobei ca. 1200 für Geruchs-, Geschmacks-, oder Vomeronasalrezeptoren kodieren. Die Gesamtzahl von "orphan" (also Rezeptoren, denen bislang keine Funktionalität zugeordnet werden konnte) GPCRs wird auf 200-500 geschätzt (Howard AD, McAllister G, Feighner SD, Liu Q, Nargund RP, Van der Ploeg LH, Patchett AA (2001) Orphan G-protein-coupled receptors and natural ligand discovery. Trends Pharmacol Sci 22:132-140.). In C. elegans machen GPCR-Sequenzen ca. 5% des Genoms aus und kodieren für ca. 1000 GPCR-Proteine (Bargmann CI (1998) Neurobiology of the Caenorhabditis elegans genome. Science 282:2028-2033 und Bargmann CI, Kaplan JM (1998) Signal transduction in the Caenorhabditis elegans nervous system. Annu Rev Neurosci 21:279-308).

Für Drosophila melanogaster liegen aus dem Stand der Technik Erkenntnisse vor, daß dort ca. 200 GPCR-Sequenzen vorhanden sind (Brody T, Cravchik A (2000) Drosophila melanogaster G protein-coupled receptors. J Cell Biol 150:83-88). Es wird davon ausgegangen, daß sich diese große Gruppe an topologisch ähnlichen Molekülen konvergent entwickelt hat, und zwar mit dem Ziel des Koppelns an G-Proteine.

Die Klasse der GPCRs wird nach dem Stand der Technik in 3 oder 4 Familien unterteilt. Familie A hat dabei die weitaus meisten Mitglieder, hierzu gehören bspw. auch die Odorant-Rezeptoren (Buck L, Axel R (1991) A novel multigene family may encode odorant receptors: a molecular basis for odor recognition. Cell 65:175-187.). Zur Familie B gehören Rezeptoren für Secretin, VIP, und Calcitonin. Familie C besteht aus Rezeptoren wie den metabotropen Glutamat-Rezeptoren, den Calcium-Rezeptoren, den GABA-B-Rezeptoren, den Geschmacksrezeptoren, und den Pheromon-Rezeptoren. Nahezu alle sog. "orphan" GPCR-Sequenzen gehören jedoch in die Familie A.

Ein Kennzeichen der GPCR-Familien ist ihre Signalweiterleitung über G-Proteine. Die Bindung eines extrazellulären Liganden induziert die Aktivierung eines G-Proteins, das dann das Signal weiterleitet. Es gibt ca. 200 verschiedene G-Proteine, und jeder Zelltyp kann eine andere Ausstattung haben. Die aktive Form eines G-Proteins ist die GTP-gebundene, im inaktiven Zustand ist das G-Protein an GDP gebunden. Das G-Protein selbst führt dabei zu seiner Inaktivierung nach GTP-Bindung, da es eine GTPase darstellt. Deswegen ist die Signalweiterleitung über G-Proteine stets ein transientes Ereignis. Jedes G-Protein besteht aus 3 Untereinheiten, alpha, beta und gamma. Dabei vermag die alpha-Untereinheit GTP- zu binden und kann daher wesentlich die nachgeschalteten Botenstoffe ("second messenger" Systeme) kontrollieren. G-Proteine Gs z.B. aktiviert (stimuliert) die Adenylat-Cyclase und führt so zu einer Erhöhung der Konzentration des intrazellulären Botenstoffes cAMP. G-Protein Gi inhibiert die Adenylat-Cyclase, und Gq aktiviert Phospholipase C ("second messenger": Inositoltriphosphat und Diacylglycerol). Andere häufig benutzte second messenger Systeme sind z.B. Calcium, K, cGMP, und andere. Es gibt auch chimäre G-Proteine. G beta und gamma Untereinheiten können ebenfalls zu einer Signalweiterleitung führen, nachdem sie sich von dem trimeren Proteinkomplex abgekoppelt haben, z.B. möglicherweise bei der Aktivierung von MAP-Kinase Signalwegen. Die Spezifität der G-Proteinkopplung eines bestimmten GPCRs stellt ein wichtiges pharmakologisches Charakteristikum dar, das u.a. zur Assayentwicklung ausgenutzt werden kann, typischerweise unter Bestimmung von Konzentrationsveränderungen der nachgeschalteten Botenstoffe, bspw. Calcium, cAMP oder Inositoltriphosphat. In jüngster Zeit haben in der Literatur mit vorläufigen Ergebnissen auch die MAP-Kinase Signalwege Beachtung gefunden, die ebenfalls GPCR-Signale weiterleiten können (Marinissen MJ, Gutkind JS (2001) G-protein-coupled receptors and signaling networks: emerging paradigms. Trends Pharmacol Sci 22:368-376.).

Schließlich ist auch eine G-Protein-unabhängige Signalweiterleitung prinzipiell möglich, so z.B. moduliert die direkte Interaktion des beta-2- adrenergen Rezeptors mit dem NHERF-Protein die Aktivität eines Na/H Austauschers (Hall RA, Premont RT, Chow CW, Blitzer JT, Pitcher JA, Claing A, Stoffel RH, Barak LS, Shenolikar S, Weinman EJ, Grinstein S, Lefkowitz RJ (1998) The beta2-adrenergic receptor interacts with the Na+/H+-exchanger regulatory factor to control Na+/H+ exchange. Nature 392:626-630).

Ein anderes Charakteristikum, das auf viele, wenn nicht alle GPCR-Rezeptoren zutrifft sind Oligomerisierungen. Besonders interessant sind hierbei Heterodimerisierungen zwischen verschiedenen GPCRs, die das pharmakologische Profil und die Ligandenspezifität verändern können (Bouvier M (2001) Oligomerization of G-protein-coupled transmitter receptors. Nat Rev Neurosci 2:274-286). So funktioniert bspw. der GABA-B-Rezeptor nur als Heterodimer zwischen GBR1 und GBR2 (Kuner R, Kohr G, Grunewald S, Eisenhardt G, Bach A, Kornau HC (1999) Role of heteromer formation in GABAB receptor function. Science 283:74-77). Eine solche Heterodimerisierung wurde inzwischen für eine ganze Reihe von GPCRs beschrieben, z.B. den mGluR5, den delta-opioid Rezeptor, und andere. In jüngster Zeit wurde am Falle der Präeklampsie belegt, dass die erhöhte Expression eines Partners in einem GPCR-Heterodimer Paar zu einer Erkrankung führen kann. Hier führt die erhöhte Expression des Bradykinin-II-Rezeptors zu einer verstärkten Bildung von BradykininII-AngiotensinII-Rezeptor Heterodimeren, deren veränderte pharmakologische Antwort den hypertonen Phänotyp erklären kann (AbdAlla, Lother,Massiery und Quitterer, Nat. Medicine (2001), 7, 1003-1009).

Schließlich handelt es sich bei den Proteinen der GPCR-Klasse um ein bevorzugtes pharmakologisches Zielmolekül. Bei den Proteinen der GPCR-Klasse greifen mehr als 25% der 100 meistverkauften Medikamente pharmakologisch an (Flower et al., 1999, Biochim. Biophys. Acta, 1422, 207-234). So sind insbesondere Agonisten und Antagonisten für folgende Rezeptorgruppen von größter pharmakologischer Bedeutung: die Gruppe der Adrenorezeptoren, der Angiotensin-II-Rezeptor, Serotoninrezeptoren, Dopaminrezeptoren, Histaminrezeptoren, Leukotrien/Prostaglandinrezeptoren. Pharmaka, die auf diese Rezeptoren einwirken, decken ein therapeutisch breites Krankheitsspektrum ab, von psychiatrischen Krankheitbildern (Schizophrenien, Depressionen), über die Beeinflussung von Bluthochdruck bis hin zu Notfallmedikamenten bei Herzstillstand. Bekannte Beispiele von gebräuchlichen Medikamenten, die auf diese Rezeptoren einwirken, sind z.B. alpha-Adrenozeptoragonisten (Norfenefrin), Beta-Adrenozeptoragonisten (Isoprenalin, Fenoterol), Alpha-Adrenozeptorblocker (Prazosin), Beta-Adrenozeptorblocker (Propanolol), 5-HT-Antagonisten (Cyproheptadin), H2-Rezeptorenblocker (Cimetidin), H1-Rezeptorenblocker (Terfenadin), Dopaminagonisten (Bromocriptin) und andere.

Trotz intensiver Forschungsbemühungen sind jedoch die Signaltransduktionswege, die durch die Rezeptoren beeinflußt werden noch unzureichend aufgeklärt. Darüber hinaus fehlt es an einem vertieften Verständnis des komplexen Netzwerkes gegenseitiger Beeinflussung der verschiedenen GPCR-Systeme und deren Wirkung auf die nachgeschalteten intrazellulären Prozesse, insbesondere auch in Hinblick auf externe physiologische Zustände.

Aufgabe der vorliegenden Erfindung ist es, weitere Mitglieder der Klasse der GPCR-Proteine und deren zugrundeliegende Nukleotidsequenzen zu identifizieren. auf der Basis identifizierter Proteine Verfahren ist es möglich zur Verfügung zu stellen, die es erlauben, therapeutische wirksame Substanzen zu entwickeln, die in eine Pathophysiologie, die bspw. durch Fehlsteuerung der Expression und/oder Expression infunktioneller Varianten bedingt ist, aber auch bei physiologischer Expression auftreten kann, therapeutisch eingreifen können.

Die vorliegende Erfindung löst diese Aufgaben durch die Gegenstände der Ansprüche 1 bis 16. Insbesondere sind alle einschließlich der jeweils im Doppelstrang komplementären Sequenzen offenbarungsgemäß mit umfasst.

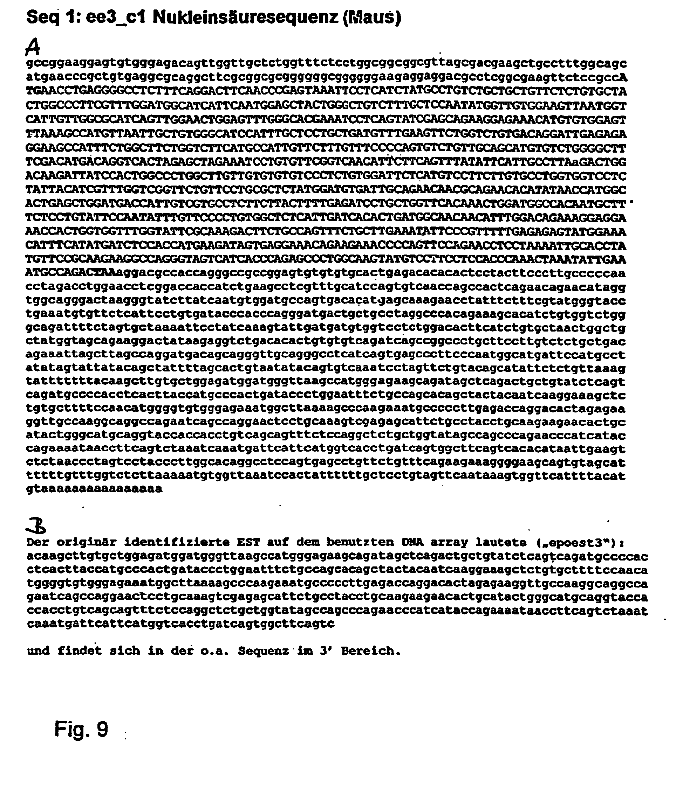

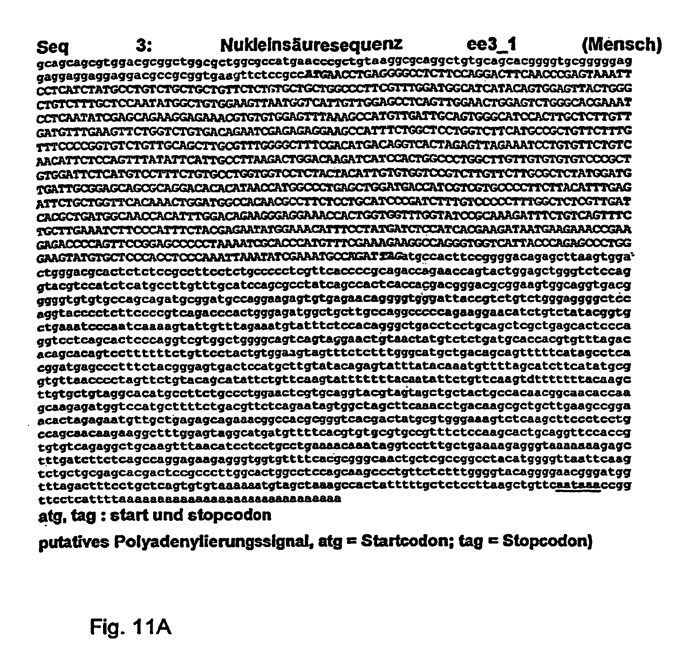

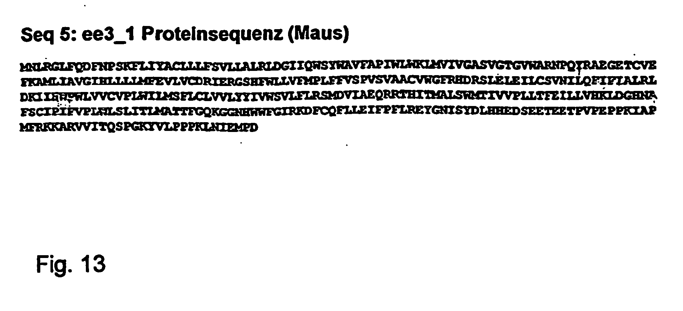

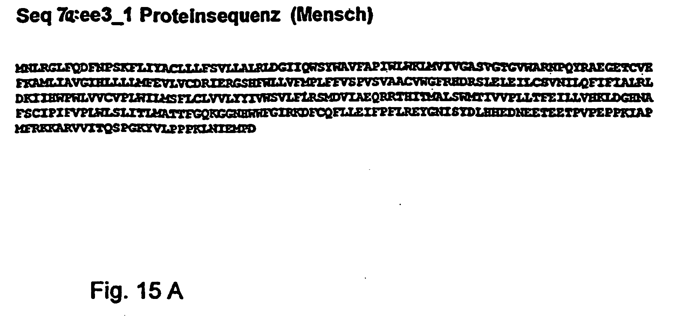

In einer weiteren bevorzugten Ausführungsform werden DNA-Sequenzen offenbart, deren Genprodukt für ein Polypeptid codiert, wie in einer der Figuren 13, oder 15A, für die Sequenzen mit den Nummern 5, bzw. 7A, wiedergegeben ausgenommen eine Nukleinsäure gemäß SEQ ID No:75 aus der WO 01/98353. Auch Sequenzen des komplementären DNA-Stranges sind mitoffenbart. Weiterhin bevorzugt sind Nukleinsäure-Sequenzen, insbesondere DNA-Sequenzen, die für ein Protein codieren, mit den Sequenzen gemäß vorliegender Numerieung 5 und 7 ausgenommen eine Nukleinsäure gemäß SEQ ID No. 75 aus der WO 01/98353 . Nach Isolierung und Sequenzierung sind die erfindungsgemäßen Nukleotidsequenzen, bspw. gemäß Figuren 9 oder 11A oder deren funktionelle oder infunktionelle Äquivalente, wie z.B. Allelvarianten oder Isoformen, erhältlich. Unter Allelvarianten werden im Sinne der vorliegenden Erfindung Varianten verstanden, die , bevorzugt 90 bis 100 % Homologie auf Aminosäure-ebene aufweisen. Allelvarianten umfassen insbesondere solche funktionellen Varianten, die durch Deletion, Insertion oder Substitution von Nukleotiden aus nativen ee3-Sequenzen, bspw. aus gemäß Figuren 9 oder 11A, dargestellten Sequenzen erhältlich sind, wobei wenigstens noch eine der wesentlichen biologischen Eigenschaften erhalten bleibt.

Homologe oder sequenzverwandte DNA-Sequenzen können aus allen Säugerspezies oder anderen Spezies, einschließlich Mensch, nach gängigen Verfahren durch Homologie-Screening durch Hybridisierung mit einer Probe der erfindungsgemäßen Nukleinsäuresequenzen oder Teilen davon isoliert werden. Unter funktionellen Äquivalenten sind auch Homologe der nativen ee3-Sequenzen, bspw. der in den Figuren 9, oder 11A, dargestellten Sequenzen, beispielsweise ihre Homologen aus anderen Mammalia, verkürzte Sequenzen, Einzelstrang-DNA oder RNA der codierenden und nicht-codierenden DNA-Sequenz ausgenommen eine Nukleinsäure gemäß SEQ ID No. 75 aus der WO 01/98353 zu verstehen. Solche funktionellen Äquivalente lassen sich bspw. ausgehend von den in den Figuren 9, oder 11A, dargestellten DNA-Sequenzen oder Teilen dieser Sequenzen, beispielsweise mit üblichen Hybridisierungsverfahren oder der PCR-Technik aus anderen Vertebraten wie Mammalia, isolieren.

Ein erfindungsgemäßes Nukleinsäurekonstrukt oder eine erfindungsgemäße Nukleinsäure kann, auch in therapeutisch oder diagnostisch geeigneter Form exprimiert werden Zur Generierung des rekombinanten Proteins können Vektorsysteme oder Oligonukleotide verwendet werden, die die Nukleinsäuren oder das Nukleinsäurekonstrukt um bestimmte Nukleotidsequenzen verlängern und damit für veränderte Polypeptide kodieren, die bspw. einer einfacheren Reinigung dienen, insbesondere wird hierbei auch auf die Verlängerung durch die oben beschriebenen Tag-Sequenzen verwiesen.

Bevorzugt sind weiterhin DNA-Sequenzen, die (c) DNA-Sequenzen erfindungsgemäßer genömischer DNA-Sequenzen enthalten oder diesen entsprechen.

Weiterhin werden erfindungsgemäß vorzugsweise alle DNA-Sequenzen mitoffenbart, die für ein Protein codieren, das im wesentlichen der Aminosäuresequenz der erfindungsgemäßen Proteine mit den Sequenznummern 5, bzw. 7A, entspricht ausgenommen eine Nukleinsäure gemäß SEQ ID No: 75 der WO 01/98353. Diese DNA-Sequenzen erhalten nur eine geringe Zahl an Veränderungen gegenüber der in den vorgenannten Figuren angegebenen Sequenzen, bspw. kann es sich um Isoformen handeln. Die Zahl der Sequenzveränderungen wird typischerweise nicht größer als 10 sein. Derartige im wesentlichen mit den für die Proteine mit den Sequenznummern 5, bzw. 7A, codierenden DNA-Sequenzen entsprechenden DNA-Sequenzen, die gleichfalls für ein biologisch aktives Protein codieren, können durch allgemein bekannte Mutagenese-Verfahren erhalten und die biologische Aktivität der durch die Mutanten codierten Proteine durch Screening-Verfahren, bspw. Bindungsstudien oder die Fähigkeit zur Ausprägung der biologischen Funktion, bspw. im Zusammenhang mit neuronalen Vorgängen oder der Apoptose, identifiziert werden. Zu den entsprechenden Mutagenese-Verfahren gehören die "site-directed"-Mutagenese, die die automatisch durchgeführte Synthese eines Primers mit mindestens einer Basenveränderung vorsieht. Nach der Polymersierungsreaktion wird der Heteroduplex-Vektor in einen geeignetes Zellsystem transferiert (z.B. E.coli) und entsprechend transformierte Klone isoliert.

Die Funktionalität erfindungsgemäßer Sequenzen steht u.a. in unmittelbarem Zusammenhang mit der Identifizierung weiter distal liegender Elemente der von erfindungsgemäßen Proteinen ausgelösten Signalkaskade. Hierbei wurde festgestellt, daß MAP-Kinasen durch erfindungsgemäße Rezeptoren stimuliert werden. Neben der Verwendung entsprechender Reporterassays (s. Ausführungsbeispiel) zur Identifizierung der MAP-Kinasen können alternativ für diese Zwecke auch vorgefertigte Kits (z.B. Mercury in vivo kinase assay kits, Fa. Clontech) benutzt werden. Dabei wird der Tet-Repressor in Fusion mit der Transaktivatordomäne eines Phosphorylierungstargets (Transkriptionsfaktoren, z.B. Jun) exprimiert. Die Aktivierung eines Luciferasekonstruktes unter Kontrolle eines Tet-Repressor-Elements findet nur statt, wenn eine spezifische Phosphorylierung der Transaktivatordomäne durch eine Kinase erfolgt. Auf diese Weise ist die Einordnung der Aktivität eines erfindungsgemäßen Rezeptors der ee3-Familie oder einer erfindungsgemäßen Variante in einen zellulären Signaltransduktionsweg möglich.

Die Identifizierung erfindungsgemäßer Sequenzen beruht unter anderem auch auf der funktionellen Erkenntnis, daß die Hochregulation von erfindungsgemäßem murinen ee3_1_m in einem Tiermodell mit erhöhter EPO-Expression eine pathophysiologische Beteiligung dieses Rezeptors an Vorgängen, die das Überleben oder die Adaptation von Zellen an diesen Zustand beeinflussen, indiziert. Deshalb sind erfindungsgemäße Rezeptoren von besonderer pharmakologischer Bedeutung für Krankheiten die mit verminderter Sauerstoffversorgung einhergehen, insbesondere verminderter cerebraler Sauerstoffversorgung einhergehen.

Darüber hinaus kommen alle dem Fachmann geläufigen Methoden für die Herstellung, Modifikation und/oder Detektion von erfindungsgemäßen DNA-Sequenzen, die in vivo, in situ oder in vitro ausgeführt werden können in Betracht (PCR (Innis et al. PCR Protocols: A Guide to Methods and Applications) oder chemische Synthese). Durch entsprechende PCR-Primer können bspw. neue Funktionen in eine erfindungsgemäße DNA-Sequenz eingeführt werden, wie z.B. Restriktionsschnittstellen, Terminationscodons. Hierdurch können erfindungsgemäße Sequenzen für den Transfer in Klonierungsvektoren entsprechend entworfen werden.

Ein weiterer Gegenstand der vorliegenden Erfindung betrifft Expressionsvektoren oder ein rekombinantes Nukleinsäurekonstrukt, das eine, wie oben beschrieben, erfindungsgemäße Nukleinsäuresequenz, typischerweise eine DNA-Sequenz enthält. Vorteilhafterweise werden hierbei die erfindungsgemäßen Nukleinsäuresequenzen mit mindestens einem genetischen Regulationselement, wie bspw. Transkriptions- und Translationssignalen, funktionell verknüpft. Diese Verknüpfung kann je nach gewünschter Anwendung zu einer nativen Expressionsrate oder auch zu einer Erhöhung bzw. Erniedrigung der nativen Genexpression führen. Mit den solchermaßen hergestellten Expressionsvektoren können anschließend Wirtsorganismen bzw. Wirtszellen transformiert werden, z.B. Zellkulturen aus Säugetierzellen.

Bei dem erfindungsgemäßen Expressionsvektor wird (werden) typischerweise das (die) native(n) Regulationselement(e) eingesetzt werden, d.h. die bspw. Promotor und/oder Enhancer-Region des Gens für ein erfindungsgemäßes Protein aus der ee3-Familie, insbesondere für ein Protein mit der Sequenznummer 5, bzw 7A, bspw. aus Säugetieren, insbesondere entsprechende humane Regulationssequenzen. Ggf. können diese nativen oben bezeichneten Regulationssequenzen auch genetisch verändert sein, um eine veränderte Expressionsintensität hervorzurufen. Zusätzlich zu diesen nativen vorbezeichneten Regulationssequenzen oder anstelle dieser nativen Regulationssequenzen können für andere Gene native Regulationselemente erfindungsgemäßen DNA-Sequenzen vor- und/oder nachgeschaltet (5'- oder 3'-Regulationssequenzen) sein und gegebenenfalls auch genetisch verändert worden sein, so daß die natürliche Regulation unter der Kontrolle der vorbezeichneten nativen Regulationssequenzen ausgeschaltet ist und die Expression der Gene - je nach Wunsch - hierdurch erhöht oder erniedrigt werden kann.

Vorteilhafte Regulationssequenzen für das erfindungsgemäße Verfahren sind beispielsweise in Promotoren wie cos-, tac-, trp-, tet-, trp-tet-, lpp-, lac-, lpp-lac-, lacIq-, T7-, T5- , T3-, gal-, trc-, ara-, SP6-, l-PR- oder im l-PL-Promotor enthalten, die vorteilhafterweise in gram-negativen Bakterien Anwendung finden. Weitere vorteilhafte Regulationssequenzen sind beispielsweise in den gram-positiven Promotoren wie amy und SPO2, in den Hefepromotoren wie ADC1, MFa, AC, P-60, CYC1, GAPDH oder in Mammaliapromotoren wie CaM-KinaseII, CMV, Nestin, L7, BDNF, NF, MBP, NSE, beta-Globin, GFAP, GAP43, Tyrosin Hydroxylase, Kainat-Rezeptor-Untereinheit 1, Glutamat-Rezeptor-Untereinheit B enthalten. Prinzipiell können alle natürlichen Promotoren mit ihren Regulationssequenzen wie die bspw. oben genannten für einen erfindungsgemäße Expressionsvektor verwendet werden.

Darüber hinaus können auch synthetische Promotoren vorteilhaft verwendet werden. Diese regulatorischen Sequenzen sollen die gezielte Expression der erfindungsgemäßen Nukleinsäuresequenzen ermöglichen. Dies kann beispielsweise je nach Wirtsorganismus bedeuten, daß das Gen erst nach Induktion exprimiert oder überexprimiert wird, oder daß es sofort exprimiert und/oder überexprimiert wird. Die regulatorischen Sequenzen bzw. Faktoren können dabei vorzugsweise die Expression positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Als Regulationssequenzen werden alle dem Fachmann geläufigen Elemente bezeichnet, die auf der Transkriptions- und/oder Translationsebene die Expression der erfindungsgemäßen Sequenzen beeinflussen können. Insbesondere sind dabei neben Promotorsequenzen sog. "Enhancer"-Sequenzen hervorzuheben, die über eine verbesserte Wechselwirkung zwischen RNA-Polymerase und DNA eine erhöhte Expression bewirken können. Als weitere Regulationssequenzen seien beispielhaft die sog. "Locus Control Regions", "Silencer" oder jeweilige Teilsequenzen davon genannt. Diese Sequenzen können vorteilhaft für eine gewebespezifische Expression verwendet werden. Auch sog. Terminatorsequenzen werden vorteilhafterweise in einem erfindungsgemäßen Expressionsvektor vorhanden sein und erfindungsgemäß unter den Terminus "Regulationssequenz" subsumiert.

Eine bevorzugte Ausführungsform der vorliegenden Erfindung ist die Verknüpfung der erfindungsgemäßen Nukleinsäuresequenz mit einem Promotor, wobei der Promotor typisch 5' "upstream" von einer erfindungsgemäßen DNA-Sequenz zu liegen kommt. Weitere Regulationssignale, wie bspw. 3'-gelegene Terminatoren, Polyadenylierungssignale oder Enhancer, können funktionell in dem Expressionsvektor enthalten sein. Darüber hinaus können erfindungsgemäße Nukleinsäuresequenzen, für die entsprechenden Proteine, in einer oder mehreren Kopien in einem Genkonstrukt nach dieser Erfindung enthalten sein, oder ggf. auch auf getrennten Genkonstrukten lokalisiert sein.

Unter den Begriff "Expressionsvektor" fallen sowohl rekombinante Nukleinsäurekonstrukte bzw. Genkonstrukte, wie zuvor beschrieben, als auch komplette Vektorkonstrukte, die neben erfindungsgemäßen DNA-Sequenzen und etwaigen Regulationssequenzen typischerweise auch weitere Elemente enthalten. Diese Vektorkonstrukte oder Vektoren werden zur Expression in einem geeigneten Wirtsorganismus verwendet. Vorteilhafterweise wird mindestens eine erfindungsgemäße DNA-Sequenz, bspw. humanes Gen aus der ee3-Familie, insbesondere ee3_1 oder ee3_2, oder bspw. eine Teilsequenz eines solchen Gens, in einen wirtsspezifischen Vektor insertiert, der eine optimale Expression der Gene im ausgesuchten Wirt ermöglicht. Vektoren sind dem Fachmann wohl bekannt und können beispielsweise aus "Cloning Vectors" (Eds. Pouwels P. H. et al. Elsevier, Amsterdam-New York-Oxford, 1985, ISBN 0 444 904018) entnommen werden. Unter Vektoren sind außer Plasmiden auch alle anderen dem Fachmann bekannten Vektoren wie beispielsweise Phagen, Viren wie SV40, CMV, Baculovirus, A-denovirus, Sindbisvirus, Transposons, IS-Elemente, Phasmide, Phagemide, Cosmide, lineare oder zirkuläre DNA zu verstehen. Diese Vektoren können autonom im Wirtsorganismus repliziert oder chromosomal repliziert werden. Für die Integration in Mammalia wird typischerweise lineare DNA verwendet.

Die Expression erfindungsgemäßer Nukleinsäuresequenzen kann vorteilhaft durch Erhöhen der Genkopienzahl und/oder durch Verstärkung regulatorischer Faktoren, die die Genexpression positiv beeinflussen, erhöht werden. So kann eine Verstärkung regulatorischer Elemente vorzugsweise auf der Transkriptionsebene erfolgen, indem stärkere Transkriptionssignale, wie Promotoren und Enhancer, verwendet werden. Daneben ist aber auch eine Verstärkung der Translation möglich, indem beispielsweise die Stabilität der mRNA verbessert oder die Ableseeffizienz dieser mRNA an den Ribosomen erhöht wird. Zur Erhöhung der Genkopienzahl können die Nukleinsäuresequenzen oder homologe Gene, beispielsweise in ein Nukleinsäurefragment bzw. in einen Vektor eingebaut werden, der vorzugsweise die den jeweiligen Genen zugeordnete, regulatorische Gensequenzen oder analog wirkende Promotoraktivität enthält. Insbesondere werden solche regulatorische Sequenzen verwendet, die die Genexpression verstärken.

Erfindungsgemäße Nukleinsäuresequenzen können zusammen mit den für interagierende oder für potentiell interagierende Proteine kodierenden Sequenzen in einen einzelnen Vektor kloniert werden und anschließend in vitro in einer Wirtszelle oder in vivo in einem Wirtsorganismus exprimiert werden. Alternativ kann auch jede der potentiell interagierenden Nukleinsäuresequenzen und die erfindungsgemäßen kodierenden Sequenzen aus der ee3-Familie in je einen einzelnen Vektor gebracht und diese getrennt in den jeweiligen Organismus über übliche Methoden, wie bspw. Transformation, Transfektion, Transduktion, Elektroporation oder Partikel-Gun verbracht werden.

In einer weiteren vorteilhaften Ausführungsform kann mindestens ein Marker-Gen (bspw. Antibiotika-Resistenz-Gene und/oder Gene, die für ein fluoreszierendes Protein kodieren, insbesondere GFP) in einem erfindungsgemäßen Expressionsvektor, insbesondere einem kompletten Vektorkonstrukt, enthalten sein.

Ein weiterer Gegenstand der vorliegenden Erfindung betrifft Wirtszellen, die mit einer erfindungsgemäßen DNA-Sequenz und/oder einem erfindungsgemäßen Expressionsvektor, insbesondere einem Vektorkonstrukt transformiert sind. Als Wirtszellen sind prinzipiell alle Zellen geeignet, die eine Expression erfindungsgemäßer DNA-Sequenzen (wodurch als Derivate bspw. auch ihre Allele oder funktionelle Äquivalente eingeschlossen sind) allein oder im Verbund mit weiteren Sequenzen, insbesondere Regulationssequenzen, gestatten. Als Wirtszellen kommen alle Zellen pro- oder eukaryontischer Natur in Betracht, beispielsweise Bakterien, Pilze, Hefen, pflanzliche oder tierische Zellen. Bevorzugte Wirtszellen sind Bakterien, wie Escherichia coli, Streptomyces, Bacillus oder Pseudomonas, eukaryotische Mikroorganismen, wie Aspergillus oder Saccharomyces cerevisiae oderr die gewöhnliche Bäckerhefe (Stinchcomb et al., Nature, 282:39, (1997)). Insbesondere um größere Mengen an erfindungsgemäßen Proteinen herstellen zu können, eignen sich vorteilhafterweise methylotrophe Hefen, insbesondere Pichia pastoris. Die Rezeptoren werden dazu in geeignete Expressionsvektoren kloniert, die z.B. auch die Expression als Fusionsprotein mit zur Reinigung geeigneten "Tag"-Sequenzen erlauben. Nach Elektroporation der Hefen werden schließlich stabile Klone selektiert. Eine gute Beschreibung der Methode sowie alle dafür nötigen Mittel werden von der Firma Invitrogen angeboten. Hernach können die Expressionsprodukte funktionell charakterisiert und ggf. für erfindungsgemäße "Screening"-Verfahren eingesetzt werden.

In einer bevorzugten Ausführungsform werden jedoch zur Expression von erfindungsgemäßen DNA-Sequenzen Zellen aus multizellulären Organismen gewählt. Dies geschieht auch vor dem Hintergrund einer möglicherweise erforderlichen Glykosylierung (N- und/oder O-gekoppelt) der codierten Proteine. Diese Funktion kann in höheren Eukaryotenzellen - im Vergleich zu Prokaryotenzellen - in geeigneter Weise ausgeführt werden. Im Prinzip ist jede höhere eukaryotische Zellkultur als Wirtszelle verfügbar, wenn auch Zellen von Säugern, beispielsweise Affen, Ratten, Hamstern oder Menschen, ganz besonders bevorzugt sind. Dem Fachmann ist eine Vielzahl von etablierten Zellinien bekannt. In einer keineswegs abschließenden Aufzählung werden die folgenden Zellinien genannt: 293T (Embryonennierenzellinie), (Graham et al., J. Gen. Virol., 36:59 (1997)), BHK (Babyhamsternierenzellen), CHO (Zellen aus den Hamsterovarien), (Urlaub und Chasin, P. N. A. S. (USA) 77:4216, (1980)), HeLa (humane Cervixkarzinomzellen) und weitere - insbesondere für den Laboreinsatz etablierte - Zellinien, wie bspw. CHO-, HeLa-, HEK293-, Sf9- oder COS-Zellen. Ganz besonders bevorzugt sind humane Zellen, insbesondere Zellen des Immunsystems oder adulte Stammzellen, bspw. Stammzellen des Blut bildenden Systems (aus dem Knochenmark). Humane erfindungsgemäße transformierte Zellen, insbesondere autologe Zellen des Patienten, eignen sich nach (vor allem ex vivo) Transformation mit erfindungsgemäßen DNA-Sequenzen oder erfindungsgemäßen Expressionsvektoren, ganz besonders als Arzneimittel für bspw. gentherapeutische Zwecke, also nach Durchführung einer Zellentnahme, ggf. ex vivo Expansion, Transformation, Selektion und abschließender Retransplantation.

Die erfindungsgemäße Proteine der ee3-Familie können heterolog in Insektenzellen zur funktionellen Charakterisierung und zum Einsatz für "Screening"-Verfahren hergestellt werden. Da die Konzentration endogener G Proteine in Insektenzellen relativ niedrig ist, so sind z.B. Gi Proteine im "Western Blot" nicht nachzuweisen, und Insektenzellen den zu untersuchenden Rezeptor in der Regel nicht exprimieren, sind sie zur in vivo Rekonstitution von Signaltransduktionswegen erfindungsgemäßer Rezeptoren der ee3-Familie besonders geeignet. Die Rezeptoren der ee3-Familie werden in diesem Fall mittels des Baculovirus-Expressionssystems in verschiedenen Insektenzellinien, z.B. Sf9, Sf21, Tn 368 oder Tn High Five, oder MB-Zellen exprimiert. Dazu werden z.B. mit dem Baculo-Gold Kit von Pharmingen rekombinante Baculoviren hergestellt und die obengenannten Insektenzellinien infiziert. Um erfindungsgemäß die Kopplung an G Proteine zu untersuchen, werden Ko-Infektionen durchgeführt. Dazu werden die Zellen mit dem Rezeptorvirus und zusätzlich noch mit den die drei G Protein Untereinheiten exprimierenden Viren infiziert und entsprechende Assays, z.B. cAMP-Assays durchgeführt. So kann der Einfluß verschiedener G Proteinuntereinheiten auf die Aktivität des Rezeptors untersucht werden. Insektenzellen die Rezeptoren exprimieren oder deren Membranen können ebenfalls in Screening-Assays eingesetzt werden. Insektenzellen können leicht in großen Mengen sowohl in Fermentern als auch in Schüttelkolben vermehrt werden und sind damit geeignetes Ausgangsmaterial, um rekombinantes Zell- oder Membranmaterial sowohl für "Screening"-Verfahren als auch für Rezeptorreinigungen bereitzustellen.

Die Kombination aus einer Wirtszelle und einem zu den Wirtszellen passenden erfindungsgemäßen Expressionsvektor, wie Plasmide, Viren oder Phagen, wie beispielsweise Plasmide mit dem RNA-Polymerase/Promoter System, die Phagen 1, Mu oder andere temperänte Phagen oder Transposons, und/oder weiteren vorteilhaften regulatorischen Sequenzen, bilden eine erfindungsgemäße Wirtszelle, die als Expressionssystem dienen kann. Bevorzugte erfindungsgemäße Expressionssysteme auf der Basis erfindungsgemäßer Wirtszellen sind beispielsweise die Kombination aus Säugetierzellen, wie bspw. CHO-Zellen, und Vektoren, wie bspw. pcDNA3neo-Vektor, oder bspw. HEK293-Zellen und CMV-Vektor, die für Säugetierzellen besonders geeignet sind.

Ein weiterer Aspekt der vorliegenden Erfindung sind die Genprodukte der erfindungsgemäßen DNA-Sequenzen. Unter Genprodukten versteht man im Sinne dieser Erfindung sowohl Primärtranskripte, also RNA, vorzugsweise mRNA, als auch Proteine bzw. Polypeptide, insbesondere in aufgereinigter Form. Diese Proteine regulieren oder transportieren insbesondere apoptotische oder nekrotische, ggf. auch inflammatorische Signale oder Signale, die das Zellwachstum oder die Zellplastizität betreffen. Ein aufgereinigtes Genprodukt kann ein funktionshomologes oder die Funktion inhibierendes (infunktionelles) Allel, Fragment, Analoges oder Derivat dieser Sequenz enthalten oder aus einer solchen Aminosäuresequenz bestehen. Funktionshomologie wird im Sinne der vorliegenden Erfindung so definiert, daß mindestens noch eine der wesentlichen funktionellen Eigenschaften der gemäß Figuren 13 oder 15a dargestellten Proteine erhalten bleibt.

Unter einem Derivat werden dabei insbesondere solche AS-Sequenzen verstanden, die durch Modifikationen ihrer Seitenketten verändert sind. Bspw. durch Konjugation eines Antikörpers, Enzyms oder Rezeptors an eine erfindungsgemäße AS-Sequenz. Derivate können aber auch die Kopplung eines Zuckers (über eine N- oder O-glykosidische Bindung) oder Fett(säure)-restes (bspw. Myristylsäure), einer oder auch mehrerer Phosphatgruppe(n) und/oder jeder-beliebigen Modifikation einer Seitenkette, insbesondere einer freien OH-Gruppe oder NH2-Gruppe oder am N- oder C-Terminus eines erfindungsgemäßen Oligo- oder Polypeptids. Darüber hinaus schließt der Begriff "Derivat" auch Fusionsproteine ein, bei denen also eine erfindungsgemäße Aminosäuresequenz an beliebige Oligo- oder Polypeptide gekoppelt ist.

Als "Analoge" werden Sequenzen bezeichnet, die sich durch mindestens eine AS-Veränderung gegenüber der nativen Sequenz auszeichnen (Insertion, Substitution). Im Rahmen der vorliegenden Erfindung sind solche konservativen Substitutionen bevorzugt, bei denen der physikochemische Charakter (Raumerfüllung, Basizität, Hydrophobizität etc.) der ausgetauschten AS erhalten bleibt (polare AS, lange aliphatische Kette, kurze aliphatische Kette, negativ oder positiv geladene AS, AS mit aromatischer Gruppe). Die Substitutionen können biologisch funktionelle, tw. Funktionelle oder biologisch infunktionelle Sequenzen ergeben. Beispielsweise können Argininreste gegen Lysinreste, Valinreste gegen Isoleucinreste oder Asparaginsäurereste gegen Glutaminsäurereste ausgetauscht werden. Es können aber auch ein oder mehrere Aminosäuren in ihrer Reihenfolge vertauscht, hinzugefügt oder entfernt werden, oder es können mehrere dieser Maßnahmen miteinander kombiniert werden. Die solchermaßen gegenüber den nativen ee3-Proteinen, gemäß Figuren 13, oder 15A, veränderten Proteine besitzen typischerweise weinigstens 90% Sequenzidentität zu den Sequenzen in den vorgenannten Figuren, berechnet nach dem Algorithmus von Altschul et al. (J. Mol. Biol., 215, 403-410, 1990). Das isolierte Protein und seine funktionellen Varianten lassen sich vorteilhafterweise aus dem Gehirn von Mammalia wie Homo sapiens, Rattus norvegicus oder Mus musculus isolieren. Auch Homologe aus anderen Mammalia sind unter funktionellen Varianten zu verstehen.

Bevorzugt sind erfindungsgemäß Analoge dann, wenn die Sekundärstruktur, wie sie in der nativen Sequenz auftritt, auch bei ihnen erhalten bleibt. Neben konservative Substitutionen können auch weniger konservative AS-Variationen erfindungsgemäß in die native Sequenz eingeführt werden. Dabei behalten sie typischerweise ihre biologische Funktion, insbesondere als Transduktor eines apoptotischen oder nekrotischen Signals oder eines Signals für die Zellproliferation, Zellplastizität oder das Zellwachstum, bei. Der Effekt einer Substitution oder Deletion kann ohne weiteres durch entsprechende Untersuchungen, Bindungsassays oder bspw. zytotoxische Tests, überprüft werden.

Zu den erfindungsgemäßen Genprodukten (Proteinen) gehören aber auch all jene Genprodukte (Proteine), die sich erfindungsgemäß von DNA-Derivaten, DNA-Fragmenten oder DNA-Allelen der in den Figuren angegebenen DNA-Sequenzen nach Transkription und Translation ableiten.

Darüber hinaus können die erfindungsgemäßen Proteine chemisch modifiziert sein. So etwa kann eine Schutzgruppe am N-Terminus vorliegen. Es können Glykosylgruppen an Hydroxyl-oder Aminogruppen angefügt sein, Lipide können kovalent mit dem erfindungsgemäßen Protein verbunden sein, ebenso Phosphate oder Acetylgruppen und ähnliches. Auch beliebige chemische Substanzen, Verbindungen oder Gruppen können auf einem beliebigen Syntheseweg an das erfindungsgemäße Protein gebunden sein. Auch zusätzliche Aminosäuren, z.B. in Form einzelner Aminosäuren oder in Form von Peptiden oder in Form von Proteindomänen und ähnliches, können mit dem N- und/oder C-Terminus eines erfindungsgemäßen Proteins.

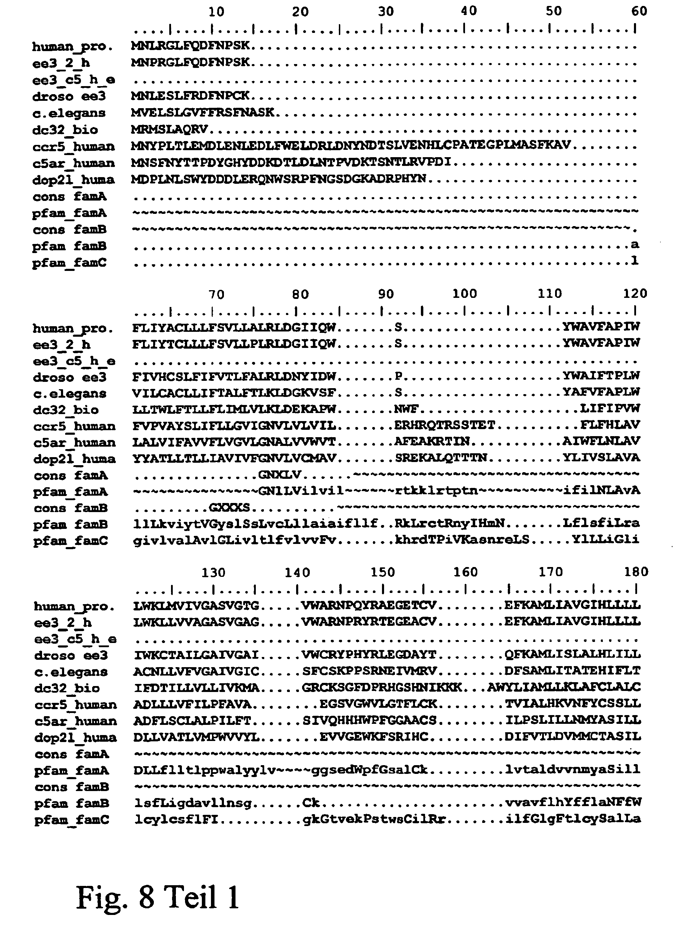

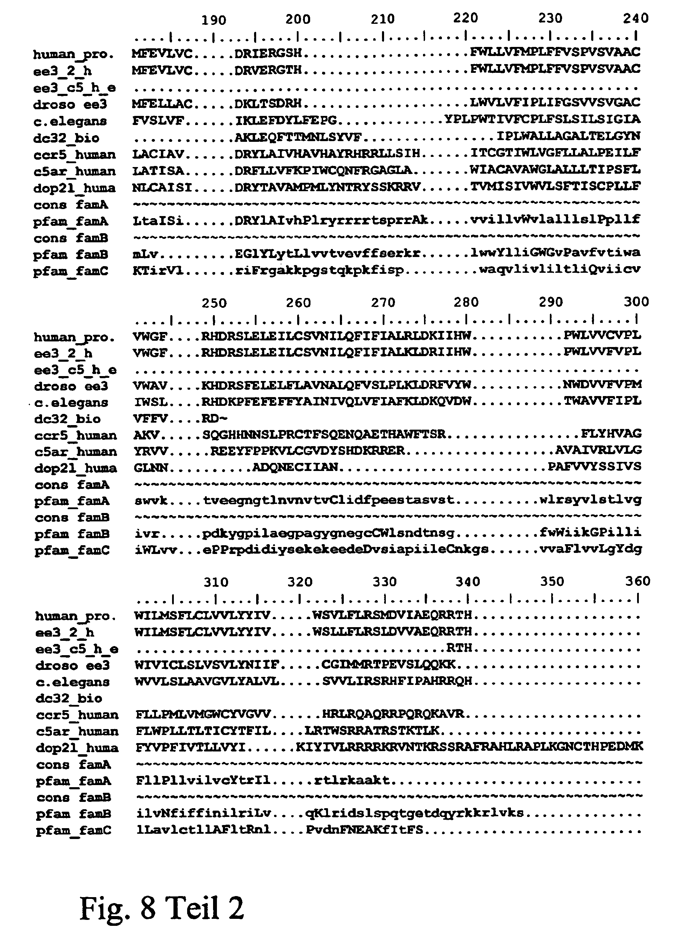

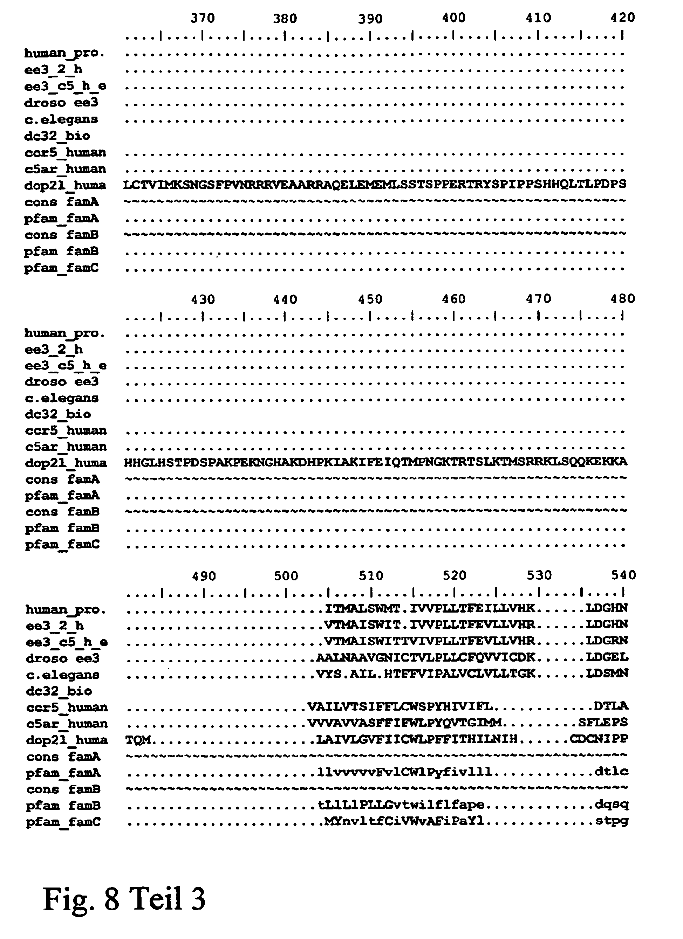

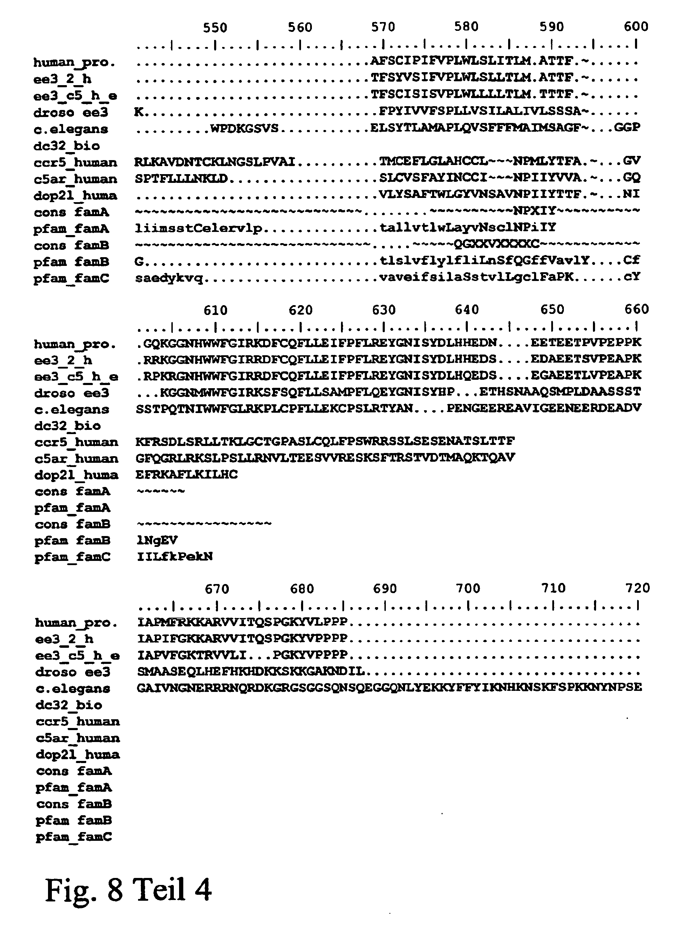

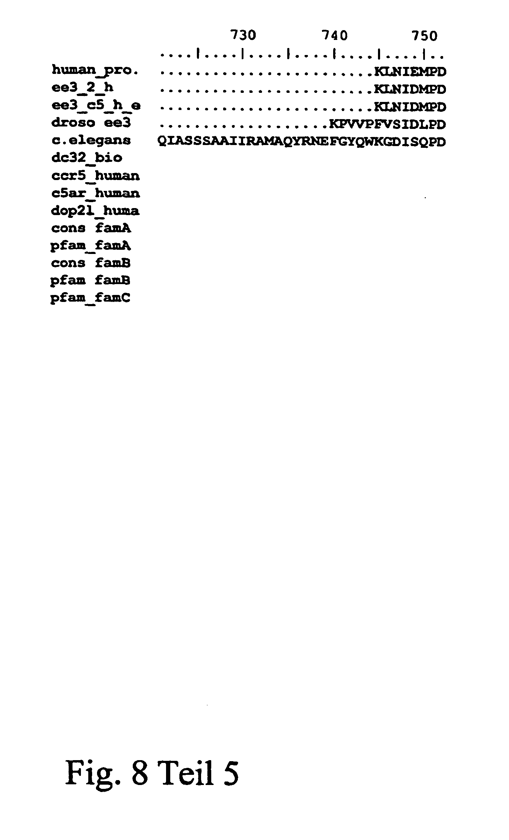

Insbesondere sind hier sogenannte Signal- oder "Leader"-Sequenzen am N-Terminus der Aminosäuresequenz eines erfindungsgemäßen Proteins bevorzugt, die das Peptid cotranslational oder posttranslational in eine bestimmte Zellorganelle oder in den extrazellulären Raum (bzw. das Kulturmedium) führen. Am N- oder am C-Terminus können auch Aminosäuresequenzen vorliegen, die als Antigen die Bindung der erfindungsgemäßen Aminosäuresequenz an Antikörper erlauben. Zu nennen ist hier insbesondere das Flag-Peptid, dessen Sequenz im Einbuchstabencode der Aminosäuren lautet: DYKDDDDK. Oder auch ein His-Tag mit mindestens 3, vorzugsweise mindestens 6 Histidin-Resten. Diese Sequenzen haben stark antigene Eigenschaften und erlaubt somit eine schnelle Überprüfung und leichte Reinigung des rekombinanten Proteins. Monoklonale Antikörper, die das Flag-Peptid binden, sind von der Firma Eastman Kodak Co., Scientific Imaging Systems Division, New Haven, Connecticut erhältlich. Teilsequenzen des erfindunsgemäßen Proteins bestehend aus mindestens 20, stärker bevorzugt mindestens 30 und noch stärker bevorzugt mindestens 50 Aminosäuren umfassende Teilabschnitte der nativen ee3-Sequenzen können nach dem Fachmann geläufigen Verfahren bspw. chemisch synthetisiert werden und können vorzugsweise als Antigene für die Produktion von Antikörpern eingesetzt werden. Vorzugsweise wird es sich bei diesen Teilabschnitten bzw. deren Derivaten, Allelen oder Fragmenten um offenbarte Sequenzen handeln, die im räumlichen Modell der Proteine solche Regionen bilden, die in den nativen erfindungsgemäßen ee3-Sequenzen, in den Figuren 13, oder zumindest teilweise die Proteinoberfläche ausmachen. Bevorzugte Teilsequenzen von mindestens 20 AS Länge werden zumindest tw. den cytoplasmatischen Abschnitt der erfindungsgemäßen Proteine der ee3-Familie umfassen, insbesondere bevorzugt wird ein erfindungsgemäßer Teilabschnitt mindestens 20 AS lange Peptide einer der erfindungsgemäßen Sequenzen gemäß Figur 8 zwischen Position 600 und Position 752 (gemäß Figur 8) aufweisen, bspw. das Peptid WWFGIRKDFCQFLLEIFPFLRE (Position 609 bis 630, 21 AS Länge).

AS-Sequenzen, bspw. die Sequenzen der humanen Proteine ee3_1 oder ee3_2, weisen darüber hinaus spezifische Sequenzmotive auf, die sich auch bei anderen Vertretern der GPCR-Klasse in ähnlicher Form wiederfinden. So tritt bspw. eine typische Signatur-Triplett-Sequenz unterhalb der dritten Transmembrandomäne bei Proteinen der GPCR-Klasse auf (Sequenz mit der Sequenzfolge DRY (AS im Ein-Buchstaben-Code).

Bei erfindungsgemäßem ee3_1 findet sich die Sequenzfolge DRI (Position 103-105) unterhalb von TM3 (83-102) (gemäß Figur 15A). Bei nach dem Stand der Technik bekannten Vertretern der GPCR-Klasse (Galanin-2-Rezeptor, C5a-Rezeptor (Ratte), BK-2 (human) oder CXCR-5 (human)) findet sich die Sequenzfolge DRY oder DRF an entsprechenden Positionen.

Nichtmenschliche transgene Tiere stellen einen weiteren Gegenstand der vorliegenden Erfindung dar. Bei erfindungsgemäßen transgenen Tieren handelt es sich um Tiere, die genetisch dahingehend verändert sind, daß sie eine im Vergleich zum Normaltier veränderte Menge eines erfindungsgemäßen Genprodukts in mindestens einem Gewebe exprimieren bzw. enthalten (bspw. durch Modifikation der Promotorbereichs eines erfindungsgemäßen Gens) oder ein verändertes Genprodukt (bspw. ein erfindungsgemäßes Derivat eines Proteins der ee3-Familie, bspw. auch ein Fragment) enthalten oder exprimieren. Hierbei sind erfindungsgemäß auch solche Tiere eingeschlossen, die die nativ vorhandene erfindungsgemäße DNA-Sequenz (a) auf der genetischen Ebene entweder tw. oder vollständig nicht mehr aufweisen oder (b) zwar auf der genetischen Ebene erfindungsgemäße Sequenzen aufweisen, diese jedoch nicht transkribieren und/oder translatieren können und daher das Genprodukt nicht mehr enthalten. Darüber hinaus kann (können) bei einem transgenen Tier die native erfindungsgemäßen Sequenzen um mindestens eine erfindungsgemäße DNA-Sequenz ergänzt bzw. durch mindestens eine erfindungsgemäße DNA-Sequenz substituiert sein. Insbesondere kann es sich bei der (den) substituierten und/oder ergänzten Sequenz (en) um erfindungsgemäße Sequenzen handeln, die nicht-nativer Natur sind.

Die Herstellung von in bezug auf erfindungsgemäße Sequenzen transgenen und/oder "knock-out" Tieren, insbesondere Mäusen, Ratten Schweinen, Rindern, Schafen, Fruchtfliegen (Drosophila), C. elegans oder Zebrafischen, erfolgt auf dem Fachmann geläufige Weise. Hierzu wird z.B. eine erfindungsgemäße cDNA Sequenz oder native oder nicht-native Variante in transgenen Mäusen exprimiert, z.B. unter einem NSE-Promotor in Neuronen, unter einem MBP-Promotor in Oligodendrozyten etc.. Die genetisch veränderten Tiere können danach in unterschiedlichen Krankheitsmodellen untersucht werden (z.B. experimentell herbeigeführte Schlaganfall, MCAO). Die Herstellung von "knock-out" Tieren kann zudem Hinweise auf die Auswirkungen von Inhibitoren auf den Gesamtorganismen liefern, da ein "knock out Modell" insoweit der Inhibition erfindungsgemäßer nativer Sequenzen entspricht. Insoweit kann ein derartiges Verfahren bei einer präklinischen Prüfung von erfindungsgemäßen inhibitorischen Substanzen, z.B. erfindungsgemäße Peptide, Peptidanaloga oder andere kleine organische Verbindungen, zum Einsatz kommen.

Sämtliche multizellulären Organismen können erfindungsgemäß transgen ausgestaltet sein, insbesondere Säugetiere, bspw. Mäuse, Ratten, Schafe, Rinder oder Schweine. Auch transgene Pflanzen sind im Prinzip denkbar. Bei den transgenen Organismen kann es sich auch um sogenannte "Knock-Out"-Tiere handeln. Dabei können die transgenen Tiere eine funktionelle oder nicht funktionelle erfindungsgemäße Nukleinsäuresequenz oder ein funktionelles oder nicht funktionelles Nukleinsäurekonstrukt allein oder in Kombination mit einer funktionellen oder nicht funktionellen Sequenz, die für erfindungsgemäße Proteine kodiert, enthalten.

Eine weiter erfindungsgemäße Ausgestaltung der oben beschriebenen nichtmenschlichen, transgenen Tiere sind transgene Tiere, in deren Keimzellen oder der Gesamtheit oder einem Teil der somatischen Zellen oder in deren Keimzellen oder der Gesamtheit oder einem Teil der somatischen Zellen die native(n) erfindungsgemäße(n) Nukleotidsequenz(en) ee3-Familie, insbesondere der Sequenzen mit den Nummern 1 bis 4), durch gentechnische Verfahren verändert oder durch Einfügen von DNA-Elementen unterbrochen wurden. Eine weitere Möglichkeit des Einsatzes einer erfindungsgemäßen Nukleotidsequenz oder Teilen davon ist die Erzeugung transgener oder knock-out- oder konditioneller oder regionenspezifischer knock-out Tiere oder spezifischer Mutationen bei gentechnisch veränderten Tieren (Ausubel et al. (eds.) 1998, Current Protocols in Molecular Biology, John Wiley & Sons, New York und Torres et al., (eds.) 1997, Laboratory protocols for conditional gene targeting, Oxford University Press, Oxford). Darüber hinaus können ebenso bestimmte Mutationen, bspw. Veränderungen der Promotoren oder Insertion von Enhancern, eingeführt werden, um beispielsweise konstitutiv aktive ee3-Proteine in den transgenen Tieren zu erzeugen ("knock-in"-Tiere). Auch derartige Tiere können bspw. erfindungsgemäß eingesetzt werden, um in präklinischen Untersuchungen Analogiemodelle für potentielle Agonisten der ee3-Proteinfunktion zu liefern.

Über transgene Überexpression oder genetische Mutation (Nullmutation oder spezifische Deletionen, Insertionen oder Veränderungen) durch homologe Rekombination in embryonalen Stammzellen kann man Tiermodelle erzeugen, die wertvolle weitere Informationen über die (Patho-)Physiologie der erfindungsgemäßen Sequenzen liefern. Solchermaßen hergestellte Tiermodelle können essentielle Testsysteme zur Evaluierung neuartiger Therapeutika darstellen, die die biologische Funktion von erfindungsgemäßen Proteinen, insbesondere von Proteinen mit einer der Sequenzen 5 für neurale, immunologische, proliferative oder andere Prozesse beeinflussen.

Ein weiterer Gegenstand der vorliegenden Erfindung ist ein Antikörper, der ein Epitop auf einem erfindungsgemäßen ee3-Genprodukt, insbesondere einem erfindungsgemäßen Protein gemäß Figuren 13, oder Derivaten, Fragmenten oder Isoformen oder Allelen, erkennt, aber auch gegen z.B. erfindungsgemäße mRNA gerichtet sein kann. Der Begriff "Antikörper" umfaßt i.S. der vorliegenden Erfindung sowohl polyklonale Antikörper als auch monoklonale Antikörper, chimärische Antikörper, anti-idiotypische Antikörper (gerichtet gegen erfindungsgemäße Antikörper), die alle in gebundener oder löslicher Form vorliegen und ggf. durch "Label" markiert sein können, sowie auch Fragmente der vorgenannten Antikörper. Neben den Fragmenten von erfindungsgemäßen Antikörpern in Alleinstellung können erfindungsgemäße Antikörper auch in rekombinanter Form als Fusionsproteine mit anderen (Protein)-Bestandteilen auftreten. Fragmente als solche oder Fragmente von erfindungsgemäßen Antikörpern als Bestandteile von Fusionsproteinen werden typischerweise durch die Methoden enzymatischer Spaltung, der ProteinSynthese oder die dem Fachmann geläufigen Rekombinationsmethoden hergestellt. Als Antikörper werden nach der vorliegenden Erfindung also sowohl polyklonale, monoklonale, humane oder humanisierte oder rekombinante Antikörper oder Fragmente davon, single chain Antikörper oder auch synthetische Antikörper bezeichnet.

Bei den polyklonalen Antikörpern handelt es sich um heterogene Mischungen von Antikörpermolekülen, die aus Seren von Tieren hergestellt werden, die mit einem Antigen immunisiert worden sind. Zum Gegenstand der Erfindung gehören aber auch polyklonale monospezifische Antikörper, die nach Aufreinigung der Antikörper (bspw. über eine Säule, die mit Peptiden eines spezifischen Epitops beladen sind) erhalten werden. Ein monoklonaler Antikörper enthält eine im wesentlichen homogene Population von Antikörpern, die spezifisch gegen Antigene gerichtet sind, wobei die Antikörper im wesentlichen gleiche Epitop-Bindungsstellen aufweisen. Monoklonale Anti-körper können durch die im Stand der Technik bekannten Verfahren erhalten werden (z. B. Köhler und Milstein, Nature, 256, 495-397, (1975); US-Patent 4,376,110; Ausübel et al., Harlow und Lane "Antikörper": Laboratory Manual, Cold Spring, Harbor Laboratory (1988); Ausubel et al., (eds), 1998, Current Protocols in Molecular Biology, John Wiley & Sons, New York).). Die in den vorgenannten Literaturstellen enthaltene Beschreibung wird als Bestandteil der vorliegenden Erfindung in die Offenbarung der vorliegenden Erfindung einbezogen.

Auch lassen sich gentechnisch manipulierte erfindungsgemäße Antikörper nach Verfahren, wie in den vorgenannten Druckschriften beschrieben herstellen. Kurz gesagt, werden dazu Antikörper-produzierende Zellen angezogen und die mRNA bei ausreichender optischer Dichte der Zellen über Zellyse mit Guanidiniumthiocyanat, Ansäuern mit Natriumacetat, Extraktion mit Phenol, Chloroform/Isoamylalkohol, Fällungen mit Isopropanol und Waschen mit Ethanol aus den Zellen in bekannter Weise isoliert. Anschließend wird mit Hilfe der Reversen Transcriptase cDNA aus der mRNA synthetisiert. Die synthetisierte cDNA kann direkt oder nach genetischer Manipulation beispielsweise durch "site directed mutagenesis", Einführung von Insertionen, Inversionen, Deletionen oder Basenaustausche in geeignete tierische, pilzliche, bakterielle oder virale Vektoren inseriert und in den entsprechenden Wirtsorganismen exprimiert werden. Bevorzugt werden bakterielle oder Hefe Vektoren wie pBR322, pUC18/19, pACYC184, Lambda oder Hefe-mu-Vektoren zur Klonierung der Gene und die Expression in Bakterien wie E. coli bzw. in der Hefe wie Saccharomyces cerevisiae. Spezifische Antikörper gegen die erfindungsgemäßen Proteine können sich sowohl als diagnostische Reagenzien als auch als Therapeutika bei Erkrankungen eignen, bei denen Proteine der ee3-Familie von pathophysiologischer Bedeutung ist.

Erfindungsgemäße Antikörper können einer der folgenden Immunglobulinklassen angehören: IgG, IdD, IgM, IgE, IgA, GILD und ggf. einer Unterklasse der vorgenannten Klassen, wie die Subklassen des IgG oder deren Mischungen zu verstehen. Bevorzugt sind IgG und seine Subklassen wie beispielsweise IgG1, IgG2, IgG2a, IgG2b, IgG3 oder IgGM. Besonders bevorzugt sind die IgG Subtypen IgG1/k oder IgG2b/k. Ein Hybridom-Zellklon, der erfindungsgemäße monoklonale Antikörper produziert, kann in vitro, in situ oder in vivo kultiviert werden. Die Herstellung von großen Titern an monoklonalen Antikörpern erfolgt vorzugsweise in vivo oder in situ.

Bei den erfindungsgemäßen chimärische Antikörpern handelt es sich um Moleküle, die verschiedene Bestandteile enthalten, wobei diese sich aus verschiedenen Tierarten ableiten (z. B. Antikörper, die eine variable Region, die aus einem Mäusemonoklonalen Antikörper abgeleitet ist, und eine konstante Region eines humanen Immunglobulins aufweisen). Chimärische Antikörper werden vorzugsweise eingesetzt, um einerseits die Immunogenizität bei der Anwendung zu reduzieren und andererseits die Ausbeuten bei der Produktion zu erhöhen, z.B. ergeben murine monoklonale Antikörper höhere Ausbeuten aus Hybridom-Zellinien, führen aber auch zu einer höheren Immunogenizität beim Menschen, so daß human/murine chimärische Antikörper vorzugsweise eingesetzt werden. Chimärische Antikörper und Verfahren zu ihrer Herstellung sind aus dem Stand der Technik bekannt (Cabilly et al., Proc. Natl. Sci. USA 81: 3273-3277 (1984); Morrison et al. Proc. Natl. Acad. Sci USA 81:6851-6855 (1984); Boulianne et al. Nature 312 643-646 (1984); Cabilly et al., EP-A-125023; Neuberger et al., Nature 314: 268-270 (1985); Taniguchi et al., EP-A-171496; Morrion et al., EP-A-173494; Neuberger et äl., WO 86/01533; Kudo et al., EP-A-184187; Sahagan et al., J. Immunol. 137: 1066-1074 (1986); Robinson et al., WO 87/02671; Liu et al., Proc. Natl. Acad. Sci USA 84:3439-3443 (1987); Sun et al., Proc. Natl. Acad. Sci USA 84:214218 (1987); Better et al., Science 240: 1041-1043 (1988) und Harlow und Lane, Antikörper: A Laboratory Manual, wie oben zitiert. Diese Zitatstellen werden als zur Offenbarung gehörig in die vorliegende Erfindung einbezogen.

Ganz besonders bevorzugt wird ein solcher erfindungsgemäßer Antikörper gegen einen extrazellulären Abschnitt auf einem erfindungsgemäßen ee3-Protein, insbesondere einem Protein gemäß Figuren 13, oder als Epitop gerichtet sein. Erfindungsgemäße Antikörper, in allen Variationen wie zuvor offenbart, können zur Inhibierung von erfindungsgemäßen Proteinen der ee3-Familie eingesetzt werden, bspw. in vitro für experimentelle Untersuchungen, in situ für bspw. Markierungszwecke oder auch in vivo zum therapeutischen Einsatz, indem sie z.B. i.v., subkutan, intraarteriell oder intramuskulär injiziert werden.

Ein erfindungsgemäßer anti-idiotypischer Antikörper ist ein Antikörper, der eine Determinante, die im allgemeinen mit der Antigenbindungsstelle eines erfindungsgemäßen Antikörpers assoziiert ist, erkennt. Ein anti-idiotypischer Antikörper kann durch die Immunisierung eines Tieres der gleichen Art und des gleichen genetischen Typs (z.B. eines Mäusestamms) als Ausgangspunkt für einen monoklonalen Antikörper, gegen welchen ein erfindungsgemäßer anti-idiotypischer Antikörper gerichtet ist, hergestellt werden. Das immunisierte Tier wird die idiotypischen Determinanten des immunisierenden Antikörpers durch die Produktion eines Antikörpers, der gegen die idiotypischen Determinanten gerichtet ist (nämlich ein erfindungsgemäßer anti-idiotypischer Antikörper), erkennen (U.S. 4,699,880). Ein erfindungsgemäßer anti-idiotypischer Antikörper kann auch als Immunogen eingesetzt werden, um eine Immunantwort in einem weiteren Tier hervorzurufen und um dort zur Produktion eines sog. anti-anti-idiotypischen Antikörpers zu führen. Der anti-anti-idiotypische Antikörper kann, muß aber nicht, bezüglich seiner Epitop-Konstruktion identisch mit dem originären monoklonalen Antikörper sein, der die anti-idiotypische Reaktion hervorgerufen hat. Auf diese Weise können durch die Verwendung von gegen idiotypische Determinanten eines monoklonalen Antikörpers gerichtete Antikörper andere Klone, die Antikörper von identischer Spezifität exprimieren, identifiziert werden.

Monoklonale Antikörper, die gegen erfindungsgemäße Proteine, Analoge, Fragmente oder Derivate dieser erfindungsgemäßen Proteine gerichtet sind, können eingesetzt werden, um die Bindung von anti-idiotypischen Antikörpern in entsprechenden Tieren, wie z. B. der BALB/c Maus, zu induzieren. Zellen aus der Milz einer solchen immunisierten Maus können verwendet werden, um anti-idiotypische Hybridom-Zellinien, die anti-idiotypische monoklonale Antikörper sekretieren, zu produzieren. Weiterhin können anti-idiotypische monoklonale Antikörper auch an einen Träger gekoppelt werden (KLH, "keyhole limpet hemocyanin") und dann verwendet werden, um weitere BALB/c-Mäuse zu immunisieren. Die Sera dieser Mäuse enthalten dann anti-anti-idiotypische Antikörper, die die Bindungseigenschaften der originären monoklonalen Antikörper haben und spezifisch für ein Epitop des erfindungsgemäßen Proteins oder eines Fragments oder Derivats von demselben sind. Die anti-idiotypischen monoklonalen Antikörper haben auf diese Weise ihre eigenen idiotypischen Epitope oder "I-diotope", die strukturell mit dem zu untersuchenden Epitop ähnlich sind.

Die Bezeichnung "Antikörper" soll sowohl intakte Moleküle als auch Fragmente derselben einschließen. Als Fragmente seien alle verkürzten oder veränderten Antikörperfragmente mit einer oder zwei dem Antigen-komplementären Bindungsstellen, wie Antikörperteile mit einer den Antikörper entsprechenden von leichter und schwerer Kette gebildeten Bindungsstelle wie Fv-, Fab- oder F(ab')₂-Fragmente oder Einzelstrangfragmente, genannt. Bevorzugt sind verkürzte Doppelstrangfragmente wie Fv-, Fab- oder F(ab')₂. Fab und F(ab')₂-Fragmente entbehren eines Fc-Fragments, wie etwa in einem intakten Antikörper vorhanden, so daß sie im Blutkreislauf schneller transportiert werden können und vergleichsweise weniger nicht-spezifische Gewebsbindung als intakte Antikörper aufweisen. Hierbei wird hervorgehoben, daß Fab- und F(ab')₂-Fragmente von erfindungsgemäßen Antikörpern, ebenso wie diese selbst, bei der Detektion (qualitativ) und Quantifizierung von erfindungsgemäßen Proteinen (ggf. auch zur Detektion der Proteinaktivität (z.B. spezifische Phosphorylierungen) der erfindungsgemäßen Proteine) eingesetzt werden können, weswegen auch Verfahren zur qualitativen und quantitativen Bestimmung bzw. zur zur Quantifizierung der Proteinaktivität von erfindungsgemäßen Proteinen Gegenstand der vorliegenden Erfindung sind.

Solche Fragmente werden typischerweise durch proteolytische Spaltung hergestellt, indem Enzyme, wie z. B. Papain (zur Herstellung von Fab-Fragmenten) oder Pepsin (zur Herstellung von F(ab')₂, Fragmenten) verwendet werden, oder durch chemische Oxidation oder durch gentechnische Manipulation der Antikörpergene erhalten werden.

Gegenstand der vorliegenden Erfindung sind auch Mischungen von Antikörpern im Sinne der vorliegenden Erfindung. Neben den Antikörpern können auch Mischungen von Antikörpern für alle gemäß der vorliegenden Erfindung beschriebenen Verfahren oder Verwendungen eingesetzt werden. Sowohl gereinigte Fraktionen monoklonaler Antikörper, polyklonale Antikörper oder Mischungen monoklonaler Antikörper kommen als Arzneimittel zum Einsatz und finden Verwendung bei der Herstellung von Arzneimitteln zur behandlung von cerebralen Ischämien (z.B. Schlaganfall), degenerativen Erkrankungen, insbesondere neurodegenerativen Erkrankungen, und neurologischen Erkrankungen wie z.B. Epilepsie.

Erfindungsgemäße Antikörper, einschließlich der Fragmente von diesen Antikörpern, bzw. deren Mischungen können zur quantitativen oder qualitativen Detektion von erfindungsgemäßem ee3-Genprodukt, insbesondere Proteinen gemäß Figuren 13, oder oder deren Fragmente oder Derivate, in einer Probe eingesetzt werden oder auch zur Detektion von Zellen, die erfindungsgemäße Proteine exprimieren und ggf. sekretieren. Insoweit wird die Verwendung erfindungsgemäßer Antikörper als Diagnostika offenbart. So kann etwa über erfindungsgemäße Antikörper die Menge an erfindungsgemäßem Genprodukt und u.U. deren Aktivität (z.B. spezifischePhosphorylierungen), bspw. der Proteine gemäß Figuren 13, oder bestimmt werden. Die Detektion kann mit Hilfe von Immunofluoreszenz-Verfahren erreicht werden, die Fluoreszenz-markierte Antikörper in Kombination mit Lichtmikroskopie, Flußzytometrie oder fluorometrischer Detektion durchgeführt werden.

Erfindungsgemäße Antikörper im Sinne der Erfindung (dies schließt Fragmente dieser Antikörper ein oder auch Mischungen von Antikörpern) eignen sich für histologische Untersuchungen, wie z.B. im Rahmen der Immunofluoreszenz oder Immunoelektromikroskopie, für die in situ Detektion eines erfindungsgemäßen Proteins. Die in situ Detektion kann dadurch erfolgen, daß eine histologische Probe von einem Patienten genommen wird und markierte erfindungsgemäße Antikörper zu einer solchen Probe hinzugegeben werden. Der Antikörper (oder ein Fragment dieses Antikörpers) wird in markierter Form auf die biologische Probe aufgetragen. Auf diese Weise ist es nicht nur möglich, die Anwesenheit von erfindungsgemäßem Protein in der Probe zu bestimmen, sondern auch die Verteilung des erfindungsgemäßen Proteins in dem untersuchten Gewebe. Bei der biologischen Probe kann es sich um eine biologische Flüssigkeit, ein Gewebeextrakt, geerntete Zellen, wie z. B. Immunzellen oder Herzmuskel- oder Leberzellen, oder allgemein um Zellen, die in einer Gewebekultur inkubiert worden sind, handeln. Die Detektion des markierten Antikörpers kann je nach Art der Markierung durch im Stand der Technik bekannte Verfahren (z. B. durch Fluoreszenzverfahren) erfolgen. Die biologische Probe kann aber auch auf einem Festphasenträger, wie z. B. Nitrocellulose oder ein anderes Trägermaterial, aufgetragen werden, so daß die Zellen, Zellteile oder löslichen Proteine immobilisiert werden. Der Träger kann dann mit einem geeigneten Puffer ein- oder mehrfach gewaschen werden, wobei nachfolgend mit einem detektierbar markierten Antikörper nach der vorliegenden Erfindung behandelt wird. Der Festphasenträger kann dann mit dem Puffer ein zweites Mal gewaschen werden, um nicht-gebundenen Antikörper zu beseitigen. Die Menge an gebundener Markierung auf dem Festphasenträger kann dann mit einem herkömmlichen Verfahren bestimmt werden.

Als Träger eignen sich insbesondere Glas, Polystyrol, Polypropylen, Polyethylen, Dextran, Nylon-Amylasen, natürliche oder modifizierte Zellulosen, Polyacrylamide und Magnetit. Der Träger kann entweder bedingt löslichen oder unlöslichen Charakters sein, um die Bedingungen nach Maßgabe der vorliegenden Erfindung zu erfüllen. Das Trägermaterial kann beliebige Formen einnehmen, z. B. in Form von Kügelchen ("beads"), oder zylindrisch oder sphärisch sein, wobei Polystyrol-Kügelchen als Träger bevorzugt sind.

Eine detektierbare Antikörpermarkierung kann auf verschiedene Weise erfolgen. Beispielsweise kann der Antikörper an ein Enzym gebunden werden, wobei das Enzym schließlich in einem Immunoassay (EIA) eingesetzt werden kann. Das Enzym kann dann später mit einem entsprechenden Substrat reagieren, so daß eine chemische Verbindung entsteht, die auf eine dem Fachmann geläufige Art und Weise detektiert und ggf. quantifiziert werden kann, z. B. durch Spektrophotometrie, Fluorometrie oder andere optische Verfahren. Bei dem Enzym kann es sich um Malat-Dehydrogenase, Staphylokokken-Nuklease, delta-5-Steroid Isomerase, Hefe-Alkohol-Dehydrogenase, alpha-Glycerophosphat-dehydrogenase, Triosephosphatisomerase, Meerrettich-Peroxidase, alkalische Phosphatase, Aspariginase, Glucoseoxidase, beta-Galactosidase, Ribonuklease, Urease, Katalase, Glucose-6-phosphat-Dehydrogenase, Glucoamylase oder Acetylcholinesterase handeln. Die Detektion wird dann über ein chromogenes Substrat, das spezifisch für das für die Markierung eingesetzte Enzym ist, ermöglicht und kann schließlich z.B. über Sichtvergleich des durch die Enzymreaktion umgesetzten Substrats im Vergleich zu Kontrollstandards erfolgen.

Weiterhin kann die Detektion durch andere Immunoassays sichergestellt werden, z.B. durch radioaktive Markierung der Antikörper oder Antikörperfragmente (also durch einen Radioimmunoassay (RIA; Laboratory Techniques and Biochemistry in Molecular Biology, Work, T. et al. North Holland Publishing Company, New York (1978). Das radioaktive Isotop kann dabei durch die Verwendung von Szintillationszählern oder durch Autoradigraphie detektiert und quantifiziert werden.

Fluoreszierende Verbindungen können gleichfalls zur Markierung eingesetzt werden, beispielsweise Verbindungen wie Fluorescinisothiocyanat, Rhodamin, Phyoerythrin, Phycocyanin, Allophycocyanin, o-Phthaldehyd und Fluorescamin. Auch fluoreszensemittierende Metalle, wie z. B. ¹⁵²E oder andere Metalle aus der Lanthanid-Gruppe, können eingesetzt werden. Diese Metalle werden an den Antikörper über Chelatgruppen, wie z. B. Diethylentriaminpentaessigsäure (ETPA) oder EDTA angekoppelt. Weiterhin kann der erfindungsgemäße Antikörper über eine mit Hilfe von Chemilumineszenz wirkende Verbindung angekoppelt werden. Die Gegenwart des Chemilumineszenzmarkierten Antikörpers wird dann über die Lumineszenz, die im Verlauf einer chemischen Reaktion entsteht, detektiert. Beispiele für derartige Verbindungen sind Luminol, Isoluminol, Acridiniumester, Imidazol, Acridiniumsalz oder Oxalatester. Gleichermaßen können auch biolumineszente Verbindungen zum Einsatz kommen. Biolumineszenz ist eine Unterart der Chemilumineszenz, die bei biologischen Systemen vorgefunden wird, wobei ein katalytisches Protein die Effizienz der chemilumineszenten Reaktion verstärkt. Die Detektion des biolumineszenten Proteins erfolgt wiederum über die Lumineszenz, wobei als biolumineszente Verbindung beispielsweise Luciferin, Luciferase oder Aequorin in Betracht kommen.

Ein erfindungsgemäßer Antikörper kann für die Verwendung in einem immunometrischen Assay, auch bekannt als "two-site" oder "sandwich" Assay, zur Anwendung gelangen. Typische immunometrische Assay-Systeme schließen sog. "Vorwärts"-Assays ein, die sich dadurch auszeichnen, daß erfindungsgemäße Antikörper an ein Festphasensystem gebunden sind und daß der Antikörper mit der Probe, die untersucht wird, auf diese Weise in Kontakt gebracht wird. Derart wird das Antigen aus der Probe durch die Bildung eines binären Festphasen-Antikörper-Antigen-Komplexes aus der Probe isoliert. Nach einer geeigneten Inkubationszeit wird der feste Träger gewaschen, um den verbleibenden Rest der flüssigen Probe zu beseitigen, einschließlich des ggf. nicht gebundenen Antigens, und daraufhin mit einer Lösung in Kontakt gebracht, die eine unbekannte Menge an markiertem Detektionsantikörper enthält. Der markierte Antikörper dient hierbei als sog. Reporter-Molekül. Nach einer zweiten Inkubationszeit, die es den markierten Antikörper erlaubt, mit dem an die Festphase gebundenen Antigen zu assoziierten, wird der Festphasenträger erneut gewaschen, um markierte Antikörper, die nicht reagiert haben, zu beseitigen.

In einer alternativen Assay-Form kann auch ein sog. "sandwich"-Assay zum Einsatz kommen. Hierbei kann ein einziger Inkubationsschritt ausreichen, wenn der an die Festphase gebundene Antikörpern und der markierte Antikörper beide gleichzeitig auf die zu testende Probe aufgebracht werden. Nach Abschluß der Inkubation wird der Festphasenträger gewaschen, um Rückstände der flüssigen Probe und der nichtassoziierten markierten Antikörper zu beseitigen. Die Anwesenheit von markiertem Antikörper auf dem Festphasenträger wird genau so bestimmt, wie bei den konventionellen "Vorwärts"-Sandwich-Assay. Bei dem sog. reversen Assay wird schrittweise zunächst eine Lösung des markierten Antikörpers zur Flüssigprobe hinzugefügt, gefolgt von der Beimischung von nicht-markiertem Antikörper, gebunden an einen Festphasenträger, nach Ablauf einer geeigneten Inkubationszeit. Nach einem zweiten Inkubationsschritt wird der Festphasenträger in herkömmlicher Weise gewaschen, um ihn von Probenüberresten und von markiertem Antikörper, der nicht reagiert hat, zu befreien. Die Bestimmung des markierten Antikörpers, der mit dem Festphasenträger reagiert hat, wird dann, so wie oben beschrieben, durchgeführt.

Nach der vorliegenden Erfindung werden weiterhin Verfahren zur Expression von erfindungsgemäßen ee3-Genprodukten, insbesondere also von Polypeptiden gemäß Figuren 13, oder einschließlich aller Derivate, Analoge und Fragmente, offenbart, wobei hierfür Wirtszellen mit einem erfindungsgemäßen Expressionsvektor transformiert werden ausgenommen ein Polypeptid gemäß SEQ ID No:31 der U0 01 /98353.. Dieses Verfahren zur Expression von Genprodukten, die auf einer erfindungsgemäßen DNA-Sequenz beruhen, dient nicht dazu, das entsprechende Genprodukt zu konzentrieren und aufzureinigen, sondern vielmehr dazu, den Zellstoffwechsel durch das Einführen der erfindungsgemäßen DNA-Sequenzen über die Expression des dazugehörigen Genprodukts zu beeinflussen. Hier ist insbesondere an die Verwendung der mit Hilfe von Expressionsvektoren transformierten Wirtszellen als Arzneimittel bzw. zur Herstellung eines Arzneimittels, insbesondere zum Zwecke der Behandlung von Erkrankungen, bspw. von Tumorerkrankungen, neurologischen Erkrankungen, neurodegenerativen Erkrankungen (bspw. Multipler Sklerose, Morbus Parkinson) cerebralen Ischämien (z.B. Schlaganfall). Allgemein werden erfindungsgemäße Wirtszellen bei Erkrankungen zur Verfügung gestellt, denen eine Fehlregulation der Apoptose, der Nekrose, des Zellwachstums, der Zellteilung, der Zelldifferenzierung oder der Zellplastizität zugrunde liegt. Die derart erfindungsgemäß ex vivo transformierten autologen oder allogenen Wirtszellen können dann Patienten transplantiert werden.

Gemäß einem weiteren Aspekt der vorliegenden Erfindung wird ein Verfahren zur Isolierung von Genprodukten mit mindestens einer mit der erfindungsgemäßen Aminosäuresequenzen, wobei die Wirtszellen mit einem erfindungsgemäßen Expressionsvektor transformiert und dann unter geeigneten, die Expression fördernden Bedingungen kultiviert werden, so daß das Genprodukt schließlich aus der Kultur aufgereinigt werden kann. Das erfindungsgemäße Genprodukt der erfindungsgemäßen DNA-Sequenz kann dabei, abhängig von dem Expressionssystem, aus einem Kulturmedium oder aus Zellextrakten isoliert werden. Der Fachmann kann ohne weiteres erkennen, daß die jeweiligen Isolierungsmethoden und das Verfahren bei der Aufreinigung des von einer erfindungsgemäßen DNA kodierten, rekombinanten Proteins stark vom Typ der Wirtszelle oder auch von dem Umstand, ob das Protein in das Medium sekretiert wird, abhängt. Zum Beispiel können Expressionssysteme eingesetzt werden, die zur Sekretion des rekombinanten Proteins aus der Wirtszelle führen. Das Kulturmedium muß in diesem Fall durch kommerziell erhältliche Proteinkonzentrationsfilter, z.B. Amicon oder Millipore Pelicon, aufkonzentriert werden. Nach dem Konzentrationsschritt kann ein Reinigungsschritt erfolgen, z.B. ein Gelfiltrationsschritt oder eine Reinigung mit Hilfe von säulenchromatographische Methoden. Alternativ kann aber auch ein Anionenaustauscher eingesetzt werden, der eine Matrix mit DEAE aufweist.

Als Matrix dienen dabei alle aus der Proteinreinigung bekannten Materialien, z.B. Acrylamid oder Agarose oder Dextran oder ähnliches. Es kann aber auch ein Kationenaustauscher eingesetzt werden, der dann typischerweise Carboxymethyl-Gruppen enthält. Zur weiteren Reinigung eines durch eine erfindungsgemäße DNA codierten Polypeptids können dann HPLC-Schritte dienen. Es kann sich um einen oder mehrere Schritte handeln. Insbesondere wird die "Reversed- Phase"-Methode eingesetzt. Diese Schritte dienen zum Erhalt eines im wesentlichen homogenen rekombinanten Proteins einer erfindungsgemäßen DNA-Sequenz.

Neben bakteriellen Zellkulturen zur Isolierung des Genprodukts können auch transformierte Hefezellen eingesetzt werden. In diesem Fall kann das translatierte Protein sekretiert werden, so daß die Proteinreinigung vereinfacht wird. Sekretiertes rekombinantes Protein aus einer Hefewirtszelle kann durch Methoden erhalten werden, wie sie bei Urdal et al. (J. Chromato. 296:171 (1994)) offenbart sind und Bestandteil der Offenbarung der vorliegenden erfindung sind.

Erfindungsgemäße Nukleinsäuresequenzen, insbesondere erfindungsgemäße DNA-Sequenzen, und/oder erfindungsgemäße Genprodukte können als Arzneimittel bzw. zur Herstellung eines Arzneimittels Verwendung finden. Diese können als solche verabreicht werden (bspw. bukkal, intravenös, oral, parenteral, nasal, subkutan) oder in Kombination mit weiteren Wirk- , Hilfs- oder arzneimitteltypischen Zusatzstoffen. Erfindungsgemäße Nukleinsäure kann als nackte Nukleinsäure, insbesondere intravenös, injiziert werden oder aber mit Hilfe von Vektoren dem Patienten verabreicht werden. Bei diesen Vektoren kann es sich um Plasmide als solche handeln, aber auch um virale Vektoren, insbesondere retrovirale oder adenovirale Vektoren, oder auch um Liposomen, die nackte erfindungsgemäße DNA oder ein Plasmid, das erfindungsgemäße DNA enthält, aufweisen können.

Die Verwendung von erfindungsgemäßen Sequenzen, bzw. deren Varianten, sowie erfindungsgemäßer Proteinheteromere sowie davon abgeleiteter erfindungsgemäßer Reagenzien (Oligonukleotide, Antikörper, Peptide) kommt somit für die Herstellung eines Arzneimittels zu therapeutischen Zwecken, d.h. zur Behandlung von Erkrankungen, in Betracht. Ganz besonders bevorzugt ist dabei der therapeutische Einsatz zur Behandlung bzw. zur Herstellung eines Arzneimittels zur Behandlung von Erkrankungen oder pathophysiologischen Zuständen, die auf fehlgesteuerter Regulation der Homöostase von Zelltod- und Proliferationsereignissen beruhen.

In diesem Zusammenhang kommt insbesondere auch die erfindungsgemäße Erkenntnis zum Tragen, daß EPO, dessen Wirkung auf die Veränderung des Transkriptionsverhaltens von Zellen zurückzuführen ist, auf direkte oder indirekte Weise die transkriptionelle Hochregulation erfindungsgemäßer Rezeptoren, bspw. ee3_1, induziert. Erfindungsgemäße Rezeptoren vermitteln also die Wirkung von EPO und haben daher auch kritische Bedeutung für die hiermit in Zusammenhang stehenden Erkrankungen. Dies bedeutet u.a., daß erfindungsgemäße Rezeptoren bestimmte Wirkungen von EPO selektiv beeinflussen können, z.B. eine neuroprotektive Wirkung (z.B bei neurodegenerativen Erkrankungen), wobei z.B. die Aktivierung des Transkriptionsfaktors NF-kappaB ein wichtiger Schritt in der neuroprotektiven Wirkung von EPO ist), oder eine Hirnleistungssteigerung, (z.B bei Demenzen). Entsprechende Untersuchungen an Tierexperimenten lassen eine funktionelle Zuschreibung erfindungsgemäßer Gegenstände bei Modellen neurologischer Erkrankungen wie cerebrale Ischämien, experimentell induzierte Enzephalomyelitis, oder Subarachnoidalblutungen zu.

Dies ist für diese Teilwirkungen wünschenswert, da die Gabe von EPO neben der neuroprotektiven Wirkung eine Erhöhung des Hämatokrit bewirken würde, die z.T. der neuroprotektiven Wirkung entgegenstehen würde, da die rheologischen Eigenschaften des Blutes verschlechtert würden, was sich negativ auf die Mikrozirkulation auswirkt, wie bei Mäusen mit Oberexpression von Erythropoetin gezeigt (Wiessner et al., 2001 , J Cereb Blood Flow Metab, 21, 857-64).

Damit kommt die Verwendung erfindungsgemäßer Gegenstände, bspw. erfindungsgemäßer Nukleotidsequenzen, Oligo- oder Polypeptide, Expressionsvektoren, oder Wirtszellen insbesondere für die Herstellung von Arzneimitteln zur Behandlung neurologischer, insbesondere neurodegenerative Erkrankungen, in Betracht.

Durch die erfindungsgemäße Verwendung können Zelltodprozesse, z.B. Kaskaden, die zur Apoptose führen, oder Prozesse, die zur Nekrose führen, in allen Zelltypen, die erfindungsgemäße Proteine der ee3-Familie oder eine native Variante hiervon exprimieren, insbesondere in neuralen Zellen, beeinflußt werden, bspw. durch Modulation von Zell-Zell-Interaktionen, insbesondere solche, an denen G-Protein gekoppelte Proteine beteiligt sind.

Die nativen erfindungsgemäßen Proteine, insbesondere die in den Ansprüchen genannten Proteine sind als Rezeptoren erfindungsgemäß Bestandteil von intrazellulären Signaltransduktionswegen, typischerweise als Start einer Signalkaskade, wobei dessen Fehlsteuerung für eine Vielzahl von Erkrankungen ursächlich ist. Insofern finden sich die vorgenannten erfindungsgemäßen Proteine insbesondere als Komponenten bei den folgenden zellulären Prozessen wieder und weisen zelluläre Funktionen z.B. auf bei: Signaltransduktion im allgemeinen, mit Wirkung auf Zelldifferenzierung, Zellteilung, Wachstum, Plastizität, Regeneration, Zelldifferenzierung, Proliferation oder Zelltod. Entsprechend kann typischerweise durch Infunktionalität von ee3_1 oder ee3_2, oder durch infunktionelle Expression oder durch Überexpression desselben ein pathophysiologischer Zustand ausgelöst werden, der mit einer Fehlsteuerung bspw. der Zelldifferenzierung, des Zellwachstums, der Zellplastizität oder der Zellregeneration einhergeht. Andererseits können auch andere Mechanismen zu pathophysiologischen Zuständen führen, bspw. eine Infunktionalität oder eine Überfunktionalität des/der nativen Liganden von erfindungsgemäßen ee3-Rezeptoren. Je nach molekularem Mechanismus der pathophysiologischen Störung kann zu therapeutischen Zwecken die Gabe funktionellen erfindungsgemäßen Proteins oder zumindest eine höhere Expression desselben oder aber eine Inhibition des zellulär überexprimierten oder des exprimierten infunktionellen Proteins erwünscht sein. Ganz besonders bevorzugt ist die Verwendung von erfindungsgemäßen Sequenzen, insbesondere Sequenzen mit den Nummern 1, 3, 5 im Zusammenhang mit deren Funktion beim neuronalen Zelltod, Excitation und Neurogenese. Aus diesen erfindungsgemäßen Erkenntnissen ergibt sich die Verwendung erfindungsgemäßer Sequenzen (Nukleotid- und Aminosäuresequenzen) sowie entsprechender Derivate (bspw. Peptide, Oligonukleotide oder Antikörper) zur Herstellung eines Arzneimittels zur Behandlung von Tumorerkrankungen und neurologischen Erkrankungen, insbesondere ischämische Zustände (Schlaganfall), multipler Sklerose, neurodegenerative Erkrankungen, wie bspw. Morbus Parkinson, Amyotrophe Lateralsklerose, heredodegenerative Ataxien, Neuropathien, Morbus Huntington, Epilepsien und Morbus Alzheimer. Aufgrund der Hochregulation im Falle erhöhter Erythropoetin-Expression kommt darüber hinaus die Verwendung erfindungsgemäßer Gegenstände für alle pathologischen Prozesse in Betracht, bei denen EPO eine (protektive) Rolle hat (z.B. dem Schlaganfall, und allen Formen akuter und chronischer Hypoxien).

Um auf auf der Basis molekularer Zusammenhänge zu weiteren Indikationen zu gelangen, erweisen sich Zell-basierte HTS-Assays zur funktionellen Rezeptor-Aktivierung, gemessen durch Enzymkomplementation, als geeignet. Der Assay basiert auf dem allgemeinen Regulationsmechanismus von GPCRs und mißt die Wechselwirkung zwischen dem aktivierten Rezeptor und beta-Arrestin. Dazu werden inaktive sich komplementierende beta-Galactosidasefragmente an den C-Terminus des Rezeptors und an beta-Arrestin fusioniert. Durch die Aktivierung des Rezeptors wird beta-Arrestin rekrutiert. Dadurch werden die zwei Hälften der beta-Galactosidase zusammengebracht so daß ein funktionierendes beta-Galactosidaseenzym entsteht, das entsprechende substrate umsetzen kann, die als Meßsignal dienen (ICAST System). Prinzipiell kann dieser Assay mit allen Enzymen durchgeführt werden, die als Fusionsproteine von zwei sich komplementierenden Hälften exprimiert werden können und eine durch gängige Meßmethoden erfaßbare Substratreaktion durchführen.

Im Zusammenhang mit der therapeutischen Anwendung erfindungsgemäßer Sequenzen steht die Verwendung einer erfindungsgemäßen Nukleinsäuresequenz oder Proteinsequenz zur Gentherapie bei Säugetieren, z.B. beim Menschen bzw. auch derartige gentherapeutische Verfahren. Gentherapie umfaßt dabei alle Therapieformen, die entweder erfindungsgemäße Sequenzen nach einem der Ansprüch 1 bis 3 in den Körper oder Teile davon, bspw. einzelne Gewebe, einbringen, oder die Expression von erfindungsgemäßer Sequenzen beeinflussen. Dazu können alle dem Fachmann geläufigen Modifikationen im Rahmen der Gentherapie verwendet werden, bspw. Oligonukleotide, z.B. antisense- oder Hybrid-RNA-DNA Oligonukleotide, mit beliebigen Modifikationen, die erfindungsgemäße Sequenzen enthalten, benutzt werden. Ebenfalls können virale Konstrukte, enthaltend eine erfindungsgemäße Sequenzen (dies schließt alle Varianten, wie Fragmente, Isoformen, Allele, Derivate ein) benutzt werden. Auch entsprechende erfindungsgemäße nackte DNA-Sequenzen, kommen im Rahmen der Gentherapie in Betracht. Ebenso können Nukleinsäurestilcke mit enzymatischer Aktivität (z.B. Ribozyme) für gentherapeutische Zwecke benutzt werden.

Neben den therapeutischen Anwendungen kommen auch diagnostische Verwendungen erfindungsgemäßer Nukleinsäuren oder Polypeptide, erfindungsgemäßer Proteinheteromere sowie davon abgeleiteter erfindungsgemäßer Reagenzien (Oligonukleotide, Antikörper, Peptide) in Betracht, bspw. zur Diagnose von menschlichen Erkrankungen oder von genetischen Prädispositionen, bspw. auch im Rahmen von Schwangerschaftsuntersuchungen. Bei diesen Erkrankungen oder Prädispositionen handelt es sich insbesondere um die oben im Zusammenhang mit therapeutischen Anwendungen, vor allem neurologische, immunologische oder Tumorerkrankungen, genannten Erkrankungen. Diese diagnostischen Verfahren können als in vivo, typischerweise jedoch ex vivo Verfahren ausgestaltet sein. Ex vivo wird eine typische Anwendung eines diagnostischen Verfahrens zum qualitativen und/oder quantitativen Nachweis einer erfindungsgemäßen Nukleinsäure in einer biologischen Probe dienen.

Neben therapeutischen und/oder diagnostischen Verwendungszwecken im Bereich der Human- und/oder Tiermedizin kommt auch die Verwendung erfindungsgemäßer Nukleinsäuren oder Polypeptide zum wissenschaftlichen Einsatz in Betracht. Insbesondere erlauben die erfindungsgemäßen Sequenzen auf des Fachmann bekannte Weise, bspw. über cDNA-Bibliotheken verwandte Sequenzen bei ein- oder mehrzelligen Organismen zu identifizieren oder aber im humanen Genom verwandte Sequenzen zu lokalisieren. Die erfindungsgemäßen Nukleotidsequenzen, insbesondere die Sequenzen mit der Numerierung 1 und 3 (einschließl. aller Varianten), können also dazu verwendet werden, Gene für mRNAs, die für diese Nukleinsäuren oder deren funktionellen Äquivalente, Homologe oder Derivate kodieren, im bspw. murinen oder anderen Tiergenomen und im menschlichen Genom mit gängigen Methoden durch Homologiescreening zu isolieren, zu kartieren und mit Markern für humane Erbkrankheiten zu korrelieren. Diese Vorgehensweise erlaubt bspw. die ursächliche Korrelation der chromosomalen Loci von Sequenzen der ee3-Familie beim Menschen (Chromosom 2 (2q14.2); X-Chromosom (Xq28, LocusID: 84548); Chromosom 5, Chromosom 8, Chromosom 3; Chromosom 7) zu bestimmten phänotypisch bekannten erblichen Erkrankungen, insbesondere auch Tumorerkrankungen (bspw. hepatozelluläres Carcinom), was ihre Diagnose erheblich vereinfacht und neue Therapieansätze ermöglicht. Gleiches gilt für die erfindungsgemäßen Proteine.

Mit Hilfe erfindungsgemäßer Nukleinsäuren lassen sich damit insbesondere humane Erbkrankheiten diagnostizieren, und zwar sowohl monogene als auch polygene Erkrankungen, weswegen sie als Marker Verwendung finden, wobei hieraus ein erfindungsgemäßes Diagnoseverfahren für erbliche Erkrankungen erwächst.

Insbesondere wird für die wissenschaftliche Anwendung ein Testsystem offenbart, das auf erfindungsgemäßen Aminosäure- und/oder Nukleotid-Sequenzen beruht. In diesem Zusammenhang können die cDNA, die genomische DNA, die regulatorischen Elemente der erfindungsgemäßen Nukleinsäuresequenzen, als auch das Polypeptid, sowie Teilfragmente davon in rekombinanter oder nicht-rekombinanter Form zur Ausarbeitung eines Testsystems verwendet werden. Ein solches Testsystem ist insbesondere geeignet, die Aktivität des Promotors oder des Proteins in Anwesenheit der Testsubstanz zu messen. Bevorzugt handelt es sich hierbei um einfache Meßmethoden (kolorimetrische, luminometrische, auf Fluoreszenz beruhende oder radioaktive), die die schnelle Messung einer Vielzahl von Testsubstanzen erlauben (Böhm, Klebe, Kubinyi, 1996, Wirkstoffdesign, Spektrum-Verlag, Heidelberg). Die beschriebenen Testsysteme erlauben das Durchsuchen von chemischen Bibliotheken nach Substanzen, die hemmende oder aktivierende Wirkungen auf erfindungsgemäße Proteine, insbesondere der Sequenzen 5 (bzw. deren Derivate oder Fragmente), haben. Die Identifizierung solcher Substanzen stellt den ersten Schritt auf dem Weg zur Identifizierung neuartiger Medikamente dar, die spezifisch auf die ee3-assoziierte Signaltransduktion wirken. Insbesondere werden hierbei Testsysteme zur Verfügung gestellt, die die bekannten Eigenschaften von G-Protein gekoppelten Proteinen benutzen, bspw. die weiter unten offenbarten Testsysteme.

Eine Inhibition der biologischen Aktivität von erfindungsgemäßem Protein, insbesondere von Proteinen gemäß Figuren 13, oder typischerweise der biologischen Aktivität im Zusammenhang mit der Apoptose, Proliferation, Regeneration, Zellwachstum, wie sie bspw. beim Schlaganfall, dem septischen Schock, GvHD (graft versus host disease), degenerativen Erkrankungen, insbesondere neurodegenerativen Erkrankungen, akuter Hepatitis oder anderen vorliegend offenbarten Indikationen erwünscht ist, kann auch dadurch erreicht werden, daß durch dem Fachmann geläufige Verfahren Oligonukleotide von typischerweise mindestens 10 Nukleotiden Länge, die für ein (Teilstück eines) Antisense-Strang(s) der nativen Sequenzen der Proteine mit der Sequenznummer 7A codieren, in die betroffenen Zellen einzuschleusen. Hierdurch wird die in den entsprechend transformierten Zellen die Translation der nativen mRNA von erfindungsgemäßen Proteinen der ee3-Familie, bspw. ee3_1 oder ee3_2 blockiert, was im Ergebnis vorzugsweise die Überlebensfähigkeit der transfizierten Zelle steigert oder modulierend auf Zellwachstum, Zellplastizität, Zellproliferation wirkt. Auch in diesem Fall kann das oben beschriebene Verfahren mit Hilfe rekombinanter Viren eingesetzt werden.

Die ggf. pathologisch gesteigerte Zellapoptose, Zellproliferation bei Erkrankungen, die auf einer entsprechenden Fehlsteuerung erfindungsgemäßer Sequenzen beruhen (bspw. bei den vorgenannten Indikationen), kann auch durch Ribozym-Methoden therapiert werden. Hierzu werden Ribozyme verwendet, die eine Ziel-mRNA schneiden können.

Neben den vorgenannten Möglichkeiten zur Modulation der biologischen Funktion erfindungsgemäßer Genprodukte, insbesondere der Genprodukte gemäß Figuren 13, oder typischerweise der Modulation der Funktion erfindungsgemäßer Genprodukte bei der apoptotischen oder nekrotischen, proliferativen oder wachstumsindizierenden Signaltransduktion ist dies auch mit Hilfe einer chemischen Verbindung Eine solche chemische Verbindung wird insbesondere die intrazelluläre Funktion der erfindungsgemäßen Proteine der ee3-Familie modulieren, typischerweise inhibieren oder auch aktivieren, oder auf der Ebene der zugrundeliegenden erfindungsgemäßen DNA-Sequenzen, bspw. durch Bindung an die DNA (bspw. den Promotorbereich) oder durch Bindung an einen der ein erfindungsgemäßes Gen steuernden Transkriptionsfafktoren, die biologische Funktion beeinflussen. Solche Verbindungen werden typischerweise spezifisch an ein erfindungsgemäßes Protein bzw. an eine erfindungsgemäße Nukleinsäuresequenz binden und dadurch eine pharmakologische, insbesondere neuroprotektive oder immunmodulierende oder antiapoptotische oder antiproliferative Wirkung, hervorrufen.

Über Strukturanalysen eines erfindungsgemäßen Proteins lassen sich gezielt Verbindungen finden, die eine spezifische Bindungsaffinität aufweisen (Rationales Drug Design (Böhm, Klebe, Kubinyi, 1996, Wirkstoffdesign, Spektrum-Verlag, Heidelberg)). Hier wird die Struktur oder eine Teilstruktur, Derivat, Allel, Isoform oder ein Teil einer solchen von einem der erfindungsgemäßen Proteine über NMR- oder Röntgenkristallographie-Verfahren (nach entsprechender Kristallisierung, z.B. nach der Methode des "hängenden Tropfens") ermittelt oder, sofern eine solche hochaufgelöste Struktur nicht vorliegt, mit Hilfe von Strukturvorhersage-Algorithmen ein Strukturmodell eines erfindungsgemäßen Proteins, bspw. auch mit Hilfe von homologen bereits strukturell aufgeklärten Proteinen (z.B. von Rhodopsin), erstellt, und diese(s) benutzt, um mit Unterstützung von Molecular Modelling Programmen Verbindungen, die als Agonisten oder Antagonisten wirken können, zu identifizieren, für die sich eine hohe Affinität zum erfindungsgemäßen Protein vorhersagen läßt. Ggf. Lassen sich die oben bezeichneten Verfahren zur Strukturaufklärung auch miteinander kombinieren. Geeignete Kraftfelder werden zur Simulation der Affinität einer potentiell affinen Verbindung an eine interessante Substrukur eines erfindungsgemäßen Proteins, bspw. das aktive Zentrum, eine Bindungstasche oder eine "hinge"-Region, eingesetzt. Diese Substanzen werden dann synthetisiert und in geeigneten Testverfahren auf ihr Bindungsvermögen und ihre therapeutische Nutzbarkeit getestet. Derartige in silico Verfahren zur Identifizierung potentieller Wirkstoffe, die ihre Wirkung durch Bindung an erfindungsgemäße ee3-Proteine entfalten, sind gleichfalls Gegenstand der vorliegenden Erfindung.

Die Kenntnis der Primärsequenz von ee3 Proteinen kann benutzt werden, um rekombinante Konstrukte herzustellen, die die Eigenschaften schon charakterisierter und nach dem Stand der Technik bekannter GPCRs ausnutzen. So können z.B. bestimmte Sequenzbereiche von erfindungsgemäßen ee3-Proteinen gegen bestimmte Sequenzbereiche eines bekannten, gut charakterisierten GPCRs ausgetauscht werden. Das resultierende Konstrukt kann herangezogen werden, um mit bekannten Liganden oder Agonisten, die G-Proteinkopplung, und die benutzten second messenger Systeme zu identifizieren, oder bekannte G-Proteinkopplung für die Auffindung von Liganden, Agonisten oder Antagonisten zu benutzen. Die Herstellung chimärer Rezeptoren bspw. zu den vorgenannten Verwendungen kann bspw. nach einem Verfahren, wie von Kobilka et al. Beschrieben , durchgeführt werden (Kobilka BK, Kobilka TS, Daniel K, Regan JW, Caron MG, Lefkowitz RJ (1988) Chimeric alpha 2-,beta 2-adrenergic receptors: delineation of domains involved in effector coupling and ligand binding specificity. Science 240:1310-1316).

Weiterhin können konstitutiv aktive Rezeptormutanten der ee3-Familie zur Charakterisierung der Wirkung dieser Rezeptoren auf Signaltransduktionswege und zum "Screening" nach Liganden eingesetzt werden.Erfindungsgemäße Rezeptoren als Vertreter der 7TM-Proteinklasse können auf bestimmte Weise mutiert werden, um Veränderungen des physiologischen und pharmakologischen Verhaltens dieser Rezeptoren hervorzurufen. Dies kann beispielsweise dazu benutzt werden, intrazelluläre Signalwege zu identifizieren, wenn der natürliche Ligand oder ein Agonist unbekannt sind. Insbesondere sind Mutationen in der DRI consensus Sequenz von ee3-Proteinen geeignet, solche Veränderungen herbeizuführen, bspw. die Mutation von R in der DRY Sequenz (Scheer A, Costa T, Fanelli F, De Benedetti PG, Mhaouty-Kodja S, Abuin L, Nenniger-Tosato M, Cotecchia S (2000) Mutational analysis of the highly conserved arginine within the Glu/Asp- Arg-Tyr motif of the alpha(1b)-adrenergic receptor: effects on receptor isomerization and activation. Mol Pharmacol 57:219-231)) zu Lys, His, Glu, Asp, Ala, Asn, und Ile führt im Falle der Mutation zu Lys zu einer starken Zunahme der konstitutiven Aktivierung. Mutation zu His oder Asp führen zu einer geringeren Zunahme der konstitutiven Aktivierung. Interessanterweise führt die Mutation zu Arg zu einer Zunahme der Agonistenaffinität, so daß auch solche Mutanten für HTS screens interessant sind

Ähnlich ist ein konserviertes Arg in der dritten TM-Domäne ein möglicher Mutationsort (Ballesteros J, Kitanovic S, Guarnieri F, Davies P, Fromme BJ, Konvicka K, Chi L, Millar RP, Davidson JS, Weinstein H, Sealfon SC (1998) Functional microdomains in G-protein-coupled receptors. The conserved arginine-cage motif in the gonadotropin-releasing hormone receptor. J Biol Chem 273:10445.-10453).

Alternativ können zur Identifikation von endogenen oder surrogaten Liganden Verfahren eingesetzt werden, die auf der Verwendung immobilisierter funktioneller erfindungsgemäßer Rezeptoren beruhen. Erfindungsgemäße Rezeptoren der ee3-Familie werden hierbei als Fusionsproteine mit GST, dem Flag-Tag oder dem TAP-Tag exprimiert. Die entsprechenden Zellen werden entweder nach gängigen Methoden zu Membranen verarbeitet oder direkt zur Solubilisation eingesetzt. Mit geeigneten Detergenzien, z.B. Dodecylmaltosid, Digitonin, Cholat oder Detergenzmischungen, werden die Rezeptoren solubilisiert und an die entsprechenden Affinitätsmatritzen wie GST-Sepharose, Anti-Flag-M2-Agarose oder IgG-Sepharose etc. gebunden. Nach Waschen der Matritzen werden sie mit Gewebsextrakten oder Zellüberständen inkubiert und wieder gewaschen. Enthält der Extrakt einen aktiven Liganden, z.B. ein Peptid, so bindet dieser an den immobiliserten Rezeptor und kann nach Elution durch analytische Methoden identifiziert werden, z.B. mittels Massenspektrometrie.

Sog. Internalisierungsassays stellen eine weitere Vorgehensweise dar, um natürlichen oder insbesondere Surrogatliganden für erfindungsgemäße Rezeptoren, bspw. ee3_1, identifizieren zu können. Hierbei werden ebnfalls die unterschiedlichen Eigenschaften eines Proteins der GPCR-Klasse ausgenutzt. Bspw. Kann das Internalisierungsverhalten von Proteinen der GPCR-Klasse herangezogen werden. Dies ist als Regulationsmechanismus nach Aktivierung des Rezeptors zu vertehen. Der Vorteil einer "Screening"-Methode die auf diesem Verhalten aufbaut, ist, daß eine genauere Kenntnis der Physiologie des jeweiligen Rezeptors nicht nötig ist. Insbesondere muß keine Kenntnis über die koppelnden G-Proteine, und die benutzten Signaltransduktionswege vorhanden sein.

Ein solcher Assay wird z.B. von Lenkei et al. (2000, J Histochem Cytochem, 48, 1553-64) beschrieben und kann analog bei den erfindungsgemäßen Rezeptoren eingesetzt werden. Hierzu wird zunächst ein C-terminals Fusionskonstrukt der erfindungsgemäßen Proteins mit EGFP hergestellt. Danach werden stabile CHO-Zellen hergestellt nach Standardverfahren. Stabile Klone werden mit Hilfe eines FACS-Sorters nach EGFP-Fluoreszenz selektiert. Die endgültige Auswahl erfolgte mit Hilfe fluoreszenzmikroskopischer Beurteilung auf Oberflächenexpression. Die Zellen werden danach mit HPLC-Fraktionen von Gewebeextrakten inkubiert, und die Internalisierung mit Hilfe eines Konfokalen Mikroskops bestimmt. Die Auswertung erfolgt mit Hilfe morphometrischer Software (NIH Image) nach dem Prinzip der Distanz fluoreszenter Signale vom Zellmittelpunkt. Eine Häufigkeits/Distanzverteilung ergab eine gute Diskrimination für die Internalisierung.

Zur Auffindung unbekannter Liganden werden sukzessive Fraktionierungen durchgeführt, und das entsprechende Peptid bis zur Reinheit zu isolieren, und danach bspw. durch Sequenzierung oder MALDI-TOF zu identifizieren. Eine andere Anwendung des prinzipiell gleichen Verfahrens wird von Ghosh et al. (2000, Biotechniques, 29, 170-5; Conway et al., 1999, J Biomol Screen, 4, 75-86) beschrieben, die beide vollinhaltlich Bestandteil der vorliegenden Offenbarung sind.

Aber auch ein funktioneller Ca-Assay kann zur Identifizierung von Liganden und/oder ggf. auch zur Charakterisierung des erfindungsgemäßen Rezeptors herangezogen werden. Hierbei macht man sich zunutze, daß eine Vielzahl von 7TM-Rezeptoren (Rezeptoren mit 7 Transmembran-Domänen), die in HEK293 Zellen, in CHO Zellen oder anderen Zellen produziert werden, über die Kopplung an G Proteine der Gq Klasse zur Aktivierung der PLC und zur Mobilisation von intrazellulärem Ca führen. Für den Fall, daß gewisse erfindungsgemäße Rezeptoren nicht an G Proteine der Gq Klasse koppeln sollten, können diese durch Co-expression von chimären G Proteinen oder den relativ unspezifisch mit Rezeptoren koppelnden G Proteinen G15 oder G16 zur Signaltransduktion über PLC, d.h. zur Ca-Freisetzung, gezwungen werden. Die erfindungsgemäßen Rezeptoren der ee3-Familie werden typischerweise in HEK293 und in CHO Zellen sowohl stabil als auch transient allein und zusammen mit dem chimären G Protein Gqi5 und alternativ mit dem G Protein Gq15 exprimiert. Die Zellen werden dann vorzugsweise mit einem membranpermeablen Cabindenden Fluoreszenzfarbstoff, z.B. Fura-2 oder Fluo-3 bzw. -4 beladen und nach dem Waschen der Zellen mit verschiedenen Testsubstanzen versetzt und gleichzeitig die Ca-Freisetzung gemessen, z.B. mit einem FLIPR Gerät der Firma Molecular Devices. Testsubstanzen, die ein positives Signal ergeben, werden schließlich vorzugsweise in Kontrollzellen (nur mit dem Vektor transfiziert) getestet und wenn das Signal sich als spezifisch herausstellt pharmakologisch, d.h. mit Konzentrations-Response-Kurven charakterisiert.

Alternativ kann jedoch die durch einen Liganden hervorgerufene Ca-Antwort auch durch andere Ca-Detektoren gemessen werden, z.B. über AequoScreen von Euroscreen (Brüssel, Belgien; siehe z.B.http://www.pharmaceuticaltechnology.com/contractors/compound_man/euroscreen/). Dabei werden Zellen eingesetzt, die das Gen des Proteins Apoaequorin exprimieren. Nach beladung der Zellen mit Coelenterazin, das an Apoaequorin bindet, entsteht Aequorin. Wird durch einen Liganden Ca freigesetzt, aktiviert das Ca das Aequorin zur Oxidation von Coelenterazin, wodurch Licht freigesetzt wird. Die Intensität der Lichtemission ist der Erhöhung der intrazellulären Ca-Konzentration proportional und damit ein Maß für die Aktivität des gefundenen Liganden (unter Berücksichtigung der entsprechenden Kontrollen).

Um Antagonisten zu identifizieren, werden die Rezeptoren in Gegenwart genügend hoher Konzentrationen verschiedenster Liganden mit einem bekannten Agonist stimuliert. Ein gegenüber der Kontrolle (nur Agonist, ohne weiteren Liganden) verändertes Signal, bspw. ein niedrigeres Ca-Signal, deutet auf einen kompetitiven Antagonisten hin.

Weiterhin können auch cAMP-Assays zur Charakterisierung der erfindungsgemäßen Rezeptoren der ee3-Familie und zur Identifikation von Liganden dienen. Hintergrund dieses Ansatzes zur pharmakologischen Charakterisierung von ee3-Rezeptoren bzw. zur Identifizierung von Liganden (Agonisten oder Antagonisten) ist die Eigenschaft von Rezeptoren der Klasse der GPCRs, bspw. also von erfindungsgemäßen Proteinen, entweder stimulierend oder inhibierend auf Adenylatzyklasen wirken zu können, in der Regel durch Aktivierung von sog. stimulierenden Gs oder inhibierenden Gi Proteinen. Abhängig von der Wirkung von Testsubstanzen, bspw. in einem Hochdurchsatz-"Screening", kann über direkte oder indirekte Messungen die damit verbundene Änderung des cAMP-Spiegels in der Zelle untersucht werden werden. Die Rezeptorgene werden hierbei stabil oder transient in Säugerzellen exprimiert (s. Ausführungsbeispiel 2). Bei GPCRs, die Adenylatzyklasen aktivieren, wodurch der cAMP Spiegel in der Zelle steigt, wird ein potentieller Agonist unter den Testsubstanzen durch eine gegenüber Kontrollzellen erhöhte cAMP-Konzentration identifiziert. Antagonisten unter den Testsubstanzen, bespw. In einem HTS-Ansatz, werden durch ihre Blockierung der durch einen Agonisten hervorgerufenen Erhöhung der cAMP-Konzentration identifiziert. Bei Gi-gekoppelten erfindungsgemäßen ee3-Rezeptoren wird im Test die Adenylatzyklase entweder direkt mit Forskolin oder durch Aktivierung eines Gsgekoppelten Rezeptors stimuliert, wodurch der cAMP-Spiegel steigt. Ein Agonist des Gi-gekoppelten Rezeptors hemmt diesen Anstieg. Für direkte cAMP-Messungen können eine Anzahl käuflicher Assays, wie bspw. des cAMP^{[3H]}Assay-Systems der Firma Amersham, verwendet werden, die z.B. auf dem Prinzip der kompetitiven Verdrängung von endogen gebildetem cAMP durch zugegebenes radioaktiv markiertes (Tritium) cAMP beruhen. Indirekte cAMP-Messungen werden in der Regel durch Reporter-Assays durchgeführt. Dazu werden die Rezeptoren in Zellinien exprimiert, die Reportersysteme enthalten, z.B. das CRE-Luziferase-System. cAMP aktiviert die Expression der Luziferase, deren Aktivität durch Umsetzung entsprechender Substrate und luminometrische Messung der Produkte gemessen wird. Reporter-Assays eignen sich ganz besonders für Massen-Screening-Methoden.

Schließlich sind - neben den vorangehend beschriebenen Assays - auch die folgenden Assay-Systeme zur Charakterisierung von second-messenger Systemen erfindungsgemäßer Rezeptoren bzw. zur Identifizierung von Liganden erfindungsgemäßer ee3-Rezeptoren möglich, insbesondere zur Bestimmung der Adenylatzyklase-Aktivität in Zellen oder Membranen nach Salomon (Salomon et al., (1979). Adv. Cyclic Nucleotide Res. 10, 35-55), zur Bestimmung der Inositol-3-phosphat-Konzentration oder zur Messung einer veränderten Arachidonsäurefreisetzung. Beispielsweise kann ee3_1 in gängigen Zellinien überexprimiert werden, und nach Aktivierung durch Gewebeextrakte kann die Aktivität der o.a. second messenger Systeme bestimmt werden. Im einzelnen sind Assays für second messenger Systemen von GPCR-Klasse dem Fachmann wohl bekannt, und im Einzelfall der Literatur zu entnehmen, z.B. Signal Transduction: A practical approach, G. Milligan, Ed. Oxford University Press, Oxford, England. Weitere Reporter-Assays zum "Screening" umfassen MAP kinase/Luziferase und NFAT-Luziferase Systeme.

Beruhend auf der erfindungsgemäßen Erkenntnis, daß die Signalweiterleitung des ee3-Rezeptors auch über MAP-Kinase Signaltransduktionswege erfolgt, kann auch dazu verwendet werden, "Screening Assays" für Ligandensuche oder Inhibitorenidentifikation aufzubauen, z.B. über einen NF-kB-Reportersysteme oder Luciferase-Systeme.

Wie oben erwähnt, dient die Aktivierung von second messenger auch zur Identifikation von Liganden, Agonisten oder Antagonisten, die an erfindungsgemäße Rezeptoren binden und derart ihre agonistische und/oder antagonistische Wirkung für gewisse zelluläre Prozesse entfalten können. Bspw. können Microphysiometern zur Identifikation von Liganden, Agonisten, oder Antagonisten eingesetzt werden. Durch Ligandenbindung an einen Rezeptor der ee3-Familie ausgelöste Signale stellen energieverbrauchende Prozesse dar. Deshalb gehen derartige Vorgänge immer mit geringen metabolischen Veränderungen, unter anderem einer geringen pH Verschiebung, einher. Diese können extracellulär von bspw. einem Microphysiometer (Cytosensor, Molecular Devices) erfaßt werden.

Nach Identifizierung von Liganden, Agonisten oder Antagonisten mit Bindungspotential an erfindungsgemäße Proteine der ee3-Rezeptorfamilie können zu deren näherer Charakterisierung Ligandenbindungstests durchgeführt werden. Ligandenbindungstests ermöglichen in direkter Weise die Pharmakologie eines Rezeptors, d.h. die Affinität verschiedenster Liganden für diesen Rezeptor, zu messen. Für Bindungsstudien wird hierbei typischerweise ein nach einem der vorgenannten Verfahren identifizierter oder auf andere Weise bekannter chemisch reiner Ligand mit einer hohen spezifischen Aktivität (30-2000 Ci/mmol) radioaktiv markiert, so daß die radioaktive Markierung die Aktivität des Liganden bezüglich des Rezeptors nicht verringert. Die Testbedingungen werden sowohl für die Verwendung von den Rezeptor exprimierenden Zellen wie auch von daraus hergestellten Membranen bezüglich Pufferzusammensetzung, Salz, Modulatoren wie z.B. Nukleotiden oder Stabilisatoren, wie z.B. Glyzerin, so optimiert, daß die Messung ein brauchbares Signal-zu-Hintergrund Verhältnis ergibt. Für diese Bindungstests wird die spezifische Rezeptorbindung definiert als die Differenz von der gesamten mit der Rezeptorpräparation (Zellen oder Membranen) assoziierten Radioaktivität, d.h. gemessen in Gegenwart von nur einem spezifischen, nämlich des Radioliganden und der Radioaktivität, die in Gegenwart sowohl des Radioliganden als auch eines Überschusses an nicht radioaktiv markiertem Ligand gemessen wird. Der nicht markierte Ligand verdrängt dabei kompetitiv den Radioligand. Wenn möglich, werden mindestens zwei chemisch verschiedene kompetitierende Liganden verwendet, um die nicht spezifische Bindung festzulegen. Eine spezifische Bindung die mindestens 50% der Gesamtbindung beträgt, ist optimal. Der Bindungstest wird entweder inhomogen als Filtrationstest durchgeführt oder homogen als "Scintillation Proximity Assay".

Im ersten Fall wird die Rezeptor enthaltende Präparation (Zellen oder Membranen) mit den Liganden in einer geeigneten Pufferlösung inkubiert bis sich das Bindungsgleichgewicht eingestellt hat, typischweise 1 h bei RT oder bei 4°C über Nacht, und dann über geeignete Filter, z.B. Whatman oder Schleicher&Schuell Glasfaserfilter, die gegebenenfalls vorbehandelt wurden, z.B. mit Polyethylenimin, abfiltriert, um den nicht-gebundenen von dem gebundenen Radioliganden zu trennen. Nach Waschen der Filter werden diese getrocknet oder feucht mit geeignetem Szintillator versetzt und nach eventuell nötiger Inkubation im Szintillationszähler die enthaltene Radioaktivität gemessen. Beim "Scintillation Proximity Assay" werden geeignete Szintillation-Kügelchen, z.B. WGA-Kügelchen, mit den Liganden und Rezeptor enthaltenden Membranen in geeigneter Pufferlösung inkubiert, bis sich das Bindungsgleichgewicht eingestellt hat, und dann die Radioaktivität in einem geeigneten Szintillationszähler gemessen. Beide Bindungstests sind im HTS-Format durchführbar.

Solubiliserte oder gereinigte Rezeptoren werden mit dem "Scintillation Proximity Assay" gemessen oder mit gängigen inhomogenen Tests wie dem Filtrationstest nach PEG-Fällung, dem Adsorptions- oder dem Gelfiltrationstest (Hulme E, Birdsall N (1986) Distinctions in acetylcholine receptor activity. Nature 323:396-397).

Anstelle eines Radioliganden kann auch ein fluoreszierender Ligand, z.B. ein Ligand der kovalent einen fluoreszierenden Farbstoff wie BODIPY gebunden hat, eingesetzt werden. Die Bindung des fluoreszierenden Liganden an den Rezeptor wird mittels Fluoreszenzpolarisation gemessen. Die Methode eignet sich sowohl für primäre Screenings im HTS-Format wie auch in Sekundärtests.

Ein weiterer Gegenstand der vorliegenden Erfindung betrifft Verfahren zur Identifizierung von zellulären Interaktionspartnern von erfindungsgemäßen Polypeptiden aus der ee3-Familie. Auf diese Weise können als Interaktionspartner Proteine identifiziert werden, die zum erfindungsgemäßen Protein spezifische Bindungsaffinitäten aufweisen, oder zur Identifizierung von Nukleinsäuren, die für Proteine kodieren, die zum erfindungsgemäßen Protein spezifische Bindungsaffinitäten aufweisen. Zelluläre Interaktionspartner von Proteinen der erfindungsgemäßen ee3-Proteins können z.B. andere GPCRs oder Ionenkanäle sein.

Ein derartiges erfindungsgemäßes Verfahren bzw. die Verwendung erfindungsgemäßer Polypeptide, erfindungsgemäßer Nukleinsäuresequenzen und/oder erfindungsgemäßer Nukleinsäurekonstrukte zur Durchführung derartiger Verfahren wird mit Hilfe einer "Yeast-two-hybrid"-Durchmusterung (y2h-"Screens") allein oder in Kombination mit anderen biochemischen Verfahren durchgeführt (Fields and Song, 1989, Nature, 340, 245-6). Derartige Screens finden sich auch bei Van Aelst et al. (1993, Proc Natl Acad Sci U S A, 90, 6213-7) und Vojtek et al. (1993, Cell, 74, 205-14) beschrieben. Typischerweise können anstelle von Hefesystemen auch Säugetiersysteme zur Durchführung eines erfindungsgemäßen Verfahrens verwendet werden, bspw. wie bei Luo et al. (1997, Biotechniques, 22, 350-2) beschrieben. Die entsprechenden vorgenannten experimentellen Ansätze werden dabei typische Eigenschaften der Klasse der GPCR-Proteine nutzen, bspw. die Signaltransduktion, z.B. über G-Proteine, also bspw. auch die bekannten intrazellulären Interaktionspartner.

Für einen y2h-Screen wird das offene Leseraster von erfindungsgemäßen Sequenzen, insbesondere von Sequenzen mit den Nummern 1 bis 4, oder einer nativen Variante, ganz besonders bevorzugt intrazelluläre Bereiche von erfindungsgemäßen Sequenzen, bspw. in einen sog. bait-Vektor "in-frame" mit der GAL4-Bindungsdomäne kloniert (z.B. pGBT10 oder pGBKT7, Fa. Clontech). Damit kann vorzugsweise eine sog. "prey-library" in einem Hefestamm nach gängigem Protokoll auf interagierende Proteine durchsucht werden.

Darüber hinaus können mit y2h-Systemen auch sog. Mapping-Experimente durchgeführt werden, um spezifische Interaktionsdomänen zu identifizieren.

Gleichwertig bevorzugt sind auch two-hybrid-Systeme, die andere Fusionspartner oder andere Zellsysteme benutzen, z.B. das BacterioMatchsystem der Fa. Stratagene, oder das CytoTrapsystem der Fa. Stratagene. Erfindungsgemäß können alternativ zu den y2h-Verfahren auch entsprechende Systeme von Säugetierzellen verwendet werden, wie bspw. bei Luo et al. (1997, Biotechniques, 22, 350-2) als Bestandteil der vorliegenden Offenbarung beschrieben.

Die Hochregulation von bspw. ee3_1 durch EPO zeigt, daß bspw. ee3_1 mit dem überleben von Zellen in Zusammenhang steht, da EPO neuroprotektive Wirkungen hat. Die erfindungsgemäßen Polypeptide, insbesondere nativ auftretende Formen oder aber auch nicht-native, artifiziell erzeugte Varianten, deren biologische Funktion untersucht werden soll, können daher in einem Apoptoseassay verwendet werden bzw. in einem Verfahren zur Untersuchung der Funktion und/oder Wirksamkeit erfindungsgemäßer Polypeptide bei Induktion, Transduktion oder Inhibition von Zelltodsignalen oder anderen zellphysiologischen Prozessen zum Einsatz kommen. Die Beteiligung von erfindungsgemäßen Proteinen der ee3-Familie oder vorgenannten erfindungsgemäßen Varianten an bspw. apoptotischen Kaskaden kann untersucht werden, indem Expressionskonstrukte mit erfindungsgemäßen ee3-Sequenzen, insbesondere Sequenzen mit den Nummern 1 und 3 oder Varianten in eukaryontische Zellen transfiziert werden (weswegen auch deren Verwendung für derartige Untersuchungen offenbart wird), und danach die Induktion von Apoptose untersucht werden kann. Dies kann z.B. durch Anfärbung mit Annexin geschehen (Fa. Roche Diagnostics), durch Antikörper, die die aktive Form von Caspase-3 erkennen (Fa. New England Biolabs), oder durch ELISAs, die DNA-Histon-Bruchstücke erkennen (cell-death elisa, Roche Diagnostics). Diese Induktion von Apoptose ist ggf. zelltyp-spezifisch, weswegen erfindungsgemäß vorzugsweise mehrere Zelllinien und primäre Zellen untersucht werden. Die Induktion von Apoptose kann ggf. auch Stimulus-spezifisch sein. Daher werden in einem Solchen Verfahren vorzugsweise mehrere Streß-Situationen zugrundegelegt, z.B. Hitzeschock, Hypoxiebedingungen, Cytokinbehandlungen (z.B. IL-1, IL-6, TNF-alpha) oder H₂O₂-Behandlung. Als typische Zelltypen für ein derartiges Verfahren kommen gebräuchliche Zellinien, z.B. Cos-Zellen, HEK-Zellen, PC12-Zellen, THP-1-Zellen, oder primäre Zellen, wie z.B. Neurone, Astrozyten, in Betracht, ebenso wie andere immortalisierte und primäre Zellinien nach Bedarf.

Zusammenfassend ist festzustellen, daß eine eine neue Familie von membranständigen G-Protein-gekoppelten Rezeptoren (GPCRs) im Säugetiersystem identifiziert wurde, die sich von den den aus dem Stand der Technik bekannten Familien deutlich abgrenzen läßt. Die Identifizierung einer neuen Proteinklasse und der zugrundeliegenden DNA-Sequenzen erfolgte aufgrund einer differentiellen Regulation derselben im zentralen Nervensystem und erlauben die Aufklärung und Charakterisierung einer Vielzahl physiologischer und pathophysiologischer Prozesse.

Die Identifizierung gelang durch die EPOinduzierte (auf direkte oder indirekte Weise) transkriptionelle Hochregulation des erfindungsgemäßen Proteins ee3_1. Dies bedeutet, daß bspw. Agonisten und Antagonisten von ee3_1 Wirkungen von EPO verstärken, ersetzen oder unerwünschte Wirkungen antagonisieren können. Möglicherweise sind bestimmte Wirkungen von EPO selektiv zu beeinflussen, z.B. eine neuroprotektive Wirkung (z.B bei neurodegenerativen Erkrankungen), oder eine Hirnleistungssteigerung, (z.B bei Demenzen).

Das vorgestellte Gen ist ein neues 7-Transmembran-Protein bei Maus und Mensch, die v.a. im Gehirn exprimiert wird. Es handelt sich um einen G-Protein-gekoppelten Rezeptor. Eine Homologiesuche in der EMBL Sequenzdatenbank ergab eine entfernte Ähnlichkeit zu GPCRs der Familie A, insbesondere Peptidrezeptoren.

Darüber hinaus wird ee3_1 nicht oder nur eingeschränkt durch folgende neurologische Krankheitsmodelle reguliert: Kindling (Hippocampus, seizure stage 5, 2 h post seizure), Kortikaler Schlaganfall (Cortex, 2.5 h occlusion und 2 und 6h Reperfusion), globale Ischämie in Ratten (Gesamthirn, 3 und 6 h post Ischämie). Dies zeigt eine hohe Spezifität der Regulation durch EPO, im Gegensatz z.B. zu "immediate early"- Genen.

Die vorliegende Erfindung wird durch die nachfolgenden Figuren näher erläutert:

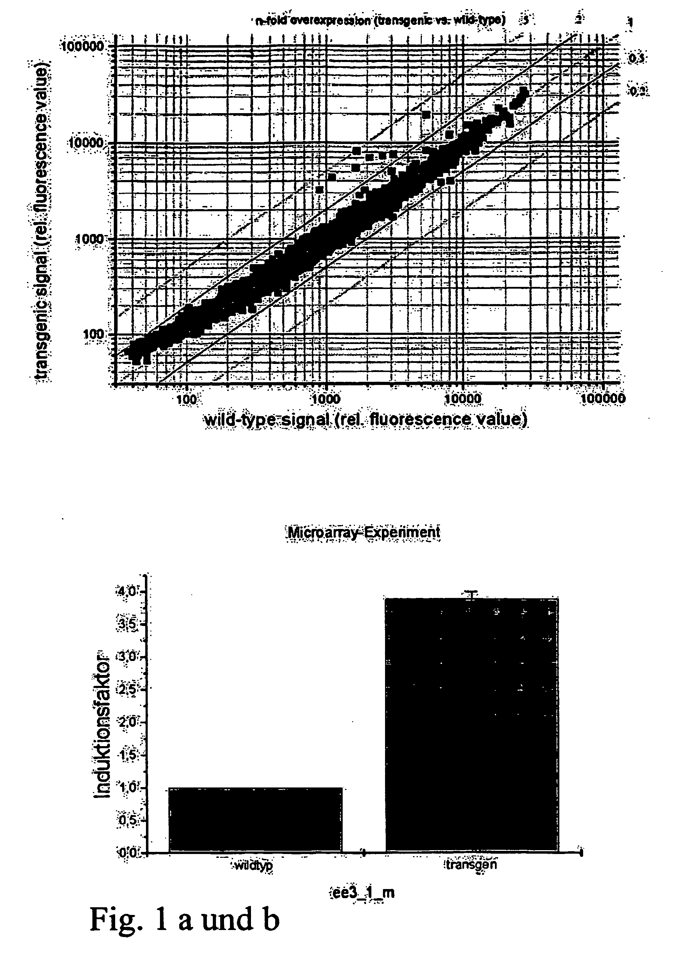

Fig. 1a zeigt eine Darstellung der Transkriptionsanalyse im Gehirn von Epo-Mäusen. Die Graphik stellt die Daten eines DNA-Array-Hybridisierungsexperimentes dar. Aufgetragen ist das Signal bei den EPO-transgenen Tieren (y-Achse) gegenüber dem Signal bei Wildtyp-Mäusen (x-Achse). Bei dem Signal handelt es sich um ein Fluoreszenzsignal (relativ). Die Punkte oberhalb der Diagonalen repräsentieren hochregulierte Genprodukte im Gehirn von EPO-transgenen Tieren. Oberhalb der Diagonale 2 (2-fache Überexpression im transgenen Tier gegenüber dem WT sind acht positive Signale zu beobachten. Fig. 1b stellt die Ergebnisse von Microarray-Experimenten dar. Es handelt sich um eine Auftragung des Induktionsfaktors (rel.) von Mäuse-ee3_1 im Gehirn von EPO-transgenen Mäusen (rechts) in Relation zur Induktion im Gehirn von WT-Tieren, und zwar als gemittelte Induktionswerte aus 2 unabhängigen Hybridisierungsexperimen-ten. Ein Induktionsfaktor von 1 entspricht der Konzentration im Gehirn der Littermate-Kontrolltiere. Die Expression einer erfindungsgemäßen Sequenz erreicht fast den vierfachen Wert.

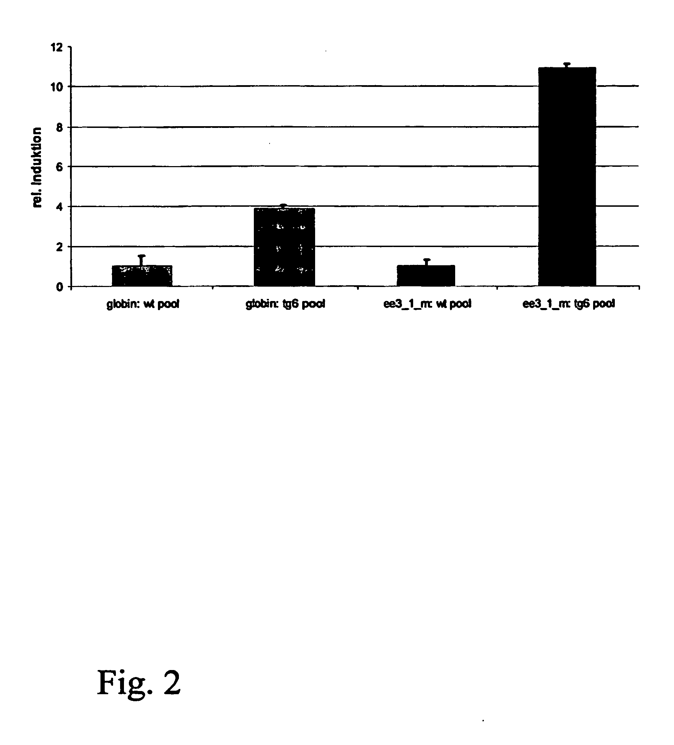

Fig. 2 zeigt die Resultate von Experimenten mit Mäusen, die zur Verifikation der erhöhten Induktion von erfindungsgemäßern ee3_1 im EPO-transgenen Tier und des ebenfalls im EPO-transgenen Tier hochregulierten Genproduktes alpha-Globin herangezogen wurden, und zwar mit Hilfe der quantitativen PCR (LightCycler). Die Daten repräsentieren gepoolte RNA-Proben aus 6 Hirnen (transgen (tg) oder Wildtyp (wt)). Auf der y-Achse ist die relative Induktion aufgetragen. Gegenüber der Kontrollmessung (rel. Induktion =1) ergibt sich für die erfindungsgemäße Sequenz eine 11-fache Erhöhung der Induktion.

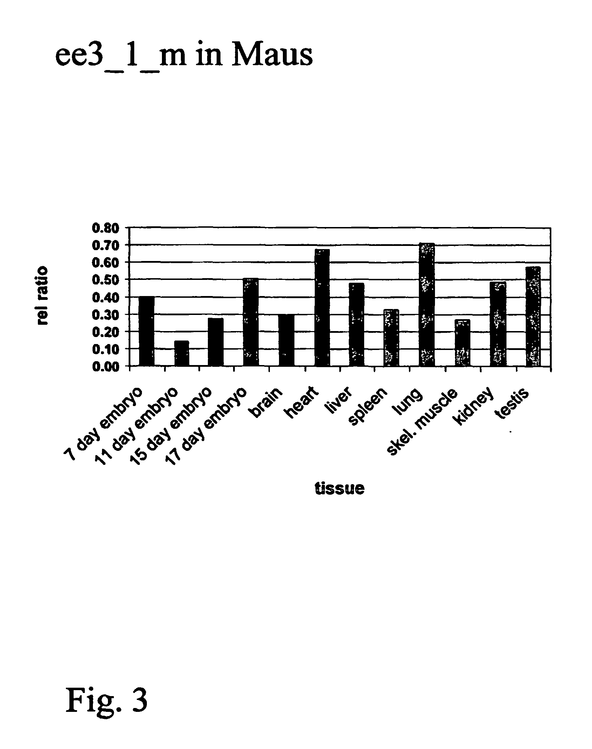

Figur 3 gibt die Expression von erfindungsgemäßem ee3_1 in der Maus (LightCycler) während der Entwicklung (Embryo nach 7, 11, 15 und 17 Tagen) und in verschiedenen adulten Geweben wieder (Gehirn, Herz, Leber, Niere, Lunge, Skelettmuskel, Milz und Testis). Aufgetragen auf der y-Achse ist die relative Abundanz von ee3 Transkripten. Im Ergebnis zeigt sich eine relativ ubiquitäre Expression von ee3_1 in allen Stadien der murinen Embryonalentwicklung und in allen untersuchten Geweben.
   ee3_2 wird in der Maus nach EST-Daten in embryonalem Carcinom, Niere, Leber, B-Zellen, Lunge, Mamma und Uterus exprimiert.

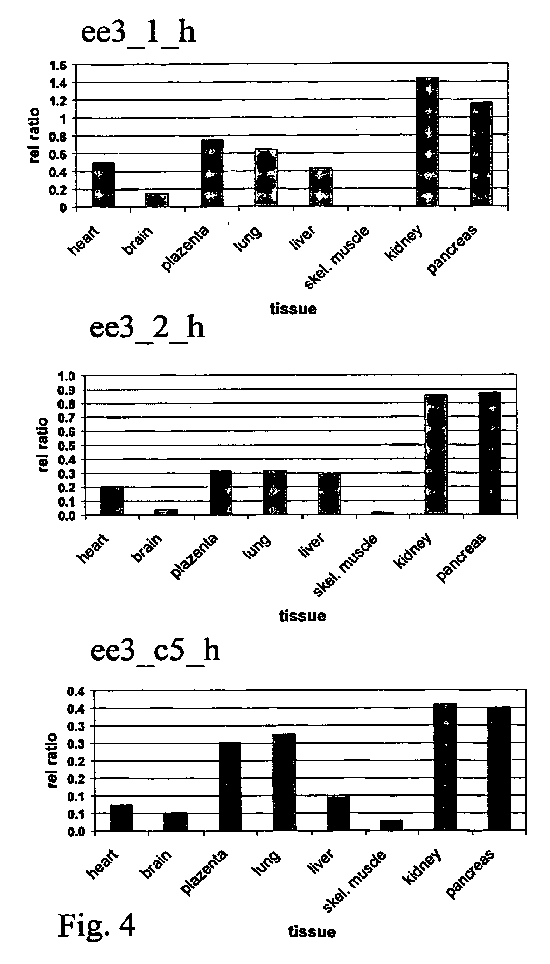

Figur 4 zeigt die Expression von erfindungsgemäßem humanem ee3_1, ee3_2, und ee3_c5 im Menschen in adulten Geweben (Herz, Gehirn, Plazenta, Lunge, Leber, Skelettmuskel, Niere, Bauchspeicheldrüse). Daten stammen aus quantitativen PCR-Experimenten (LightCycler). Die Auftragung auf der y-Achse entspricht jener in Figur 3. Es zeigt sich eine nahezu ubiquitäre und parallele Expression von Genen der erfindungsgemäßen ee3-Familie in den untersuchten Geweben. Besonders auffällig ist die stark erhöhte Expression der ee3-Familie in der Nieren und der Bauchspeicheldrüse, während die Expression in Hirn und im Skelettmuskel geringer ausfällt. Die Expression von ee3_1 und ee3_2 stimmt weitgehend überein, so daß ggf. von einer redundanten Funktion dieser beiden erfindungsgemäßen Proteine auszugehen ist.
   Beim Menschen finden sich ESTs mit ee3_1 Sequenzen in folgenden Organen: Gehirn, Auge, Keimzellen, Herz, Niere, Lunge, Placenta, Prostata, Gesamtembryo, Nebenniere, Mamma, Colon, Magen, Hoden. Dies lässt auf eine relativ breite Expression schliessen. Es finden sich ESTs von ee3_2 beim Menschen in Gehirn, Colon, Herz, Niere, Lunge, Pancreas, Parathyroidea, Prostata, Testis, Uterus, Blase, Mamma, Haut.

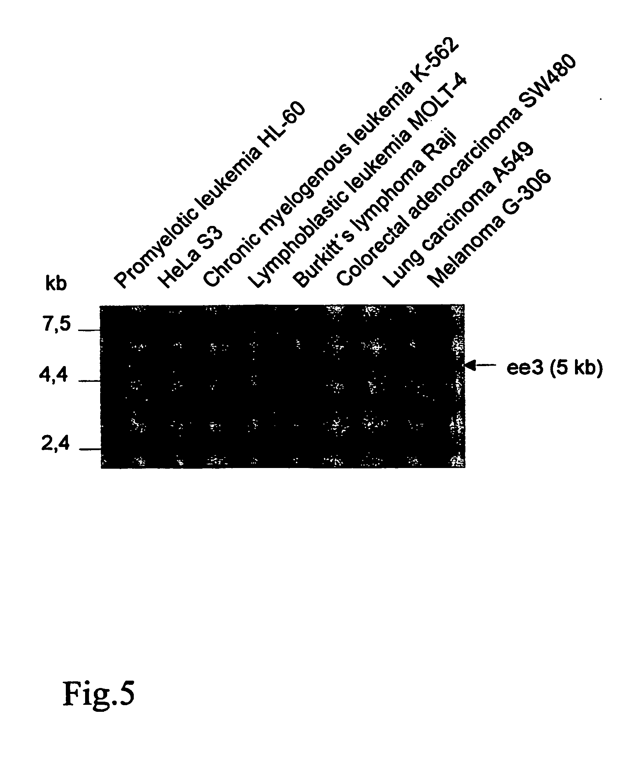

Figur 5 stellt das Ergebnis eines Northern-Blots dar, der die Expression von erfindungsgemäßem humanem ee3_1 in verschiedenen humanen Tumorzellinien dar. Zur Hybridisierung auf dem Northern-Blot (Fa. Clontech) wurde eine Maussonde eingesetzt, die den ORF von humanem ee3_1 umfaßt. Es zeigt sich eine ubiquitäre Expression eines erfindungsgemäßen humanen ee3_1-RNA-Transkriptes.

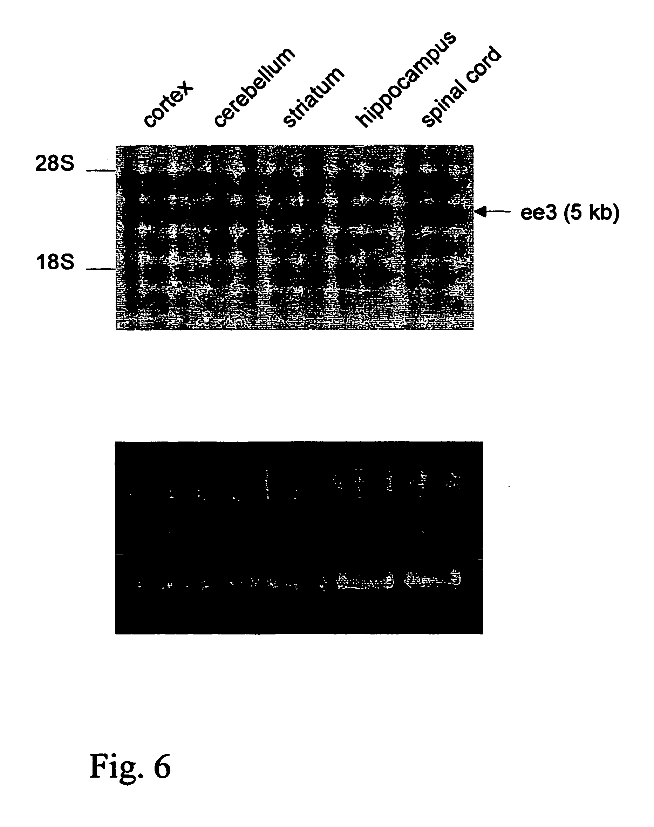

Figur 6 stellt die Expression von ee3_1 in verschiedenen Hirnarealen (Ratte). Auch hier gibt es eine ubiquitäre Verteilung in verschiedenen Hirnarealen, etwas stärker in Cerebellum und Rückenmark. Benutzte Sonde: Maus ee3_1. Darunter abgebildet ist das Bild des Ethidiumbromid-gefärbten Gels als Ladekontrolle.

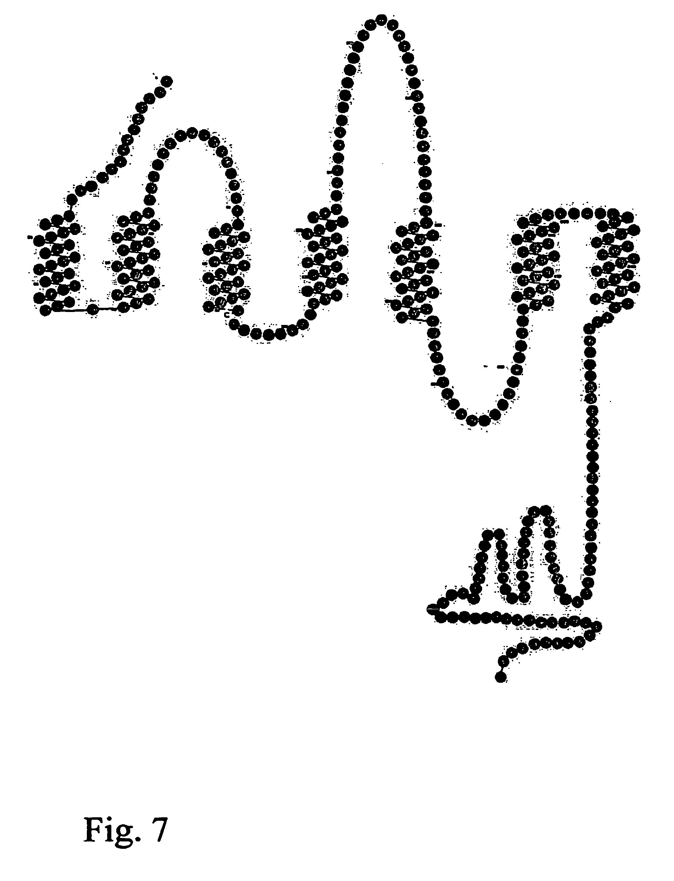

Figur 7 zeigt ein Modell der Proteintopologie von ee3_1_m anhand von Strukturvorhersagen unter besonderer Berücksichtigung der Transmembrandomänen (TM-Domänen). Es zeigt sich eine typische Topologie der GPCR-Proteine mit 7 TM-Domänen (horizontal nebeneinander dargestellt, einem kurzen extrazellulären N-Terminus (oberhalb von TM-Domäne 1 liegend) und einem intrazellulären C-Terminus (Unterhalb von TM-Domäne 7 abgebildet). Hydrophobe Aminosäuren sind grün gekennzeichnet.
Fig. 8 zeigt ein "Alignment" (Sequenzvergleich) von erfindungsgemäßem Proteinen ee3_1 ("human pro"), ee3_2 und einem Proteinfragment von ee3_5 mit verschiedenen aus dem Stand der Technik vorbekannten GPCR-Proteinen (bspw. dc32_bio, ccr5-human oder dop21_human), und Konsensusmotiven der nach dem Stand der Technik bekannten GPCR-Familien A, B, und C (in Figur 8 als cons fam A, cons fam B). "Pfam" bedeutet "protein family" und beschreibt eine Gruppe von Konsensusmotifen, die sich aus dem "Clustering" von Proteinen ergeben. Die aufgeführten Motive sind aus den pfam-Datenbanken entnommen. Es wird deutlich, daß die erfindungsgemäße Familie der ee3-Proteine die stärkste Ähnlichkeit zu den GPCR-Proteinen der Familie A hat. Besonders charakteristisch für die erfindungsgemäße Proteinfamilie gegenüber vorbekannten GPCR-Proteinen sind die (nachfolgende AS-Numerierung entspricht jener von Figur 8) folgenden Sequenzabschnitte für humanes ee3_1 und ee3_2: AS 75-85, AS 129-135 (insbesondere Glycin und Serin an Positionen 129 bzw. 132, Glycin an Position 174, AS 193-200 (insbesondere Glycin an Position 198), AS an den Positionen 260 und 261, AS an Position 308 (Cys), AS 334-340, AS an Position 539 (His), AS an Position 608 (His), AS an Position 611 (Asp), und schließlich der gesamte C-terminale Sequenzabschnitt ab Position 637 (insbesondere mit dem sauren Motiv zwischen den Positionen 640 und 655, dem basischen Motiv zwischen 666 und 670 und dem prolinreichen Motiv zwischen Position 680 und 685).
Fig. 9A stellt unter der Bezeichnung ee3_c1 (ee3_1) eine erfindungsgemäße DNA-Sequenz der Maus (Sequenz 1) dar, die den translatierten Bereich umfaßt (Lesart bei allen dargestellten Sequenzen: durchlaufend von links nach rechts und von oben nach unten, also mit Fortsetzung nach dem Zeilenende an der nächstunteren Zeile, links). Das Start- und das Stopcodon auf diesem Sequenzbereich sind durch Fettdruck hervorgehoben. Figur 9B enthält eine weitere erfindungsgemäße Sequenz, die einen Unterbereich (im 3' nicht-translatierten Bereich) der Sequenz gemäß Figur 9A darstellt.

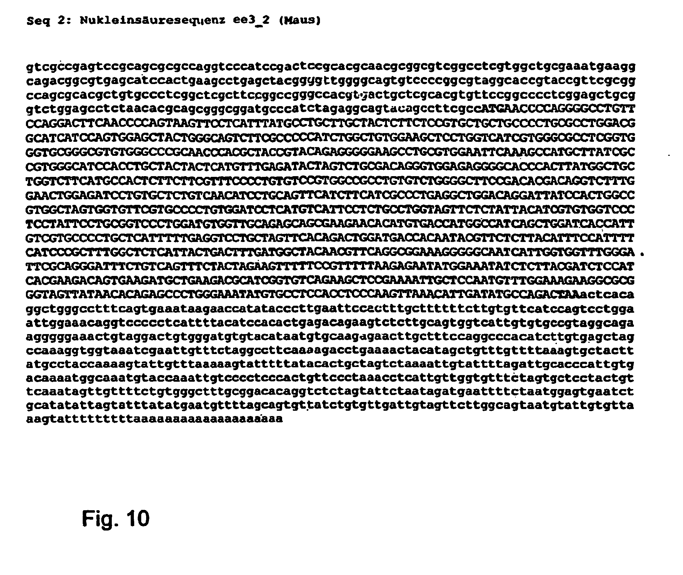

Fig. 10 stellt unter der Bezeichnung ee3_2 eine erfindungsgemäße DNA-Sequenz der Maus (Sequenz 2) dar, die auch den translatierten Bereich umfaßt Das Start- und das Stopcodon auf diesem Sequenzbereich sind durch Fettdruck hervorgehoben.

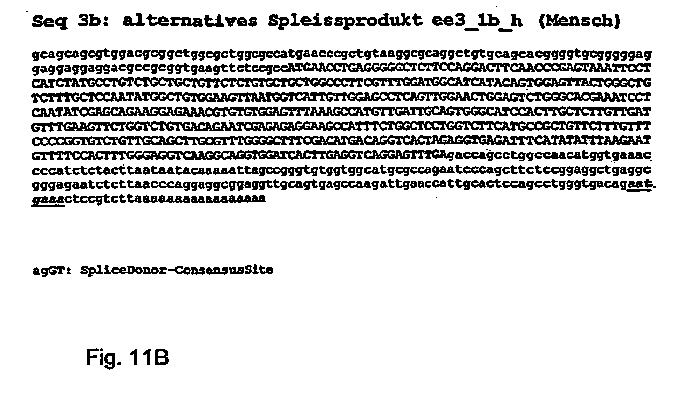

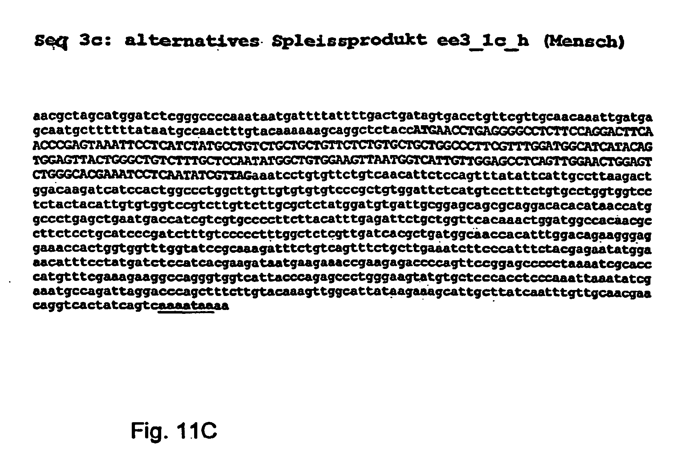

Fig. 11A stellt unter der Bezeichnung ee3_1 eine erfindungsgemäße humane DNA-Sequenz (Sequenz 3) dar, die auch den translatierten Bereich umfaßt. Das Start- und das Stopcodon auf diesem Sequenzbereich sind durch Fettdruck hervorgehoben. Darüber hinaus ist das putative Polyadenylierungssignal durch Fettdruck und Unterstreichung hervorgehoben. Figuren 11B und 11C stellen alternative C-terminal Spleißformen dar, die für ein C-terminal trunkiertes erfindungsgemäßes Protein kodieren. Neben den in beiden Figuren im Fettdruck hervorgehobenen Start- und Stopcodons ist in Figur 11B außerdem die Konsensussequenz der Spleißstelle hervorgehoben.

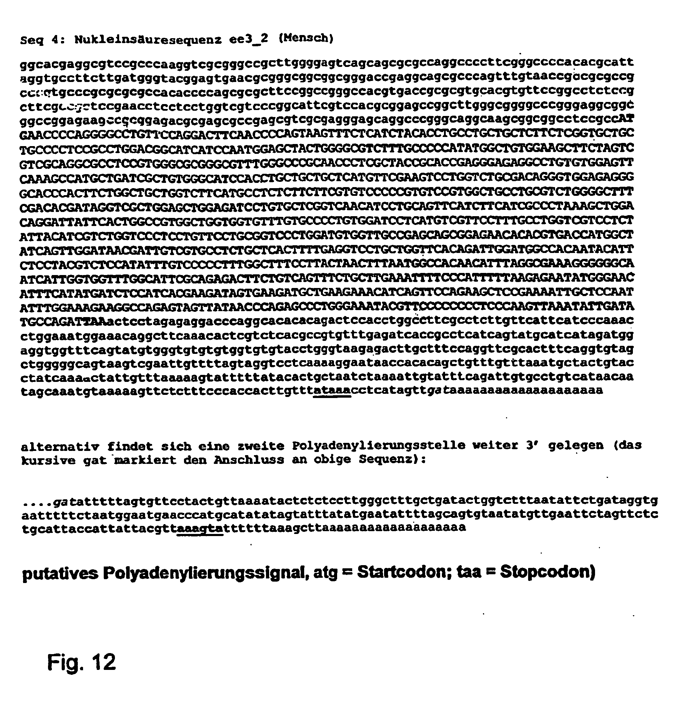

Figur 12 stellt unter der Bezeichnung ee3_2 eine erfindungsgemäße humane DNA-Sequenz (Sequenz 4) dar, die auch den translatierten Bereich umfaßt. Das Start- und das Stopcodon auf diesem Sequenzbereich sind durch Fettdruck hervorgehoben (ATG bzw. TAA). Darüber hinaus ist das putative Polyadenylierungssignal im Fettdruck hervorgehoben. Die untere Sequenz in Figur 12 setzt den ersten Sequenzteil fort (überlappender Bereich des ersten bzw. zweiten Teils im Kursivdruck).
Figur 13 stellt eine erfindungsgemäße murine AS-Sequenz unter der Bezeichnung ee3_1 dar (Sequenz 5), und zwar durchlaufend vom N- zum C-Terminus (s. auch zugrundeliegende DNA-Sequenz gemäß Figur 9)

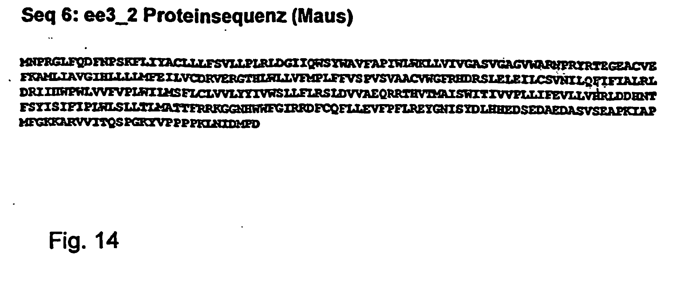

Figur 14 stellt eine erfindungsgemäße murine AS-Sequenz unter der Bezeichnung ee3_2 dar (Sequenz 6), und zwar durchlaufend vom N- zum C-Terminus (s. auch zugrundeliegende DNA-Sequenz gemäß Figur 10)

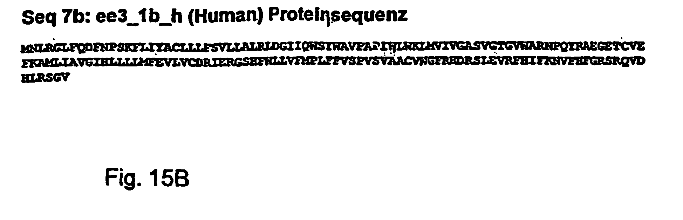

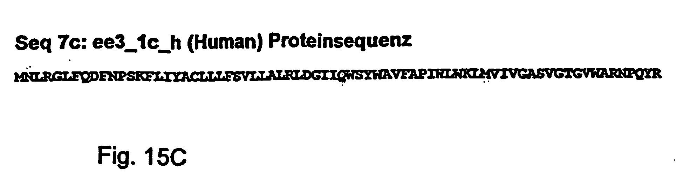

Figur 15A stellt eine erfindungsgemäße humane AS-Sequenz unter der Bezeichnung ee3_1 dar (Sequenz 7), und zwar durchlaufend vom N- zum C-Terminus (s. auch zugrundeliegende DNA-Sequenz gemäß Figur 11A). Figur 15B stellt eine erfindungsgemäße humane AS-Sequenz unter der Bezeichnung ee3_1b_h dar (Sequenz 7b), und zwar durchlaufend vom N- zum C-Terminus (s. auch zugrundeliegende DNA-Sequenz gemäß Figur 11B). Figur 15C stellt eine erfindungsgemäße humane AS-Sequenz unter der Bezeichnung ee3_1c_h dar (Sequenz 7c), und zwar durchlaufend vom N- zum C-Terminus (s. auch zugrundeliegende DNA-Sequenz gemäß Figur 11C). Die Sequenzen gemäß Figuren 15B und 15C sind die AS-Sequenzen alternativer Spleißprodukte der gemäß Fig. 11A dargestellten DNA-Sequenz.

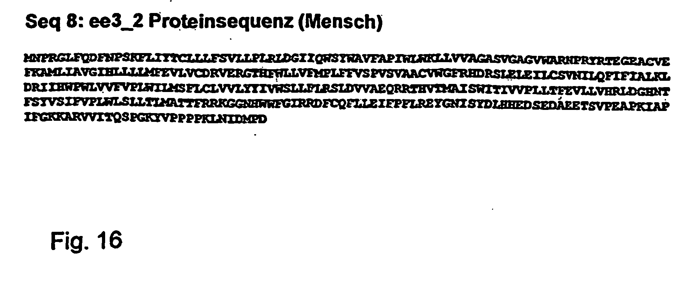

Figur 16 stellt eine erfindungsgemäße humane AS-Sequenz unter der Bezeichnung ee3_2 dar (Sequenz 8), und zwar durchlaufend vom N- zum C-Terminus (s. auch zugrundeliegende DNA-Sequenz gemäß Figur 12).

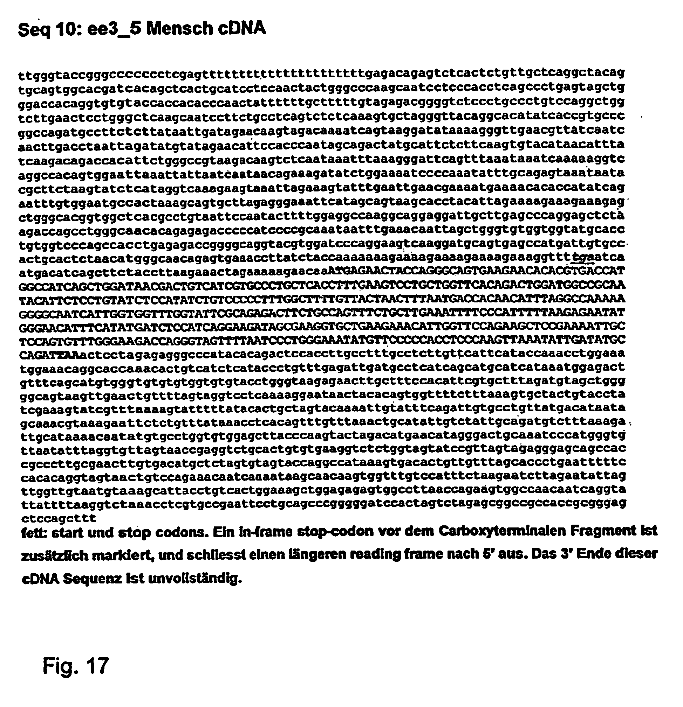

Figur 17 bildet unter der Bezeichnung ee3_5 eine erfindungsgemäße humane cDNA-Sequenz (Sequenz 10) ab, die auch den translatierten Bereich umfaßt. Das Start- und das Stopcodon (ATG bzw. TAA) auf diesem Sequenzbereich sind durch Fettdruck hervorgehoben.

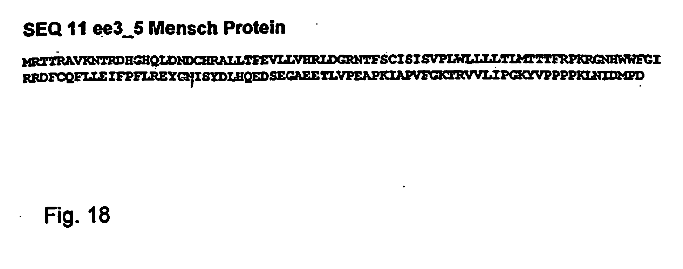

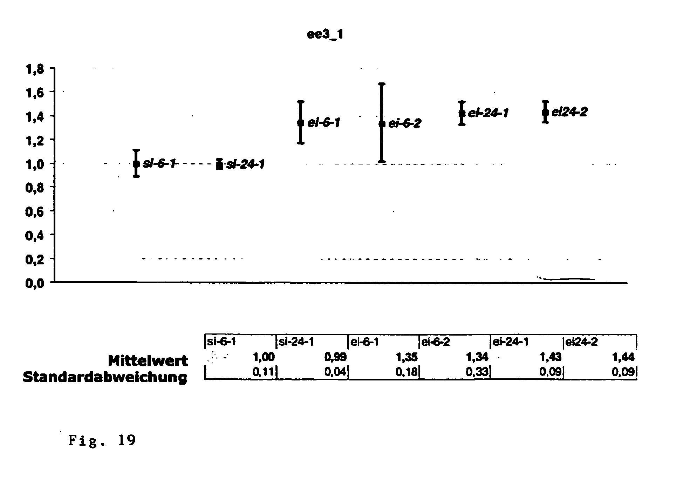

Figur 18 stellt eine erfindungsgemäße humane AS-Sequenz unter der Bezeichnung ee3_5 dar (Sequenz 11), und zwar durchlaufend vom N- zum C-Terminus (s. auch zugrundeliegende DNA-Sequenz gemäß Figur 19).
Figur 19 zeigt das Ergebnis einer quantitativen PCR für ee3_1 im Gehirn von Mäusen, die intraperitoneal mit 5000 U Erythropoetin(EPO) / kg Körpergewicht behandelt wurden, und 6 oder 24 Stunden danach perfundiert und untersucht wurden. si-6-1, si-24-1: Kochsalz-injizierte Tiere nach 6 bzw. 24 Stunden. ei-6-1, ei-6-2 EPO-injizierte Tiere nach 6 Stunden; ei-24-1, ei-24-2: EPO-injizierte Tiere nach 24 Stunden. Es zeigt sich eine erhöhte ee3 RNA-Expression, die im Zeitverlauf ansteigt. Die Werte der EPO-behandelten Tiere sind statistisch signifikant von denen der Kochsalz-behandelten verschieden (ANOVA gefolgt von Newman-Keuls post hoc Test).

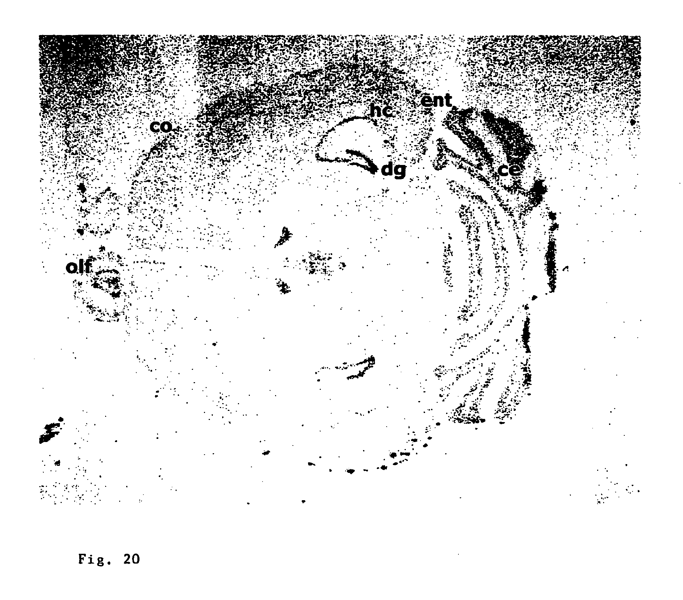

Figur 20 zeigt die Abbildung einer in situ Hybridisierung auf einen horizontalen Schnitt durch ein Mäusegehirn. Mit der eingesetzten radioaktiv markierten Sonde (ee3_1.3as AACGAAGGGCCAGTAGCACAGAGAACAGCAGCAGACAGGCATAGATGAGG) konnte die Expression von ee3_1 im Cerebellum (ce), Hippocampus (hc), Gyrus Dentatus (dg) und im Cortex (co), insbesondere im entorhinalen Cortex (ent), im Bulbus olfactorius (olf) sichtbar gemacht werden. Eine entsprechende sense Kontrolle (ee3_1.3s,CCTCATCTATGCCTGTCTGCTGCTGTTCTCTGTGCTACTGGCCCTTCGTT ) ergab kein spezifisches Signal (nicht gezeigt).

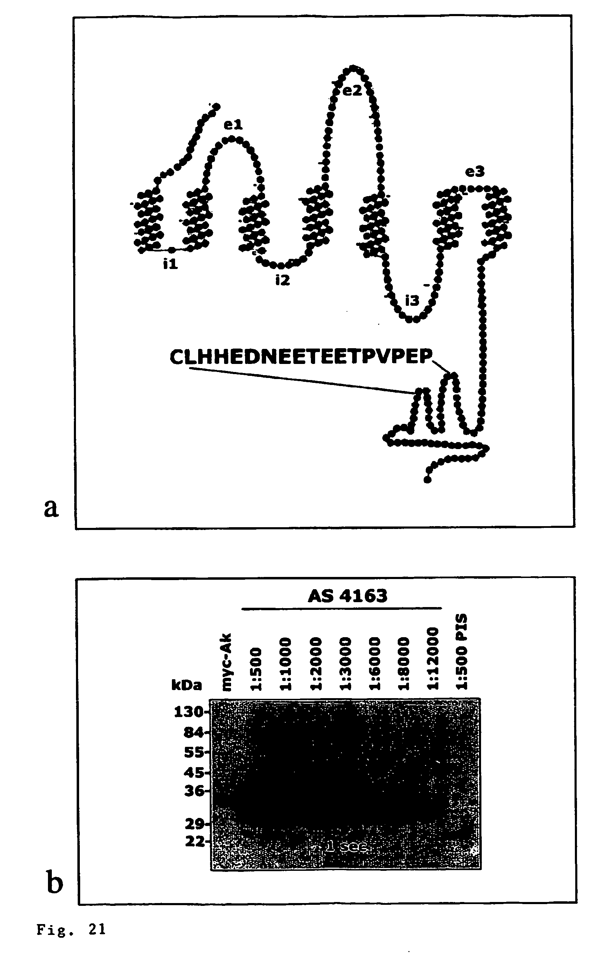

Figur 21 veranschaulicht die Herstellung eines C-terminalen polyklonalen Antiserums gegen das ee3_1 Protein (Mensch). a: Auswahl eines Peptidepitops am Carboxyterminus mit hohem Antigenizitätspotential (CLHHEDNEETEETPVPEP). b: Immunoblot, der den spezifischen Nachweis von ee3_1 in transient transfizierten HEK293 Zellen zeigt. Aufgetragen wurden jeweils gleiche Lysatmengen aus HEK293 Zellen, die mit dem Konstrukt Exp.ee3-1-h-Nter-myc transfiziert wurden, was zur Produktion von ee3_1 Protein mit N-terminal fusioniertem myc-tag führte. Spur1: Nachweis des ee3_1 Proteins mit N-terminal fusioniertem myc-tag über einen myc-spezifischen Antikörper (Firma Upstate Biotechnology (vertrieben über Biomol Feinchemikalien GmbH), eingesetzt in einer Verdünnung von 1:2000). Spur 2-8: Nachweis von ee3_1 mit verschiedenen Verdünnungen des ee3_1-spezifischen Antiserums AS4163 (1:500 - 1:12000). Das Antiserum detektiert spezifisch und hochsensitiv die ee3_1-spezifische Bande (ca. 35 kDa). Spur 9: Das entsprechende Präimmunserum (PIS) detektiert in einer Verdünnung von 1:500 keine Bande.

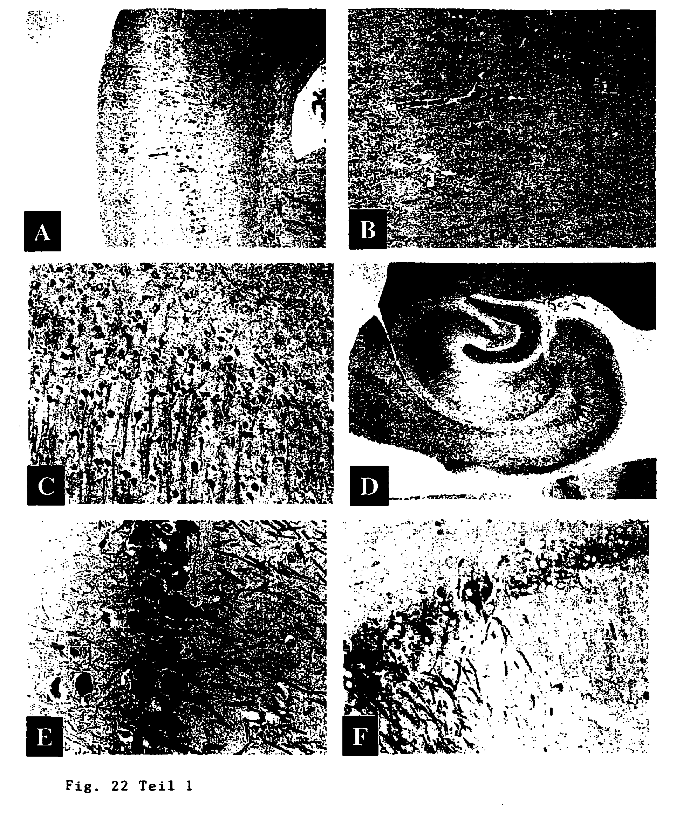

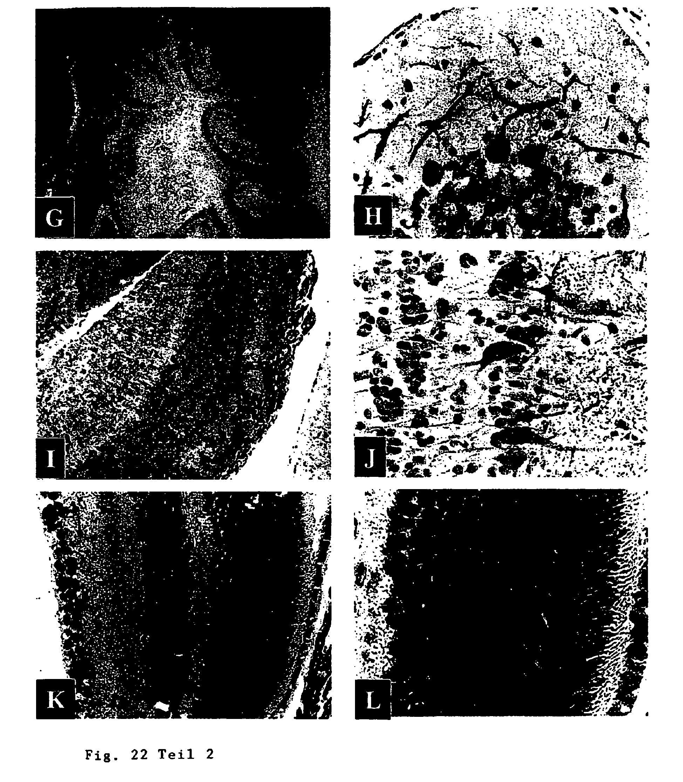

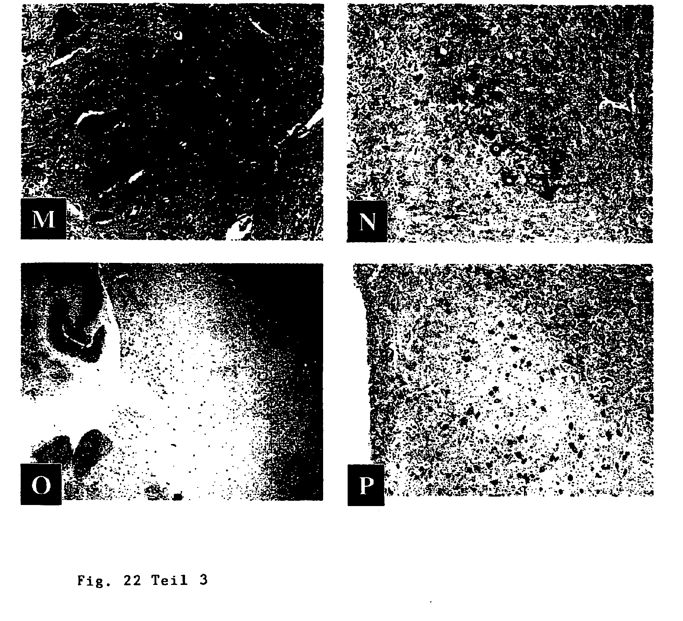

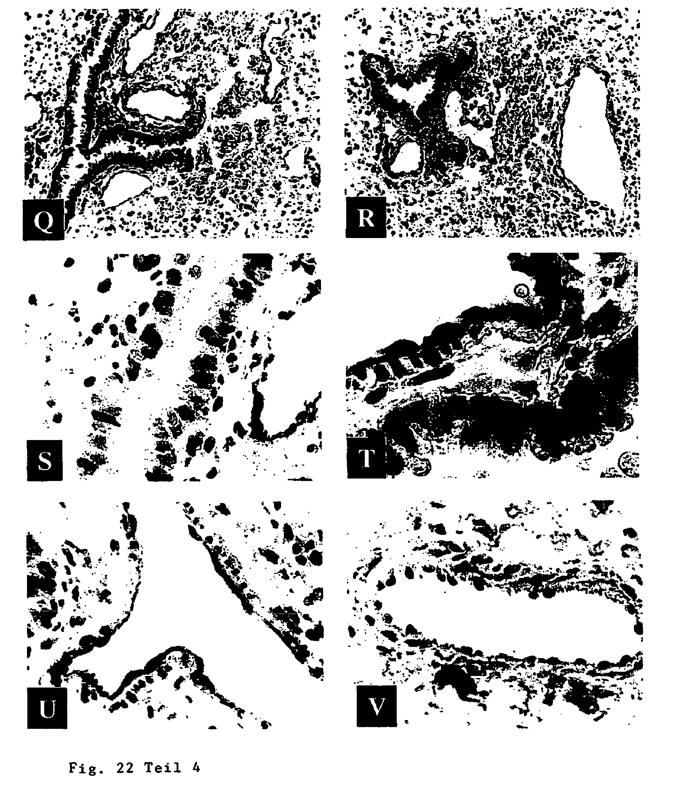

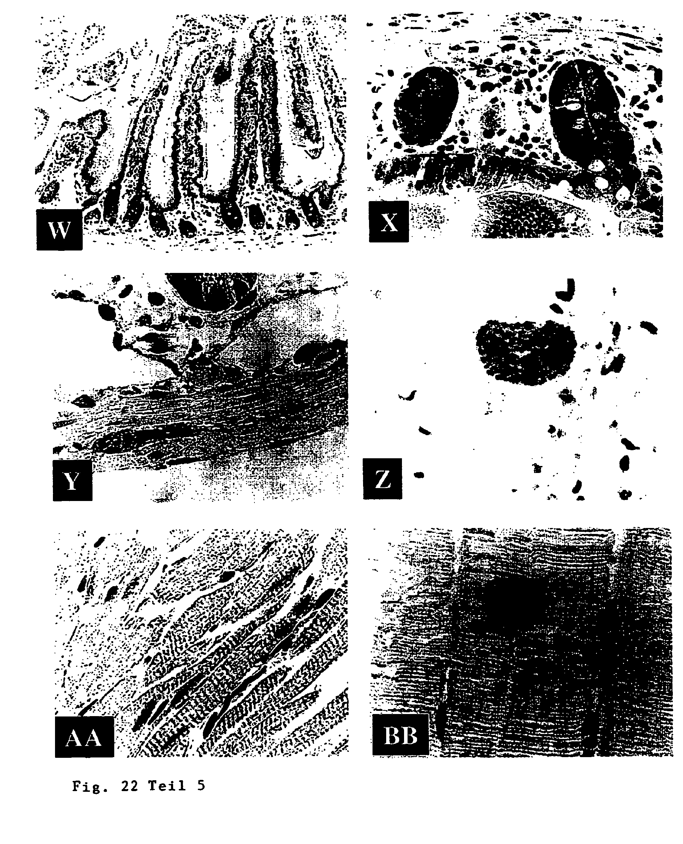

Figur 22 zeigt die immunhistochemische Detektion von ee3_1 in verschiedenen Geweben mittels des Antiserums AS4163. A: Spezifische Anfärbung von Neuronen des Layers V im somatosensorischen Cortex. B: Vergrösserung von A. C: Neurone im entorhinalen Cortex. D: Expression von ee3_1 im Gyrus dentatus und in der CA3 Region des Hippocampus, E, Vergrösserung der CA3 Region des Hippocampus. F: Grenze der Expression von ee3_1 im Hippocampus zwischen CA3 und CA2. G: Cerebellum, spezifische immunhistochemische Färbung in der Purkinjezellschicht und in Kleinhirnkernen. H: Purkinjezellen im Kleinhirn. I: Bulbus olfactorius. J: Vergrösserung, Anfärbung der grossen Mitralzellen. K: Retina, Anfärbung der Ganglienzellen und der Sinneszellen der Retina. L :Vergrösserung von K. Anfärbung der Sinneszellen der Retina. M: Expression von ee3_1 in den grossen Motoneuronen des Vorderhorns im Rückenmark. N: Expression von ee3_1 im motorischen Kern des Ncl. trigeminus. O: Anfärbung der Substantia nigra, pars reticulata. P: Vergrösserung von Q. Substantia nigra. Q: ee3_1 wird extraneural in der Lunge exprimiert. Anfärbung basaler Zellen in den Bronchioli. Anfärbung der Arteriolen, keine Färbung der Venolen. R: Darstellung der typischen pulmonalen Trias Bronchus, Arterie und Vene. S: Vergrösserung der Bronchioli. Expression in spezifischen, noch nicht näher definierten basalen Zellen. T: Vergrösserung mit Arteriolenwand (links oben) und Brochioluswand (rechts unten). U: Darstellung einer Arteriole im Verlauf. Anfärbung des Endothels und einzelner glatter Muskelzellen in der Gefässwand. V: Arteriole im Querschnitt mit immunhistochemischer Färbung glatter Muskelzellen und einzelner Endothelzellen. W: Dünndarm mit Krypten- und Zottenstruktur. Anfärbung basaler Kryptenanteile durch den Antikörper gegen ee3_1. In den Zotten sind einzelne vegetative Nerven angefärbt. X: Vergrösserung von W. Y: Darstellung von Nervenfasern in der Dünndarmwand, die zum darmeigenen vegetativen Plexus myentericus gehören. Z: Querschnitt eines peripheren Nerven im subkutanen Fett-/ Bindegewebe. AA: Herzmuskel mit spezifisch angefärbten Nervenfasern: BB: Quergestreifte Muskulatur (Skelettmuskel). Die immunhistochemische Färbung in der Bildmitte ist gut vereinbar mit einer motorischen Endplatte. Im Perimysium einzelne periphere Nervenfasern (rechts unten).

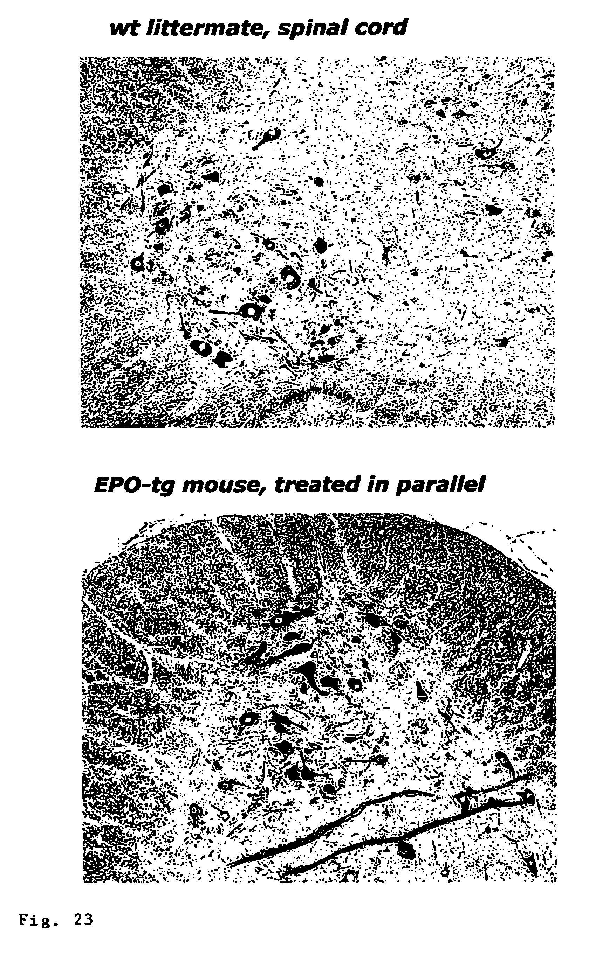

Figur 23 zeigt die Gegenüberstellung einer immunhistochemischen Färbung von ee3_1 im Rückenmark einer Wildtyp-Maus (oberer Bildteil) und einer Maus, die transgen Erythropoetin überexprimiert [(tg6) unterer Bildteil]. Bei gleichen Färbebedingungen findet sich ein deutlich stärkeres Signal in den transgenen Mäusen. Dieser Befund liess sich an je zwei weiteren Mäusen verifizieren.

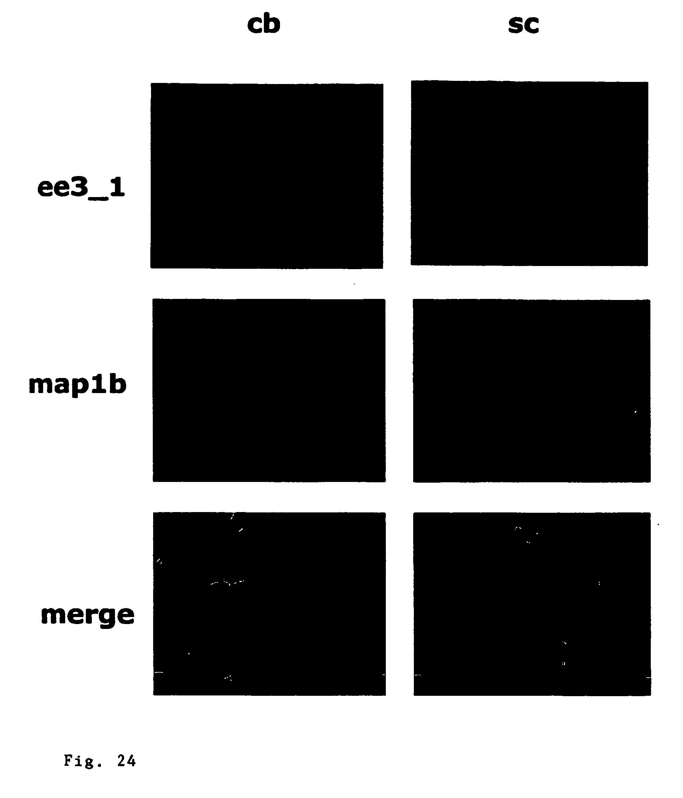

Figur 24 zeigt eine Doppelimmunfluoreszenz für ee3_1 und map1b in der Maus. Diese beiden Proteine wurden als Interaktionspartner in einem y2h System detektiert. Es findet sich eine erstaunliche Übereinstimmung der Lokalisation der beiden Proteine im ZNS. Grün: ee3_1 Färbung; Rot: Map1b Färbung; Gelb: elektronische Überlagerung beider Signale. Gezeigt sind Beispiele aus dem Rückenmark (spinal cord; sc) und aus dem Cerebellum (cb).

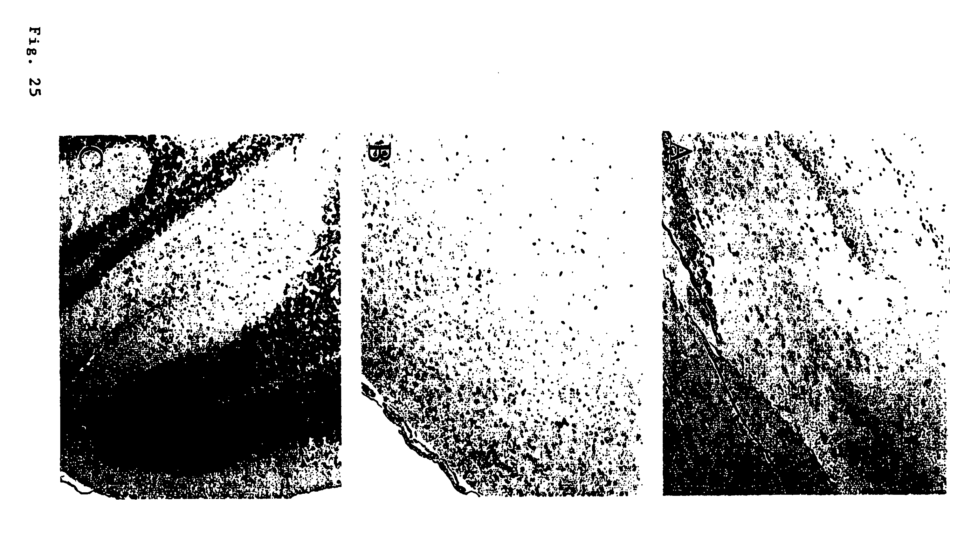

Figur 25 zeigt immunhistochemische Färbungen aus einer Mausmutante für das map1b Gen (Meixner, et al. (2000), J Cell Biol, 151, 1169-78.). Es zeigt sich, daß in den map1b homozygoten ko Tieren nur noch Spuren von ee3_1 nachzuweisen sind. a:: Hippocampus, b: Cortex, c: Cerebellum

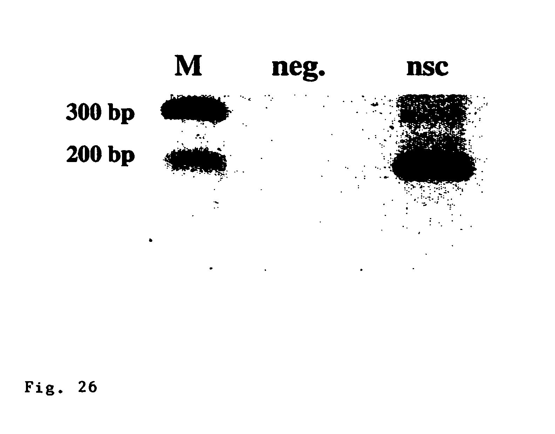

Figur 26 zeigt eine PCR auf ee3_1 in adulten neuralen Stammzellen (nsc) aus dem Rattenhippocampus. Es lässt sich kein Signal in der Negativspur (N) nachweisen. ee3_1 wird von diesen neuralen Stammzellen exprimiert.

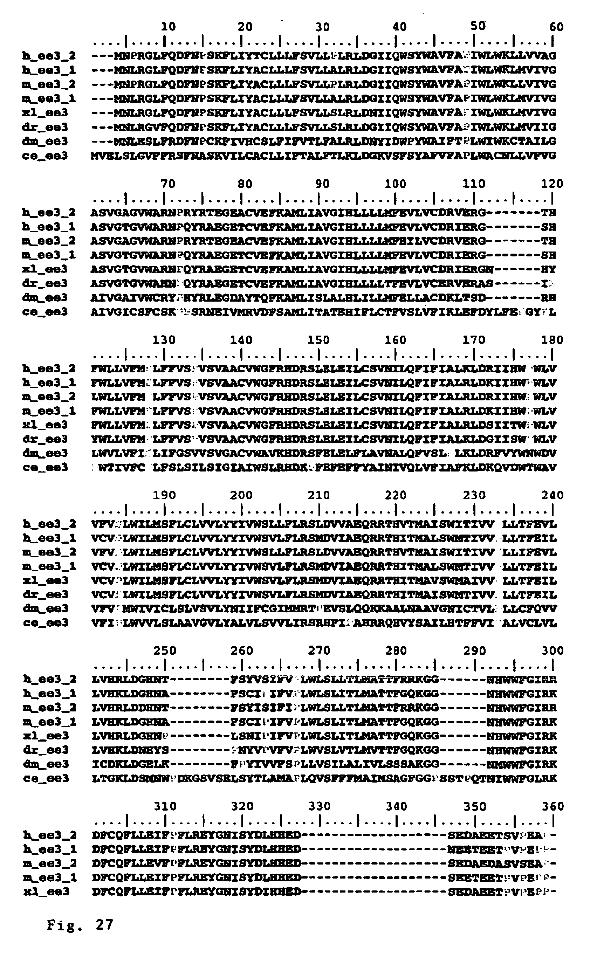

Figur 27 zeigt das Proteinalignment für ee3 Proteine aus verschiedenen Spezies unter Berücksichtigung der Sequenzen aus X. laevis und D. rerio.

Die vorliegende Erfindung wird durch das nachfolgende Ausführungsbeispiel näher erläutert:

### Ausführungsbeispiel 1

### Identifizierung und Molekulare Klonierung von ee3_1_m und Homologen

### (a) Identifizierung von ee3_1_m

Das Gehirn transgener Erythropoietin-überexprimierender Mäuse wurde in Narkose nach transkardialer Perfusion entnommen und in flüssigem Stickstoff schockgefroren. RNA wurde nach der Methode von Chomczynski und Sacchi (Anal Biochem (1987), 162, 156-9) gewonnen. Hybridisierungsexperimente von 2 transgenen und 2 Littermate-Kontrollen auf einem Maus-cDNA-Array ("Chip") wurden nach der Vorgehensweise von Incyte (s. http://www.incyte.com/reagents/lifearray/lifearray service.s html) durchgeführt. Hierbei wird eine kompetitive Hybridisierung mit Hilfe zweier unterschiedlich markierter Proben (mit Cy5 und Cy3 markiert) durchgeführt. Das Hybridisierungsexperiment ergab eine Reihe von hochregulierten Sequenzen.

Insbesondere wurde der EST-Klon AA185432 identifiziert, der in einem Wiederholungsexperiment ebenfalls in den Epo-transgenen Mäusen hochreguliert war. Der relative Induktionsfaktor betrug +3,9 ± 0,1 gegenüber den nicht-trangenen Littermates (Fig. 1). Diese Hochregulation wurde mit Hilfe einer quantitativen PCR mit dem Lightcyclersystem bestätigt (Fig. 2, Vorwärts-Primer: 5'-GGTGTGGGAGAAATGGCTTA-3', Rückwärts-Primer: 5'-ATACCAGCAGAGCCTGGAGA-3').

### (b) Klonierung von ee3-Sequenzen

Die identifizierte EST-Sequenz konnte mit Hilfe von BLASTN-Suchen in EST-Datenbanken verlängert werden. Auf diese Weise wurde eine weitere homologe Sequenz der Maus, ee3_2_m, identifiziert. Durch Benutzung von Homologiesuchen unter Einsatz entsprechender Programme (BLAST, TBLASTN) konnten in EST-und genomischen Datenbanken (ensembl) humane Homologe identifiziert werden.

Zur Bestätigung der erhaltenen Sequenzen wurden Durchmusterungen ("screens") mit Hilfe des PCR-Klonierungsverfahrens von Shepard (Shepard AR, Rae JL (1997) Magnetic bead capture of cDNAs from double-stranded plasmid cDNA libraries. Nucleic Acids Res 25:3183-3185) durchgeführt, und zwar in Maus-und humanen Sequenzbanken. Die vorgenannte Veröffentlichung und der dort zitierte Stand der Technik ist vollinhaltlich Bestandteil der Offenbarung für die vorliegende Erfindung. Dieses Verfahren beruht auf Hybridisierung von cDNA-Molekülen aus einer Plasmid-Bibliothek an eine Biotingekoppelte Oligonukleotidsequenz, nachfolgende Extraktion der Plasmide mit Hilfe von Streptavidin-gekoppelten magnetischen Perlen ("beads"). Kontrolle des Ergebnisses mittels diagnostischer PCR, und zweimaliger Wiederholung der Schritte nach Retransformation der erhaltenen Plasmidselektion, bis zur Erhaltung der Einzelklone. Folgende Primer-Kombinationen wurden verwendet:
(1) Oligonukleotide, mit denen die volle Länge des Genabschnitts kloniert wurde:
   **Für ee3_1-h:**
      - ee3_1-5'biotin1-hs: AATTCCTCATCTATGCCTGTCTGCT
      - ee3_1-3'block1-hs: GCTGTTCTCTGTGCTGCTGGCCCTTCGTTTGGATGGCATC
      - ee3_1-5'block1-hs: ATGAACCTGAGGGGCCTCTTCCAGGACTTCAACCCGAGTA
      - ee3_1-1s-hs: TGCTCCAATATGGCTGTGGA
      - ee3_1_las-hs: CTCTAGTGACCTGTCATGTC
      - ee3_1-2s-h: GACAGAGCTTAAGTGGACTG
      - ee3_1-2as-h: TACAGTTCCTACTGACTGCC
      - ee3_1-5'block2-h: ACGCACTCTCTCCGCCTTCCTCTGCCCCCTCGTTCACCCC
      - ee3_1-5'biotin2-h: GCAGACCAGAACCAGTACTGGAGCT
      - ee3 1-3'block2-h: GGGTCTCCAGGTACGTCCATCTCATGCCTTGTTTGCATCC
   **Für ee3_1-m:**
      - ee3_1-5'biotin1-m: ATTCCTCATCTATGCCTGTCTGCTG
      - ee3_1-3'block1-m: CTGTTCTCTGTGCTACTGGCCCTTCGTTTGGATGGCATCA
      - ee3_1-5'block1-m: TGAACCTGAGGGGCCTCTTTCAGGACTTCAACCCGAGTAA
      - ee3_1-1s-m: GGATGGCATCATTCAATGGAG
      - ee3_-1as-m: GAACAATGGCATGAAGACCAG
      - ee3_1-2s-m: ACTGAGCTGGATGACCATTGT
      - ee3_1-2as-m: TCCTCACTATCTTCATGGTGG
      - ee3_1-5'biotin2-m: TCATCACCCAGAGCCCTGGCAAGTA
      - ee3_1-5'block2-m: CCTAAAATTGCACCTATGTTCCGCAAGAAGGCCAGGGTAG
      - ee3 1-3'block2-m: TGTCCTTCCTCCACCCAAACTAAATATTGAAATGCCAGAC
   **Für ee3_2-h:**
      - ee3_c2-las-h: TGAACTGCAGGATGTTGACC
      - ee3 c2-1s-h: TCATCCAATGGAGCTACTGG
      - ee3_c2-5'block1-h: ATGAACCCCAGGGGCCTGTTCCAGGACTTCAACCCCAGTA
      - ee3_c2-5'biotin1-h: AGTTTCTCATCTACACCTGCCTGCT
      - ee3_c2-3'block1-h: GCTCTTCTCGGTGCTGCTGCCCCTCCGCCTGGACGGCATC
   **Für ee3_2-m:**
      - ee3_c2-3'block1-m: ACTCTTCTCCGTGCTGCTGCCCCTGCGCCTGGACGGCATC
      - ee3_c2-5'biotin1-m: AGTTCCTCATTTATGCCTGCTTGCT
      - ee3_c2-1as-m: TGGATAATCCTGTCCAGCCT
      - ee3_c2-1s-m: ATCATCCAGTGGAGCTACTG
      - ee3_c2-5'block1-m: ATGAACCCCAGGGGCCTGTTCCAGGACTTCAACCCCAGTA
      - ee3_c2-m-2s: TGTGGAAGCTCCTGGTCATCGT
      - ee3_ c2-m-2as: GATAATCCTGTCCAGCCTCAGG
      - ee3_c2-5'block2-m: GAAGCTCCTGGTCATCGTGGGCGCCTCGGTGGGTGCGGGC
      - ee3_c2-5'biotin2-m: GTGTGGGCCCGCAACCCACGCTACC
      - ee3_c2-3'block2-m: GTACAGAGGGGGAAGCCTGCGTGGAATTCAAAGCCATGCT
      - ee3_c2-5'biotin3-m: ACAGAGCCCTGGGAAATATGTGCCT
      - ee3_c2-3'block3-m: CCACCTCCCAAGTTAAACATTGATATGCCAGACTAAACTC
      - ee3_c2-5'block3-m: TTGCTCCAATGTTTGGAAAGAAGGCGCGGGTAGTTATAAC
   **Für ee3_c3-h :**
      - ee3_c3-5'blockl-h: CCACCTTGGGCACCTTGGTGTCTTTCAAAAGTGCCAGGCT
      - ee3_c3-5'biotin1-h: CCTTCCTGCCTCAGGGCCTTTGCAC
      - ee3_c3-3'block1-h: TTGCTGCTCCCTCCGTTTGAAATACTGTATCCCAGAGAGT
      - ee3_c3-1s-h: GGCACCTTGGTGTCTTTCAA
      - ee3_c3-1as-h: CAGTCTGAATTAGGAGCCAG
   **Für ee3_c5-h :**
      - ee3_c5-1as-h: TCGGAGCTTCTGGAACCAAT
      - ee3_c5-1s-h: CCATCAGCTGGATAACGACT
      - ee3_c5-5'block1-h: ACCATGGCCATCAGCTGGATAACGACTGTCATCGTGCCCC
      - ee3_c5-5'biotin1-h: TGCTCACCTTTGAAGTCCTGCTGGT
      - ee3_c5-3'block1-h: TCACAGACTGGATGGCCGCAATACATTCTCCTGTATCTCC
   **Für ee3_c8-h :**
      - ee3_c8-5'block1-h: AATTTTGGTATATGGTGCAAAAAAAGGGGTCCAATTTCTT
      - ee3_c8-5'biotin1-h: CTGCAACTGGCCAGCCAGTTATCTC
      - ee3_c8-3'block1-h: AGCATCATTAATTGAATAGGGAATCCTTACCCCACTGATT
      - ee3_c8-1s-h: AACTGGCCAGCCAGTTATCT
      - ee3_c8-1as-h: AATGGATTGTTGGGTGCAGC
      - ee3_c8-2s-h: CCAGCCAGTTATCTCAGCATCA
      - ee3_c8-2as-h: ACCATGGCATGTGTATCCCAGA
(2) Darüber hinaus wurde der kodierende Bereich der ee3-Sequenzen in GATEWAY(tm) kompatible Vektoren einkloniert, um funktionelle Analysen durchführen zu können. Folgende Oligonukleotide wurden hierfür verwendet:
   **Für ee3_1-h:** 
- ee3_1_h_B1:
GGGG ACA AGT TTG TAC AAA AAA GCA GGC
TACCATGAACCTGAGGGCCTCTTCCA

- ee3_1_h_B2:
GGGG AC CAC TTT GTA CAA GAA AGC TGG GTC
CTAATCTGGCATTTCGATATTTAATTTGGGAGGT

- ee3_1-h-C-fus-B2:
GGGG AC CAC TTT GTA CAA GAA AGC TGG GTC
GCATTTCGATATTTAATTTGGGAGGTGGGAG
   **Für ee3_2-h :** 
- ee3_c2-h-B1:
GGGG ACA AGT TTG TAC AAA AAA GCA GGC TCTACC
ATGAACCCCAGGGGCCTGTTCC

- ee3_c2-h-B2:
GGGG AC CAC TTT GTA CAA GAA AGC TGG GTC
TTAATCTGGCATATCAATATTTAACTTGGGAGGG

- ee3_c2-h-c-fus-B2:
GGGG AC CAC TTT GTA CAA GAA AGC TGG GTC
ATCTGGCATATCAATATTTAACTTGGGAGGG
   **Für ee3_c2-m :**

- ee3_c2-m-B1:
GGGG ACA AGT TTG TAC AAA AAA GCA GGC
TCTACCATGAACCCCAGGGGCCTGTTCC

- ee3_c2-m-B2:
GGGG AC CAC TTT GTA CAA GAA AGC TGG GTC
TTAGTCTGGCATATCAATGTTTAACTTGGGAG

### (c) Herstellung der humanen cDNA-Bibliothek

Mit dem cDNA-Synthese Kit der Fa. Stratagene (Amsterdam, Niederlande) wurden ausgehend von 2 µg humaner fötaler Gehirn-mRNA (Fa. Clontech, Heidelberg, Deutschland) und von 5 µg mRNA aus adultem Mausgehirn entsprechende cDNA-Bibliotheken hergestellt. Dabei wurde im wesentlichen entsprechend der Angaben des Herstellers verfahren. Zur Synthese der Erststrang cDNA wurde nach den Herstellerangaben ein oligodT-Primer verwendet. Die klonierungs-kompatiblen (Eco-RI/XhoI) doppel-strängigen cDNA Fragmente wurden größenselektioniert (nach Herstellerangaben/ Fa. Stratagene) und in den Plasmidvektor pBluescript SKII (Stratagene) ligiert. Die Ligation wurde durch Elektroporation in E.coli (DH10B, Gibco) transformiert und auf LB-Ampizillin Agar-Platten amplifiziert. Die Plasmid-DNA wurde mittels alkalischer Lyse und Ionenaustauscher-Chromatographie isoliert (QIAfilter-Kit, Fa. Qiagen, Hilden, Deutschland).

Die Komplexität an Einzelklonen betrug für die fötale humane Gehirn-cDNA-Bank 4 Millionen. Von jeder cDNA-Bank wurden nach dem Zufallsprinzip 24 Einzelklone nach Insertgrößen analysiert, die eine Größenverteilung von 800 bp bis zu 4.5 kB zeigten, die durchschnittliche Länge der cDNA-Inserts betrug für die humane Bank ca. 1,2 kB.

### Ausführungsbeispiel 2

### Regulation von ee3_1 durch Erythropoetin (EPO)

ee3_1 wurde als hochreguliertes Genprodukt in Gehirnen von Epo-transgenen Mäusen identifiziert (Maus-Linien tg6 und tg21).

Die für die erfindungsgemäßen Experimente eingesetzten Mäuse wurden zuvor mehrmals hinsichtlich ihrer Konstitution charakterisiert (Ruschitzka, et al., 2000, Proc Natl Acad Sci U S A, 97, 11609-13.; Wagner, et al., 2001, Blood, 97, 536-42.; Wiessner, et al., 2001, J Cereb Blood Flow Metab, 21, 857-64.). Die Mäuse wurden mit einem transgenen Konstrukt nach dem bei Hergersberg (Hergersberg et al., Hum. Mol. Genet. 4, 359-366) beschriebenen Verfahren hergestellt. Diese Konstrukt bestand aus einem PDGF Promotor und der kodierenden Sequenz für Erythropoetin. Dabei entstanden mehrere transgene Linien, von denen vorliegend tg6 und tg21 untersucht wurden. Nur tg6 wies eine systemisch erhöhte EPO-Expression auf, was durch Serumuntersuchungen nach der Methode von Ruschitzka, et al. (2000, Proc Natl Acad Sci U S A, 97, 11609-13) belegt wurde. Die Linie tg21 wies keine erhöhten sytemischen EPO-Level aus. In Analogie zu den Ergebnissen von Sasahara, et al. (1991, Cell, 64, 217-27.) kann davon ausgegangen werden, daß das verwendete PDGF-Promotorfragment eine Expression des transgenen EPO vor allem in neuronalen Zellen bewirkt.

Bei den Mäusen der Linie tg6 führt die erhöhte systemische Expression von EPO zu einer deutlichen Steigerung der E-rythropoese mit der Folge einer Polyglobulie bis zu einem Hämatokrit von 0.8 und einem deutlich gesteigerten Blutvolumen (bis 4.0 ml) (Wagner, et al., 2001, Blood, 97, 536-42.).

Die Linie tg21 weist demgegenüber keine starken phänotypischen Auffälligkeiten auf.

Die RNA-Produkte bspw. des Gens ee3_1 wurden im Gehirn von Mäusen, die transgen Erythropoietin (Linien tg6 und tg21 (Ruschitzka, et al., 2000, Proc Natl Acad Sci U S A, 97, 11609-13., Wagner, et al., 2001, Blood, 97, 536-42., Wiessner, et al., 2001, J Cereb Blood Flow Metab, 21, 857-64.)) überexprimieren, verstärkt exprimiert, und wurden durch ein DNA-Array-Experiment identifiziert. Die physiologische und pathophysiologische Bedeutung der transkriptionellen EE3_1-Regulation durch die Überexpression von EPO wurde durch das Auffinden eines anderen regulierten Genproduktes, nämlich alpha-Globin, belegt. Dieses wurde ebenfalls mit Hilfe einer Transkriptionsanalyse mit Microarrays in beiden transgenen Linien reguliert gefunden. Dies konnte mit Hilfe des Lightcyclersystems belegt werden (Fig. 2). Die Erhöhung zeigte sich v.a. in hippokampalen Arealen (in situ Hybridisierung).

### Ausführungsbeispiel 3

### Expression erfindungsgemäßer Sequenzen in Säugetierzellen und Herstellung stabiler Zellinien

Das offene Leseraster der Gene der ee3-Familie wurde in einen gängigen eukaryotischen Expressionsvektor aus der pcDNA-Reihe von Clontech (Heidelberg, Deutschland), kloniert. Mit den so entstehenden Expressionsplasmiden wurden humane embryonale Nierenzellen (HEK293) insbesondere nach der Calciumphosphat-Methode, CHO- Zellen und CHO-dhfr⁻-Zellen mittels Lipofectamin oder COS-Zellen mittels DEAE-Dextran-Kügelchen transfiziert und mit 400-500 mg/ml G418 selektioniert. Drei Wochen nach der Selektion wurden individuelle Klone gepickt und zur weiteren Analyse expandiert. Etwa 30 Klone wurden durch Northern-Blot- und Western-Blot-Verfahren analysiert. Zur Selektion von transfizierten CHO-dhfr⁻-Zellen in Nukleotid-freiem Medium wurde das offene Leseraster der Gene der ee3-Familie in einen eukaryotischen Expressionsvektor mit dem Dihydrofolatreduktase-Gen als Selektionsmarker kloniert und das entstehende Expressionsplasmid zur Transfektion eingesetzt. Derart transfizierte CHO-dhfr⁻Zellen, aber auch andere derart transfizierte Zellen können mit zunehmenden Konzentrationen an Methotrexat behandelt werden und wurden derart behandelt, wodurch Zellen selektioniert wurden, die erhöhte Mengen and Dihydrofolatreduktase und somit auch erhöhte Rezeptormengen exprimieren.

### Ausführungsbeispiel 4

### Yeast-2-hybrid-Experiment mit einem aarbozyterminalen Abschnitt von ee3_1

Im yeast-2-hybrid-System wurde zur Identifizierung potentieller Interaktionspartner der carboxyterminale Teil von erfindungsgemäßem ee3_1 in den "bait"-Vektor pGBKT7 (Clontech) kloniert.

Die benutzte Proteinsequenz war:

KGGNHWWFGIRKDFCQFLLEIFPFLREYGNISYDLHHEDNEETEETPVPEPPKIAPMFRK
KARVVITQSPGKYVLPPPKLNIEMPD,

Die korrespondierende Nukleinsäuresequenz war:

AAGGGAGGAAACCACTGGTGGTTTGGTATCCGCAAAGATTTCTGTCAGTTTCTGCTTGAA

ATCTTCCCATTTCTACGAGAATATGGAAACATTTCCTATGATCTCCATCACGAAGATAAT
GAAGAAACCGAAGAGACCCCAGTTCCGGAGCCCCCTAAAATCGCACCCATGTTTCGAAAG
AAGGCCAGGGTGGTCATTACCCAGAGCCCTGGGAAGTATGTGCTCCCACCTCCCAAATTA
AATATCGAAATGCCAGAT

Die Suche nach Interaktionspartnern wurde mit einer humanen Gehirnbibliothek durchgeführt, und zwar nach dem Fachmann geläufigen Standardverfahren (Mating Verfahren, Fa. Clontech). Dabei wurden 2 Klone (Klon 11 und 36) erhalten, die überlappende Sequenzen enthielten.

**Die Sequenz im identifizierten Klon 11 lautete:**

GGGGACTCGGCCCTGAACGAGCAGGAGAAGGAGTTGCAGCGGCGGCTGAAGCGTCTNTAC
CCGGCCGTGGACNAACAAGAGACGCCGTTGCCTCGGTCCTGGAGCCGAAGGACAAGTTC
AGCNTACATCGGCCTNTNTNAGAACAACCTGCGGGTGCACTACAAAGGTCATGGCAAAAC
CCCAAAAGATGCCGCGTCAGTTCGAGCCACGCATCCAATACCAGCAGCCTGTGGATTTA
TTATTTTGAAGTAAAAATTGTCAGTAAGGAAGAGATGGTTNCATGGGAATTGGTCTTTC
TGCTCAAGGNGTGAACATGAATAGACTACCAGGTTGGGATAAGCATTCATATGGTTACCA
TGGGGATGATGGACATTCGTTTTGTTCTTCTGGAACTGGACAACCTTATGGACCAACTTT
CACTACTGGTGATGTCATTGGCTGTTGTGTTAATCTTATCAACAATACCTGCTTTTACAC
CAAGAATGGACATAGTTTAGGTATTGCTTTCACTGACCTACCGCCAAATTTGTATCCTAC
TGTGGGGCTTCAAACACCAGGAGAAGTGGTCGATGCCAATTTTGGGCAACATCCTTTCGT
GTTTGATATAGAAGACTATNTGCGGGAGTGGAGAACCAAAATCCAGGCNCAGATAGATCG
ATT.

Das interagierende Genprodukt konnte als RANBPM oder RANBP9 identifiziert werden (Nishitani H, Hirose E, Uchimura Y, Nakamura M, Umeda M, Nishii K, Mori N, Nishimoto T (2001) Full-sized RanBPM cDNA encodes a protein possessing a long stretch of proline and glutamine within the N-terminal region, comprising a large protein complex. Gene 272:25-33). Ebenso konnten zwei weitere interagierende Proteine identifiziert werden, nämlich Map1a und Map1b. Interessanterweise wurde bei beiden Proteinen der carboxyterminale Teil als interagierend identifiziert. Darin findet sich ein homologer Bereich in beiden Proteinen. Gezeigt ist ein Alignment von Map1a und Map1b in diesem Bereich, wobei die obere Sequenz Map1a und die untere Map1b darstellt:

| | |
|---|---|
| ALIGN calculates a global alignment of two sequences | |
| version 2.0uPlease cite: Myers and Miller, CABIOS (1989) 4:11-17 | |
| Sequence 1 | 212 aa vs. |
| Sequence 2 | 177 aa |
| scoring matrix: BLOSUM50, gap penalties: -12/-2 | |
| 43.6% identity: | Global alignment score: 614 |

                10        20        30        40        50
/tmp/f KEKVQGRVGRRAPGKAKPASPARRLDLRGKRSPTPGKGPADRASRAPPRP--RSTTSQVT
                                            :  ...:..    :  ... ..
Sequen ------------------------------------KKESVEKAAKPTTTPEVKAARGEEK
                                                  10        20

       60        70        80        90                100       110
/tmp/f PAEEKDGHSPMSKGLVNGLKAGPMALSSKGSS----GAPVYVDLAYIPNXCSGKTADLDF
        : :.. .  .. ..  ::: . .. ::   : :::.:: ::::  ..:..:..:
Sequen DKETKNAANASASKSAKTATAGPGTTKTTKSSAVPPGLPVYLDLCYIPNHSNSKNVDVEF
          30        40        50        60        70        80

          120       130       140       150       160       170
/tmp/f FRRVRASYYVVSGNDPANGXPSRAVLDALLEGKAQWGENLQVTLIPTHDTEVTREWYQQT
       :.:::.:::::::::::::   ::::::::::::::::: :.:::::::::.:: :::::.:
Sequen FKRVRSSYYVVSGNDPAAEEPSRAVLDALLEGKAQWGSNMQVTLIPTHDSEVMREWYQET
          90        100       110       120       130       140

          180       190       200       210
/tmp/f HEQQQQLNVLVLASTXTVVMQDESFPACRLSSEKPPSL
       ::.::.::..::::. :::::::::::::..
Sequen HEKQQDLNIMVLASSSTVVMQDESFPACKIEL------
          150       160       170

### Ausführungsbeispiel 5

### Humane homologe Sequenzen von ee3_1/ee3_2

### (a) auf Chromosom 5q33.1

Mit Hilfe von Tblastn wurde eine weitere homologe Sequenz auf Contig AC11406.00015 ermittelt:
>AC011406.00015
Length: 40,820
Minus Strand HSPs:
Score = 389 (136.9 bits), Expect = 1.3e-41, Sum P(3) = 1.3e-41
Identities = 72/303 (71%), Positives = 78/303 (77%), Frame = -1 
Query: 224    LLTFEILLVHKLDGHNAFSCIPIFVPLWLSLITLMATTFGQKGGNHWWFGIRKDFCQFLL 283
               LLTFE+LLVH+LDG N FSCI I VPLWL L+TLM TTF  K GNHWWFGIR+DFCQFLL
 Sbjct: 14312 LLTFEVLLVHRLDGRNTFSCISISVPLWLLLLTLMTTTFRPKRGNHWWFGIRRDFCQFLL 14253 
Query: 284    EIFPFLREYGNISYDLHHEDNXXXXXXXXXXXXKIAPMFRK 324
              EIFPFLREYGNISYDLH ED+            KIAP+F K
 Sbjct: 14252 EIFPFLREYGNISYDLHQEDSEGAEETLVPEAPKIAPVFGK 14010
Score = 86 (30.3 bits), Expect = 1.3e-41, Sum P(3) = 1.3e-41
Identities = 15/51 (88%), Positives = 16/51 (94%), Frame = -3 
Query: 334   PGKYVLPPPKLNIEMPD 350
              PGKYV PPPKLNI+MPD
 Sbjct: 13992 PGKYVPPPPKLNIDMPD 13942
Score = 67 (23.6 bits), Expect = 1.3e-41, Sum P(3) = 1.3e-41
Identities - 12/57 (63%), Positives - 17/57 (89%), Frame = -2 
Query: 206    QRRTHITMALSWMT-IVVP 223
              Q RTH+TMA+SW+T ++VP
 Sbjct: 14368 Q*RTHVTMAISWITTVIVP 14312

Die entsprechende cDNA konnte erhalten werden, allerdings ergibt die Translation nur ein carboxyterminales Bruchstück, das homolog zu den ee3 Proteinen ist.

Der Sequenzvergleich mit ee3_1_m sieht folgendermaßen aus:

| | |
|---|---|
| ALIGN calculates a global alignment of two sequences | |
| version 2.0uPlease cite: Myers and Miller, CABIOS (1989) 4:11-17 | |
| Sequence 1 | 350 aa vs. |
| Sequence 2 | 148 aa |
| scoring matrix: BLOSUM50, gap penalties: -12/-2 | |
| 29.4% identity; | Global alignment score: 649 |

                    10        20        30        40        50        60
/tmp/f MNLRGLFQDFNPSKFLIYACLLLFSVLLALRLDGIIQWSYWAVFAPIWLWKLMVIVGASV

Sequen ------------------------------------------------------------


                    70        80        90       100       110       120
/tmp/f GTGVWARNPQYRAEGETCVEFKAMLIAVGIHLLLLMFEVLVCDRIERGSHFWLLVFMPLF

Sequen ------------------------------------------------------------


                   130       140       150       160       170       180
/tmp/f FVSPVSVAACVWGFRHDRSLELEILCSVNILQFIFIALRLDKIIHWPWLVVCVPLWILMS

Sequen ------------------------------------------------------------


                   190       200       210       220       230       240
/tmp/f FLCLVVLYYIVWSVLFLRSMDVIAEQRRTHITMALSWMTIVVPLLTFEILLVHKLDGHNA
                       .:.  .. .. : :    :.       :::::.::::.:::.:.
Sequen ----------------MRTTRAV-KNTRDH-GHQLD-NDCHRALLTFEVLLVHRLDGRNT
                                 10        20        30        40

                   250       260       270       280       290       300
/tmp/f FSCIPIFVPLWLSLITLMATTFGQKGGNHWWFGIRKDFCQFLLEIFPFLREYGNISYDLH
       :::: : ::::: :.:::.:::  : :::::::::.::::::::::::::::::::::::
Sequen FSCISISVPLWLLLLTLMTTTFRPKRGNHWWFGIRRDFCQFLLEIFPFLREYGNISYDLH
               50        60        70        80        90       100

310        320        330        340        350
/tmp/f HEDSEETEETPVPEPPKIAPMFRKKARVVITQSPGKYVLPPPKLNIEMPD
       .:::: .::: ::: :::::.:  :.:::.   ::::: :::::::.:::
Sequen QEDSEGAEETLVPEAPKIAPVF-GKTRVVLI--PGKYVPPPPKLNIDMPD
          110        120        130        140

Die Generierung nur eines GPCR-Bruchstückes ist sicher, da die erhaltenen cDNA Sequenzen völlig mit genomischen Daten übereinstimmen, und die Präsenz eines in-frame Stopcodons vor dem ATG zeigen (siehe Sequenz):
Minus Strand HSPs:
Score = 6976 (1046.7 bits), Expect = 0.0, Sum P(2) = 0.0
Identities = 1396/1397 (100%), Positives - 1396/1397 (100%), Strand = Minus / Plus

Query: 2499 AGGTTTAGACCTTAAAATAATACCTGATTGTTGGCCACTTCTGGTTAAGGCCACTCTCTC 2440
| ||||||||||||||||||||||||||||||||||||||||||||||||||||||||||
Sbjct: 13048 AAGTTTAGACCTTAAAATAATACCTGATTGTTGGCCACTTCTGGTTAAGGCCACTCTCTC 13107

Query: 2439 CAGCTTTCCAGTGACAGGTAATGCTTTACATTACAACCAACTAATATTCTAAGATTCTTA 2380
||||||||||||||||||||||||||||||||||||||||||||||||||||||||||||
Sbjct: 13108 CAGCTTTCCAGTGACAGGTAATGCTTTACATTACAACCAACTAATATTCTAAGATTCTTA 13167

Query: 2379 GAAATGGACAAACCACTTGTTGCTTATTTGATTGTTTCTGGACAGTTACTACCTGTGTG 2320
||||||||||||||||||||||||||||||||||||||||||||||||||||||||||||
Sbjct: 13168 GAAATGGACAAACCACTTGTTGCTTATTTGATTGTTTCTGGACAGTTACTACCTGTGTG 13227

Query: 2319 GAAAAATTCAGGGTGCTAAACAACAGTGTCACTTTATGGCCTGGTACTACACTAGAGCAT 2260
||||||||||||||||||||||||||||||||||||||||||||||||||||||||||||
Sbjct: 13228 GAAAAATTCAGGGTGCTAAACAACAGTGTCACTTTATGGCCTGGTACTACACTAGAGCAT 13287

Query: 2259 GTCACAAGTTCGCAAGGGCGGTGGCTGCTCCCTCTACTAACGGATACTACCAGAGACCTT 2200
||||||||||||||||||||||||||||||||||||||||||||||||||||||||||||
Sbjct: 13288 GTCACAAGTTCGCAAGGGCGGTGGCTGCTCCCTCTACTAACGGATACTACCAGAGACCTT 13347

Query: 2199 CACACAGTGCAGACCTCGGTTACTAACACCTAAATATTAACACCCATGGGATTTGCAGTC 2140
||||||||||||||||||||||||||||||||||||||||||||||||||||||||||||
Sbjct: 13348 CACACAGTGCAGACCTCGGTTACTAACACCTAAATATTAACACCCATGGGATTTGCAGTC 13407

Query: 2139 CCTATGTTCATGTCTAGTACTTGGGTAAGCTCCACACCAGGCACATATTGTTTATGCAA 2080
||||||||||||||||||||||||||||||||||||||||||||||||||||||||||||
Sbjct: 13408 CCTATGTTCATGTCTAGTACTTGGGTAAGCTCCACACCAGGCACATATTGTTTATGCAA 13467

Query: 2079 TCTTTAAAGACATCTGCAATAGACAATATGCAGTTTAAACAAACTGTGAGGTTTATAAAC 2020
||||||||||||||||||||||||||||||||||||||||||||||||||||||||||||
Sbjct: 13468 TCTTTAAAGACATCTGCAATAGACAATATGCAGTTTAAACAAACTGTGAGGTTTATAAAC 13527

Query: 2019 AGAGAATTCTTTACGTTTGCTATTATGTCATAACAGGCACAATCTGAAATACAATTTTGT 1960
||||||||||||||||||||||||||||||||||||||||||||||||||||||||||||
Sbjct: 13528 AGAGAATTCTTTACGTTTGCTATTATGTCATAACAGGCACAATCTGAAATACAATTTTGT 13587

Query: 1959 ACTAGCAGTGTATAAAAATACTTTTAAACGATACTTTCGATAGGTACAGTAGCACTTTAA 1900
||||||||||||||||||||||||||||||||||||||||||||||||||||||||||||

Sbjct: 13588 ACTAGCAGTGTATAAAAATACTTTTAAACGATACTTTCGATAGGTACAGTAGCACTTTAA 13647

Query: 1899 AGAAAACCACTGTGTAGTTATTCCTTTTGAGGACCTACTAAAACAGTTCAACTTACTGCC 1840
||||||||||||||||||||||||||||||||||||||||||||||||||||||||||||
Sbjct: 13648 AGAAAACCACTGTGTAGTTATTCCTTTTGAGGACCTACTAAAACAGTTCAACTTACTGCC 13707

Query: 1839 CCCAGCTACATCTAAAGCACGAATGTGGAAAGCAAGTTCTCTTACCCAGGTACACACCAC 1780
||||||||||||||||||||||||||||||||||||||||||||||||||||||||||||
Sbjct: 13708 CCCAGCTACATCTAAAGCACGAATGTGGAAAGCAAGTTCTCTTACCCAGGTACACACCAC 13767

Query: 1779 ACACACCCACATGCTGAAACAGTCTCCATTTATGATGCATGCTGATGAGGCATCAATCTC 1720
||||||||||||||||||||||||||||||||||||||||||||||||||||||||||||
Sbjct: 13768 ACACACCCACATGCTGAAACAGTCTCCATTTATGATGCATGCTGATGAGGCATCAATCTC 13827

Query: 1719 AAACAGGGTATGAGATGACAGTGTTTGGTGCCTGTTTCCATTTCCAGGTTTGGTATGAAT 1660
||||||||||||||||||||||||||||||||||||||||||||||||||||||||||||
Sbjct: 13828 AAACAGGGTATGAGATGACAGTGTTTGGTGCCTGTTTCCATTTCCAGGTTTGGTATGAAT 13887

Query: 1659 GAACAAGAGGCAAAGGCAAGGTGGAGTCTGTGTATGGGCCCTCTCTAGGAGTTTAATCTG 1600
||||||||||||||||||||||||||||||||||||||||||||||||||||||||||||
Sbjct: 13888 GAACAAGAGGCAAAGGCAAGGTGGAGTCTGTGTATGGGCCCTCTCTAGGAGTTTAATCTG 13947

Query: 1599 GCATATCAATATTTAACTTGGGAGGTGGGGAACATATTTCCCAGGGATTAAAACTACCT 1540
||||||||||||||||||||||||||||||||||||||||||||||||||||||||||||
Sbjct: 13948 GCATATCAATATTTAACTTGGGAGGTGGGGAACATATTTCCCAGGGATTAAAACTACCT 14007

Query: 1539 GGTCTTCCCAAACACTGGAGCAATTTTCGGAGCTTCTGGAACCAATGTTTCTTCAGCACC 1480
||||||||||||||||||||||||||||||||||||||||||||||||||||||||||||
Sbjct: 14008 GGTCTTCCCAAACACTGGAGCAATTTTCGGAGCTTCTGGAACCAATGTTTCTTCAGCACC 14067

Query: 1479 TTCGCTATCTTCCTGATGGAGATCATATGAAATGTTCCCATATTCTCTTAAAAATGGGAA 1420
||||||||||||||||||||||||||||||||||||||||||||||||||||||||||||
Sbjct: 14068 TTCGCTATCTTCCTGATGGAGATCATATGAAATGTTCCCATATTCTCTTAAAAATGGGAA 14127

Query: 1419 AATTTCAAGCAGAAACTGGCAGAAGTCTCTGCGAATACCAAACCACCAATGATTGCCCCT 1360
||||||||||||||||||||||||||||||||||||||||||||||||||||||||||||
Sbjct: 14128 AATTTCAAGCAGAAACTGGCAGAAGTCTCTGCGAATACCAAACCACCAATGATTGCCCCT 14187

Query: 1359 TTTTGGCCTAAATGTTGTGGTCATTAAAGTTAGTAACAAAAGCCAAAGGGGGACAGATAT 1300
||||||||||||||||||||||||||||||||||||||||||||||||||||||||||||
Sbjct: 14188 TTTTGGCCTAAATGTTGTGGTCATTAAAGTTAGTAACAAAAGCCAAAGGGGGACAGATAT 14247

Query: 1299 GGAGATACAGGAGAATGTATTGCGGCCATCCAGTCTGTGAACCAGCAGGACTTCAAAGGT 1240
||||||||||||||||||||||||||||||||||||||||||||||||||||||||||||
Sbjct: 14248 GGAGATACAGGAGAATGTATTGCGGCCATCCAGTCTGTGAACCAGCAGGACTTCAAAGGT 14307

Query: 1239 GAGCAGGGCACGATGACAGTCGTTATCCAGCTGATGGCCATGGTCACGTGTGTTCTTCAC 1180
||||||||||||||||||||||||||||||||||||||||||||||||||||||||||||
Sbjct: 14308 GAGCAGGGCACGATGACAGTCGTTATCCAGCTGATGGCCATGGTCACGTGTGTTCTTCAC 14367

Query: 1179 TGCCCTGGTAGTTCTCATTTGTTCTTTTTCTAGTTTCTTAAGGTAGAAGCTGATGTCATT 1120
||||||||||||||||||||||||||||||||||||||||||||||||||||||||||||
Sbjct: 14368 TGCCCTGGTAGTTCTCATTTGTTCTTTTTCTAGTTTCTTAAGGTAGAAGCTGATGTCATT 14427

Query: 1119 GATTCAAAACCTTTCTT 1103
|||||||||||||||||
Sbjct: 14428 GATTCAAAACCTTTCTT 14444

### (b) auf Chromosom 8q11.22

Es findet sich eine weitere homologe Sequenz auf Ensembl Contig Ac034174.

Die Proteinsequenz eines homologen Nukleotidabschnittes lautet:

AATTTTGGTATATGGTGCAAAAAAAGGGGTCCAATTTCTTCTGCAACTGGCCAGCCAGTTATCTCAGCATCATTAATTGA
ATAGGGAATCCTTACCCCACTGATTGTTTTTGTCAGGTTTGTCAAAGATGAAATAGTTGTAGGTGTATGGTCTTATTTCT
GGGTTCCCCATTCTGTTCCACTGGTATATGTGTCTGGTTTTGAACTAGTGCCATGCTGTTTTGGTTACTATAGCCCTGTT
TAAAATCAAATGGAGTGATGCCGCCACGTTGTATTTATTTATTTTTTATTATACTTTAAGTTCTGGGATACACATGCC
ATGGTGGTTTGCTGCACCCAACAATCCATTATCTATGTTGTTTTCTC

### (c) auf Chromosom 3p25.3

Es findet sich eine homologesequenz auf Chromosom 3:

CCACCTTGGGCACCTTGGTGTCTTTCAAAAGTGCCAGGCTCCTTCCTGCCTCAGGGCCTTTGCACTTGCTGCTCCCTCCG
TTTGAAATACTGTATCCCAGAGAGTCCCATTTCTGGCTCCTAATTCAGACTGA

### (d) auf Chromosom Xp21.1:

>AC027722.00010
Length: 3,576
Minus Strand HSPs:
Score = 65 (22.9 bits), Expect = 4.1e+02, Sum P(2) = 1.0
Identities = 11/90 (37%), Positives = 19/90 (63%), Frame = -1 
Query: 159  RLDKIIHWPWLVVCVPLWI-LMSFLCLVVL 187
             R+   ++W  L  C+P+W+  +SF CL+ L
 Sbjct: 1848 RIMSSLNWDSLTSCLPIWMTFISFSCLIAL 1759
Score = 57 (20.1 bits), Expect = 4.1e+02, Sum P(2) = 1.0
Identities = 16/147 (33%), Positives = 24/147 (49%), Frame = -2 
Query: 88   VGIHLLLLMFEVLVCDRIERG-SH-FWLLVFMPLFFVSPVSVAACVWGF 134
            +G   L++ F   V D++  G  H FW L  +PLF VS      C + +
 Sbjct: 2507 IGCCFLIVCFVCFVEDQMYVGLQHYFWALYSVPLFCVSVFVPVPCSFSY 2361

Entsprechende Nukleotidsequenzen zu diesen Homologieabschnitten lauten: 
ATCATGTCATCTCTAAACTGGGATAGTTTGACTTCCTGTCTTCCTATTTGGATGACTTTTATTTCTTTCTCTTGCCTGAT
TGCTCTGG
 Und 
ATAGGGTGTTGTTTCCTCATTGTTTGTTTTGTCTGCTTTGTGGAAGATCAGATGTATGTAGGTTTGCAGCATTATTTCTG
GGCTCTCTATTCTGTTCCTTTGTTCTGTGTGTCTGTGTTTGTACCAGTACCATGTTCTTTTAGTTACT
 Allerdings fehlt die Konsensussequenz DRI.

### (d) Alternative SpleiBprodukte ee3_1b_h und ee3_1c_h

Es findet sich ein alternatives Spliceprodukt zum humanen Genprodukt ee3_1_h, nämlich ee3_1b_h (s. Fig. 11B, Seqenznummer 3B). Dieses resultiert aus einer Konsensus-Spleiß-Donorsite im Exon 3, und führt zu einem geänderten offenen Leseraster ("open reading frame") mit geändertem Carboxyterminus, und früherem Translationsstop. Es resultiert daraus ein Protein (166 Aminosäuren, Molgew.: 19,2 kD), das nach der vierten TM-Domäne abbricht (s. Fig. 15B, Sequenznummer 7B). Darüber hinaus wurde ein weiteres alternatives Spleißprodukt identifiziert, nämlich ee3_1c_h (s. Fig. 11C, Sequenznummer 3C) mit der dazu gehörigen Proteinsequenz gemäß Figur 15C (Sequenznummer 7C), die nach der zweiten TM-Domäne trunkiert ist. Die Spleißprodukte konnten im Zuge der Klonierungen und Sequenzierungen mit Hilfe des Klonierungsverfahrens von Shepard et al. (siehe Beispiel 1) identifiziert werden.

Eine Vorhersage der TM-Bereiche für ee3_1b_h sieht folgendermaßen aus:

| -----> STRONGLY prefered model: N-terminus outside | | | | | | |
|---|---|---|---|---|---|---|
| 4 strong transmembrane helices, total score : 6315 | | | | | | |
| # | from | to | length | score | orientation | |
| 1 | 15 | 33 | (19) | 2066 | o-i | (o:outside, i: inside) |
| 2 | 40 | 56 | (17) | 1143 | i-o | |
| 3 | 83 | 102 | (20) | 1765 | o-i | |
| 4 | 112 | 134 | (23) | 1341 | i-o. | |

Dieses Spleißprodukt hat eine funktionelle Bedeutung bei der Regulation der Funktion der Vollängen-Rezeptoren, vgl. bspw. V2-Vasopressin-Rezeptor (Zhu und Wess, 1998, Biochemistry, 37, 15773-84; Schulz, et al., 2000, J Biol Chem, 275, 2381-9)). Da GPCR-Proteine Homo- oder Heterodimerisierungen unterliegen (Bouvier, 2001, Nat Rev Neurosci, 2, 274-86.), können derartige trunkierter Formen erfindungsgemäßer Sequenzen eine dominant negative Rolle einnehmen.

Damit wird erfindungsgemäß insbesondere die Verwendung derartiger Spleißformen (bspw. als nackte DNA, in einem erfindungsgemäßen Expressionsvektor, als erfindungsgemäße Proteinsequenz etc.) erfindungsgemäßer ee3-Proteine sowie Varianten solcher Spleißformen zur Herstellung von Arzneimitteln zur Behandlung von Erkrankungen, wie vorliegend offenbart, offenbart. Ebenso umfaßt die Offenbarung auch deren Verwendung zur Untersuchung der pharmakologischer Beeinflußbarkeit von erfindungsgemäßen Rezeptoren der ee3-Familie.

### Ausführungsbeispiel 6

### Proteintopologiedaten von Proteinen der ee3-Familie

Eine TM(Transmembran)-Suche mit dem Programm TMPred ergibt folgendes stark favorisiertes Modell:

### (a) ee3_1

| -----> STRONGLY prefered model: N-terminus outside | | | | | | |
|---|---|---|---|---|---|---|
| 7 strong transmembrane helices, total score : 11863 | | | | | | |
| # | from | to | length | score | orientation | |
| 1 | 15 | 33 | (19) | 2066 | o-i | FLIYACLLLFSVLLALRLD |
| 2 | 40 | 56 | (17) | 1143 | i-o | YWAVFAPIWLWKLMVIV |
| 3 | 83 | 102 | (20) | 1765 | o-i | AMLIAVGIHLLLLMFEVLVC |
| 4 | 112 | 134 | (23) | 1341 | i-o | WLLVFMPLFFVSPVSVAACVWGF |
| 5 | 168 | 191 | (24) | 2978 | o-i | WLWCVPLWILMSFLCLWLYYIV |
| 6 | 211 | 227 | (17) | 1070 | i-o | ITMALSWMTIWPLLTF |
| 7 | 240 | 258 | (19) | 1500 | o-i | AFSCIPIFVPLWLSLITLM |

Abstände der Segmente zwischen den TM-Domänen beträgt 15, 7, 27, 10, 34, 20, 13 AS, der intracelluläre Rest hat eine Länge von 92 AS.

### (b) für ee3_2 ergibt sich ein identisches Bild:

| -----> STRONGLY prefered model: N-terminus outside | | | | | |
|---|---|---|---|---|---|
| 7 strong transmembrane helices, total score : 12351 | | | | | |
| # | from | to | lenght | score | orientation |
| 1 | 15 | 36 | (22) | 1811 | o-i |
| 2 | 32 | 50 | (19) | 1219 | i-o |
| 3 | 83 | 102 | (20) | 1733 | o-i |
| 4 | 112 | 134 | (23) | 1330 | i-o |
| 5 | 168 | 191 | (24) | 3068 | o-i |
| 6 | 211 | 227 | (17) | 1239 | i-o |
| 7 | 243 | 260 | (18) | 1951 | o-i |

Abstände der Segmente zwischen den TM-Domänen beträgt 15, 0, 33, 10, 34, 20, 16 AS und der Rest hat eine Länge von 90 AS.

### (c) Als Kontrollexperiment dient zum Vergleich die Topologie des CCR-5 Rezeptors (gehört ebenfalls zur Klasse der 7TM-Rezeptoren) aus dem Stand der Technik:

| | | | | | |
|---|---|---|---|---|---|
| 1 | 51 | 76 | (26) | 2895 | o-i |
| 2 | 89 | 108 | (20) | 1155 | i-o |
| 3 | 124 | 145 | (22) | 1163 | o-i |
| 4 | 163 | 187 | (25) | 1415 | i-o |
| 5 | 220 | 239 | (20) | 2183 | o-i |
| 6 | 260 | 281 | (22) | 1782 | i-o |
| 7 | 298 | 325 | (28) | 1325 | o-i |

Die Abstände der Segmente zwischen den TM-Domänen betragen 51, 13, 16, 18, 33, 21 und 17 AS, und der intrazelluläre Rest hat eine Länge von 46 AS.

Ein Vergleich der generellen Topologie (gezeigt ist die Anzahl der Aminosäuren in den jeweiligen nicht-transmembranen Anteilen der Proteine, d.h. N-Terminus und C-Terminus und die Loop-Anteile) der entfernt verwandten 7TM-Rezeptoren Bradykinin-2, CXCR5, Galaninrezeptor-2 und Anaphylotaxin C5a ergibt folgendes Bild im Vergleich zum erfindungsgemäßen ee3_1:

| rezeptor | n-terminus | 1 | 2 | 3 | 4 | 5 | 6 | rest |
|---|---|---|---|---|---|---|---|---|
| C5a | 39 | 12 | 14 | 22 | 28 | 16 | 20 | 45 |
| BK-2 | 63 | 13 | 14 | 20 | 27 | 26 | 24 | 56 |
| galanin2 | 28 | 10 | 17 | 19 | 24 | 25 | 1 | 105 |
| cxcr-5 | 55 | 11 | 14 | 21 | 30 | 18 | 23 | 46 |
| mw | 43.3 | 11.7 | 15.0 | 20.3 | 26.3 | 22.3 | 15.0 | 63.0 |
| ee3_1 | 15 | 7 | 27 | 10 | 34 | 20 | 13 | 92 |

Es findet sich eine deutliche Ähnlichkeit der generellen Topologie in diesen Proteinen.

### Ausführungsbeispiel 7

### Ermittlung von Motiven und Signalsequenzen bei ee3_1

Unter Verwendung des Programms Prosite:
Matching pattern PS00001 ASN_GLYCOSYLATION
AS 294: NISY
Total matches: 1
Matching pattern PS00006 CK2_PHOSPHO_SITE
AS 77: TCVE
Total matches: 1
Matching pattern PS00008 MYRISTYL
AS 57: GASVGT
AS 263: GQKGGN
Total matches: 2

Damit befinden sich eine CK2-Phosphorylierungsstelle an Position 77, eine Asparagin-Glykosilierungsstelle an Position 294 und 2 Myristylierungsstellen an den Positionen 57 und 263 (durchgezählte Numerierung gemäß Figur 7). Es findet sich keine typische Phosphorylierungsstelle im Carboxyterminus.

### Ausführungsbeispiel 8

### Induktion von ee3 durch eine einmalige Gabe von Erythropoetin

Wie in Fig. 19 gezeigt wurde, wird ee3_1 in der Ratte durch eine einmalige intraperitoneale Injektion von Erythropoetin (Erypo, Janssen; 5000 U / kg Körpergewicht) auf transkriptioneller Ebene induziert. 6 und 24 h nach Injektion von Erythropoetin wurde die Ratte durch Injektion von Rompun / Ketanest terminal betäubt, und das Gehirn schonend entnommen. éKontrollratten wurden mit Kochsalz behandelt. Zur Messung der ee3_1-Boten-RNA in der Ratte wurde die semiquantitative RT-PCR auf dem LightCycler (Roche, Mannheim, Germany) eingesetzt. Die Quantifizierung erfolgte durch den Vergleich der relativen Fluoreszenz der Probe mit einer Standardkurve für Cyclophilin.

Gesamt-RNA wurde aus Rattenvorderhirn (ohne Cerebellum und Bulbus olfactorius) isoliert mit der Methode von Chomczynski/Sacchi (saure Phenol-Extraktion) gefolgt von einer Aufreinigung mit dem RNeasy-Extraktionskit gemäß den Anweisungen des Herstellers (Qiagen, Santa Clarita, CA, USA). Die RNA-Konzentration wurde photometrisch bestimmt und die Qualität der Gesamt-RNA über Agarose-Gelelektrophorese beurteilt. Die RNA wurde bis zum Gebrauch bei -80 °C aufbewahrt. Nach reverser Transkription mit Superscript II (Invitrogen-Life Technologies, Carlsbad, CA, USA) wurden die Reaktionsprodukte durch Real-time online PCR mittels der LightCycler-Technologie relativ quantifiziert. Dafür wurden Gesamt-RNA Proben aus dem Gehirn von drei Wildtyp-Mäusen und drei transgenen tg6-Mäusen eingesetzt. Die spezifischen Sequenzen der Oligonukleotid-Primer lauteten für Cyclophilin
5'ACCCCACCGTGTTCTTCGAC-3'
für den Vorwärts- und
5'CATTTGCCATGGACAAGATG-3'
für den Rückwärts-Primer bei einer Bindungstemperatur von 60 °C und für Ratten ee3_1:
Vorwärts-Primer: 5'-GGTGTGGGAGAAATGGCTTA-3', Rück-wärts-Primer: 5'-ATACCAGCAGAGCCTGGAGA-3'.

Zur Quantifizierung wurden serielle cDNA-Verdünnungen von 1:3, 1:9, 1:27, 1:81 und 1:243 nach folgendem Schema amplifiziert: Initiale Denaturierung 5 min bei 94°C, Amplifizierung über 50 Zyklen bestehend aus 5 s Denaturierung bei 94°C, 10 s Bindung bei 55°C oder 60°C - je nach spezifischen Primer (s. o.) - und 30 s Extension bei 72°C. Am Ende jedes Zyklus wurde die Fluoreszenz jeder Probe bei 80°C für 10 s gemessen. Die Spezifität des Reaktionsproduktes wurde durch Agarose-Gelelektrophorese und Schmelzkurvenanalyse (nicht gezeigt) nachgewiesen. Jede PCR-Reaktion brachte genau ein Reaktionsprodukt hervor.

Für die Quantifizierung wurde die logarithmische Phase der PCR-Reaktion benutzt. Dabei wird eine Asymptote durch die entsprechende Kurve gelegt. Für Hämoglobin ergab sich ein nahezu paralleler Anstieg der Geradenschar, so daß die Steigungen dieser Kurven zum Vergleich mit den Standardkurven für Cyclophilin herangezogen werden konnten. Mittelwerte ± Standardabweichung wurden für jede cDNA-Verdünnung aus dem normalisierten PCR-Produkt bestimmt. Die so erhaltenen quantitativen Unterschiede entsprechen relativen Veränderungen der RNA-Expression in transgenen und Wildtyp-Tieren. Alle Reaktionen führten zu einem einzigen Reaktionsprodukt. Der mittlere Induktionsfaktor betrug für ee3 nach 6 Stunden 1,35, und nach 24 h 1,44-fach.

### Ausführungsbeispiel 9

### Verteilung von ee3_1 RNA im Gehirn

Mittels *in-situ*-Hybridisierung mit einer radioaktiv markierten Oligosonde wurde die Lokalisation des Transkriptes von ee3_1 bei der Maus untersucht. Dazu wurden 15 µm dicke Gehirnschnitte mit einem Kryostat bei -20° geschnitten, auf Poly-L-Lysin beschichtete Objektträger aufgezogen und in 4% Paraformaldehyd in PBS (pH 7.4) fixiert. Das Oligonukleotid wurde mittels Terminaler Transferase (Roche Diagnostics, Mannheim) mit α-³⁵S-dATP radioaktiv markiert. Markierung wie anschließende Hybridisierung erfolgten nach einem Protokoll von Wisden & Morris (In situ-Hybridization Protocols for the brain, Academic Press 1994).

Mit der eingesetzten radioaktiv markierten Sonde (ee3_1.3as AACGAAGGGCCAGTAGCACAGAGAACAGCAGCAGACAGGCATAGATGAGG) konnte die Expression von ee3_1 im Cerebellum (ce), Hippocampus (hc), Gyrus Dentatus (dg) und im Cortex (co), insbesondere im entorhinalen Cortex (ent), im Bulbus olfactorius (olf) sichtbar gemacht werden. Eine entsprechende sense-Kontrolle (ee3_1.3s,CCTCATCTATGCCTGTCTGCTGCTGTTCTCTGTGCTACTGGCCCTTCGTT ) ergab kein spezifisches Signal (nicht gezeigt) (Fig. 20).

### Ausführungsbeispiel 10

### Immunohistochemische Darstellung der ee3_1 Verteilung in Mausgewebe

Paraffin-eingebettetes Gewebe wurde geschnitten (2 µm), auf vorbehandelte Objektträger (DAKO, Glostrup, Denmark) aufgezogen, über Nacht luftgetrocknet, und anschliessend deparaffiniert (Xylol und absteigende Alkoholreihe). Nach Mikrowellenbehandlung in Zitratpuffer bei 500W für 10 min wurden die Schnitte mit Anti-ee3_1-serum (AS4163) in einer Verdünnung von 1:500 für 1 h bei Raumtemperatur in einer feuchten Kammer inkubiert. Die Immunreaktion wurde durch ABC Technik mit DAB als Chromogen entsprechend den Angaben des Herstellers (DAKO, Glostrup, Denmark) sichtbar gemacht. Negativkontrollen beinhalteten gleichermassen behandelte Schnitte, bei denen jedoch der Erstantikörper weggelassen wurde sowie Schnitte, bei denen statt des ersten Antikörpers das entsprechende Präimmunserum verwendet wurde.

Die Ergebnisse der immunhistochemischen Färbungen sind in Fig. 22 dargestellt. Insgesamt zeigt sich eine weitgehend neuronenspezifische Lokalisation von ee3, mit Ausnahme einiger Strukturen in Darm und Lunge. Häufig wird ee3 von Neuronen exprimiert, die integrative Aufgaben haben (die grossen Pyramidenzellen der corticalen Schicht (Layer) V, Mitralzellen im Bulbus olfactorius, Purkinjezellen im Kleinhirn). Die Bilder A-C zeigen kortikale Lokalisationen von ee3 im Layer V, mit deutlicher Anfärbung der Neuronenfortsätze. Im entorhinalen Kortex ist eine intensivere Immunfärbung zu sehen (C). Aus physiologischer Sicht gehen vom entorhinalen Cortex Informationen zum Hippocampus und tragen zu Lernen und Gedächtnis bei.

Alterationen des entorhinalen Cortex werden häufig bei Patienten mit Schlaganfall, Alzheimerscher Erkrankung oder nach Schädel-Hirn-Trauma gefunden. Durch Störungen des entorhinalen Cortex können Verhaltensänderungen auftreten, die mangelnde Verarbeitung sensorischer Eindrücke und Lernschwächen einschließen. (Davis et. Al, Nurs Res 50 (2) 77-85 (2001)). Die Bilder D-F zeigen das hippocampale Verteilungsmuster von ee3. Dabei ist die scharfe Grenze zwischen der Expression im CA3 Sektor, und der fehlenden Expression in den Sektoren CA2 und CA1 auffällig. Neurone der CA1-Region und in geringerem Ausmaß auch der CA4-Region sind besonders anfällig für den nekrose- und entzündungsfreien physiologischen Zelltod (Apoptose), insbesondere dann wenn eine allgemeine zentralnervöse Schädigung besteht (z.B.(Hara, et al., Stroke, 31, 236-8, (2000)). Im Gegensatz dazu scheint der Gyrus dentatus eher von einer nekrotischen Schädigung betroffen zu sein. Der Gyrus dentatus wird mit Neuronenneubildung nach pathologischen Stimuli in Verbindung gebracht (Takagi, et al., Brain Res, 831, 283-7, (1999)) (Parent, et al., J Neurosci, 17, 3727-38, (1997)). In Gebieten nicht neokortikaler Genese findet sich ee3 ebenfalls: Im Kleinhirn in den Purkinjezellen (G,H), die dort als integrierende Neurone wirken, und im Bulbus olfactorius in den Mitralzellen (I,J). Eine intensive Expression von ee3 findet sich in den Ganglienzellen und in den Sinneszellen der Retina (K,L).

Kürzlich wurde die neuroprotektive Wirkung von Erythropoetin in der Retina gezeigt(Junk et al., Erythropoietin administration protects retinal neurons from acute ischemiareperfusion injury. Proc Natl Acad Sci U S A. 2002 Aug 6;99(16):10659-64.; Grimm et al., HIF-1-induced erythropoietin in the hypoxic retina protects against light-induced retinal degeneration. Nat Med. 2002 Jul;8(7):718-24.) Diese Befunde legen nahe, daß ein Zusammenhang zwischen EPO-Induktion und ee3-Expression besteht. EE3 ist ebenfalls stark in Neuronen exprimiert, die dem motorischen System angehören. So findet sich im Rückenmark eine spezifische Expression in den grossen Motoneuronen des Vorderhorns (Fig. 22 M) und in den funktionell gleichwertigen Neuronen des motorischen Kerns des Nucleus trigeminus (Fig.. 22 N).

Diese Verteilung von ee3 im Rückenmark kann möglicherweise zur therapeutischen und diagnostischen Intervention bei Amyotropher Lateralsklerose ausgenutzt werden. Die Amyotrophe Lateralsklerose (ALS; Lou-Gehrig's disease; Charcot'sche Erkrankung) ist eine neurodegenerative Erkrankung mit einer jährlichen Inzidenz von 0.4 bis 1.76 pro 100.000 (Adams et al., Principles of neurology, 6th ed., New York, pp 1090-1095). Es ist die häufigste Form der Motoneuronerkrankungen mit typischen Manifestationen wie generalisierten Faszikulationen, progressiver Atrophie and Schwäche der Skelettmuskulatur, Spastizität und positiven Pyramidenbahnzeichen, Dysarthrie, Dysphagie, and Dyspnoe. Die Pathologie besteht hauptsächlich im Verlust von Nervenzellen im Vorderhorn des Rückenmarks und in den Motorkernen des unteren Hirnstammes, kann aber auch die Motoneuronen erster Ordnung im Kortex betreffen. Die Pathogenese dieser Erkrankung ist grösstenteils unbekannt, obwohl die Rolle von Superoxiddismutasemutationen in familiären Fällen sehr gut herausghearbeitet wurde. Bis jetzt wurden über 90 Mutationen im SOD1 Protein beschrieben, die ALS auslösen können (Cleveland and Rothstein (2001), Nat Rev Neurosci, 2, 806-19.). Auch Neurofilamente scheinen bei dieser Erkrankung eine Rolle zu spielen. Exzitotoxizität, ein Mechanismus der durch einen Überschuss an Glutamat ausgelöst wird, ist ein weiterer pathogenetischer Faktor, was durch die Wirkung von Riluzol in humanen Patienten belegt werden kann. Aktivierung von Caspasen und Apoptose scheint die gemeinsame Endstrecke der Pathogenese der ALS zu sein (Ishigaki, et al. (2002), J Neurochem, 82, 576-84. , Li, et al. (2000), Science, 288, 335-9.). Die Lokalisation des ee3 Proteins auf den bei ALS betroffenen Neuronen zeigt eindeutig die potentielle therapeutischfunktionell/ diagnostische Anwendbarkeit von ee3 Agonisten oder -Antagonisten bei dieser Erkrankung.

Die Lokalisation von ee3 in der Substantia nigra des Mittelhirns (Fig. 22 O,P) eröffnet möglicherweise therapeutische und diagnostische Möglichkeiten beim Morbus Parkinson. Die Parkinsonsche Erkrankung ist die häufigste Bewegungserkrankung (movement disorder) mit ungefähr 1 Million Patienten in Nordamerika. Ca. 1% der Bevölkerung über 65 Jahre ist betroffen. Die Hauptsymptome sind Rigor, Tremor, Akinesie (Adams et al., Principles of neurology, 6th ed., New York, pp 1090-1095). Die Ursache der Erkrankung ist nicht bekannt. Trotzdem weisen Analysen von post mortem Gewebe und von Tiermodellen auf einen fortschreitenden Prozess von oxidativem Stress in der Substantia nigra hin, der die dopaminerge Neurodegeneration unterhalten könnte. Oxidativer Stress, der durch die Neurotoxine wie 6-Hydroxydopamin und MPTP (N-Methyl-4-phenyl-1,2,3,6-tetrahydropyridin) hervorgerufen werden kann, wird in Tiermodellen benutzt, um den Vorgang der Neurodegeneration zu untersuchen. Obwohl eine symptomatische Therapie existiert (e.g. L-DOPA plus einen Decarboxylase Inhibitor; Bromocriptin, Pergolid als Dopamin Agonisten und anticholinerge Substanzen wie Trihexyphenidyl (Artane)), gibt es klaren Bedarf für eine kausale, d.h. neuroprotektive Therapie, die den Krankheitsprozess aufhalten kann. Apoptotische Mechanismen sind eindeutig an der Pathogenese im Tiermodell wie im Menschen beteiligt (Mochizuki, et al. (2001), Proc. Natl. Acad. Sci. U S A, 98, 10918-23, Xu et al. (2002), Nat. Med., 8, 600-6, Viswanath, et al. (2001), J. Neurosci., 21, 9519-28, Hartmann, et al. (2002), Neurology, 58, 308-10).

Die Lokalisationvon ee3 im Nervensystem ist in hohem Maße evident für einen Zusammenhang zwischen der Expression dieses Proteins und neuronalem Zelltod, Neurogenese und neuraler Plastizität.

In der Lunge findet sich ee3 in distinkten Strukturen (Fig. 22 Q-V). Es finden sich basale Zellen in den termianlen Bronchioli, die ee3 exprimieren. Möglicherweise handelt es sich dabei um neuroendokrin aktive Zellen. Diese Lokalisation legt eine therapeutische Bedeutung für ee3 für Erkrankungen der Bronchien nahe. Ebenso gibt es eine Expression n den Endothelien und glatten Muskelzellen von Arteriolen (12 U, V). Dahingegen zeigen Venolen keine immunhistochemisch faßbare Expression von ee3 (Fig. 22 Q und R). Die Expression bestimmter Rezeptoren durch Endothelzellen ist von grosser pharmakologischer Bedeutung, da Therapeutika sofortigen Kontakt mit dieser Zellschicht im Blut haben. Wichtige kreislaufwirksame Medikamente greifen an den Arteriolen an, inbesondere dem Endothel oder der glatten Muskulatur. Deshalb ist ee3 ein sehr attraktives Zielprotein zur Beeinflussung von Erkrankungen des Kreislaufs, z.B. dem arteriellen Hochdruck.

Im Darm findet sich ee3 basal in den Krypten (Fig. 22 W, X) und in Nervenzellen , die vermutlich zum darmeigenen Plexus gehören (Fig. 22 Y). In den meisten histologisch untersuchten Organen findet sich keine spezifische Anfärbung organspezifischer Zellen, sondern vielfach nur eine deutliche Anfärbung von Nerven (siehe z.B. im Herzmuskel Fig. 22 AA, oder im Bindegewebe Fig. 22 Z). Diese klare Lokalisation von ee3 auf Axonen prädisponiert das Molekül für die Diagnostik und Therapie von Erkrankungen der peripheren Nerven (Neuropathien), zu denen z.B. die weit verbreitete diabetische Polyneuropathie zählt. Ebenso könnten häufige erbliche Erkrankungen des peripheren Nervensystems, z.B. die HMSN Gruppe (hereditary motor-sensory neuropathies) davon profitieren.
Im Skelettmuskel schliesslich fand sich ein Expressionsmuster, das am ehesten mit der Lokalisation auf motorischen Endplatten zu vereinbaren ist (Fig. 22 BB). Dies ist potentiell interessant für Erkrankungen der motorischen Endplatte, z.B. der Myasthenia gravis.

### Ausführungsbeispiel 11

### ee3 wird auf Proteinebene in EPO-überexprimierenden Mäusen hochreguliert

Durch die Immunhistochemie (Antiserum AS 4163) konnte auch klare Hochregulation des ee3 Proteins durch Erythropoetin gezeigt werden (Fig. 23). Parallel behandelte Schnitte aus dem ZNS weisen klar verstärkte Signale für ee3 auf. Dies ließ sich an insgesamt je 3 Mäusen (Wildtyp und EPO überexprimierend (tg6)) zeigen, die jeweils geblindet gegenüber dem Genotyp gefärbt und beurteilt wurden.

### Ausführungsbeispiel 12

### Rolokalisierung von ee3 und map1b, und Abwesenheit von ee3 Expression in map1b defizienten Mäusen

In einem yeast-two-hybrid Screen mit dem Carboxyterminus von ee3_1 wurden die leichten Ketten von Map1a und Map1b als interagierende Proteine identifiziert (siehe vorhergehende Beispiele, Ausführungsbeispiel 4). Figur 24 zeigt eine Doppelimmunfluoreszenz für ee3_1 und map1b in der Maus, und demonstriert die unerwartete überlappung der Expression von ee3_1 und map1b in der Maus.

Für Doppelimmunfluoreszenzfärbungen wurden entparaffinierte Schnitte nach Mikrowellenbehandlung (Zitratpuffer, 500W, 10 min) simultan mit dem ee3_1 Antikörper aus Kaninchen (AS4163) und einem gegen map 1a gerichteten Antikörper aus Ziege (MAP-1B (C20): sc-8971; Santa Cruz; Santa Cruz, USA) für eine Stunde bei Raumtemperatur in einer feuchten Kammer inkubiert. Nach entsprechenden Waschschritten wurde mit einem Gemisch der sekundären Antikörper FITC anti-Kaninchen und TRITC anti-Ziege für 30 min inkubiert (beide Antikörper jeweils 1:30 verdünnt in PBS, bezogen von Dianova, Hamburg, Deutschland). Nach erneutem Waschen der Schnitte mit PBS wurden die Päparate mit Histosafe eingedeckelt und in einem Fluoreszenzmikroskop (Olympus IX81, Olympus, Deutschland) unter Verwendung entsprechender Barrierefilter analysiert. Signalüberlagerungen wurden mit Hilfe der Software Analysis (soft imaging systems, Stuttgart, Deutschland) angefertigt. In parallelen Fluoreszenz-Einzelfärbungen zeigte sich jeweils die Abwesenheit von Signal im anderen Kanal, was das Phänomen des "Überstrahlens" von Signalen in den jeweils anderen Kanal, z.B. durch Insuffizienz der Filter, ausschließt. Ebenso ergibt eine Doppelfärbung mit vertauschten Chromophoren für den Zweitantikörper dasselbe Bild (nicht gezeigt).

Figur 24 zeigt eine erstaunliche Übereinstimmung der Lokalisation der beiden Proteine im ZNS. Grün: ee3_1 Färbung; Rot: Map1b Färbung; Gelb: elektronische Überlagerung beider Signale. Gezeigt sind Beispiele aus dem Rückenmark (spinal cord; sc) und aus dem Cerebellum (cb).

Map1b ist ein wichtiges neuronales Protein. Es ist eines der ersten microtubule associated proteins (maps), die während der Entwicklung des zentralen Nervensystems der Säuger exprimiert werden. Eine Beteiligung an der Axongenese in Neuronen ist wahrscheinlich (Gonzalez-Billault, et al. (2001), Mol Biol Cell, 12, 2087-98., Gonzalez-Billault, et al. (2002), Brain Res, 943, 56-67.). Eine funktionell wichtige Rolle von map1b für die Interaktion von Neuronen wird durch jüngste Studien gestützt, die zeigen, daß map1b in die Pathogenese des Fragilen X Syndroms involviert ist. Dies ist die häufigste vererbbare Form von mentaler Retardierung. Die mRNA von map1b wird von FMRP, einem Protein, das direkt von der Fragile X mRNA reguliert wird, kontrolliert. In einer Arbeit in Drosophila wurde eine zentrale Rolle für map1b (futsch in Drosophila) für die Ausprägung des Fragile-X analogen Phänotyps gefunden (Sohn (2001), Science, 294, 1809. , Zhang, et al. (2001), Cell, 107, 591-603.). Ebenso bindet map1b an gigaxonin, ein Protein dessen mutiertes Gen für die rezessive erbliche Erkrankung Giant axonal neuropathy (GAN) verantwortlich ist (Ding, et al. (2002), J Cell Biol, 158, 427-33.). Nach Läsion peripherer Nerven wird map1b verstärkt in den auswachsenden Neuronen myelinisierter Nerven induziert, und ist wahrscheinlich am axonalen Sprouting beteiligt (Soares, et al. (2002), Eur J Neurosci, 16, 593-606.). Schliesslich lokalisiert map1b wahrscheinlich den GABA-C Rezeptor an seine synaptische Lokalisation (Pattnaik, et al. (2000), J Neurosci, 20, 6789-96.).

Das Map1b Gen wurde in Mäusen genetisch inaktiviert (knock-out). Der beste verfügbare Knock-out ist die Maus die in Meixner et al. beschrieben ist (Meixner, Haverkamp, Wassle, Fuhrer, Thalhammer, Kropf, Bittner, Lassmann, Wiche and Propst (2000), J Cell Biol, 151, 1169-78.). Mäuse mit homozygoter Inaktivierung des map1b Gens wurden immunhistochemisch auf ee3 Expression untersucht. Es zeigte sich eine sehr stark reduzierte Anfärbbarkeit von ee3_1 (Figur 25), was auf eine Abhängigkeit der ee3_1 Proteinexpression oder - stabilität von der Interaktion mit map1b hindeutet. Ein Inhibitor dieser Interaktion würde also eine deutlich verringerte Expression von ee3 zur Folge haben.

### Ausführungsbeispiel 13

### Herstellung eines Antiserums für den Nachweis von ee3_1

Die Proteinsequenz des humanen ee3_1 Proteins wurde mit dem Teilprogramm Protean des Programmpackets DNAStar (Lasergene) analysiert und entsprechend der Sekundärvorhersage sowie hoher vorhergesagter Oberflächenwahrscheinlichkeit und hoher Antigenizität das im intrazellulären C-Terminus gelegene Epitop LHHEDNEETEETPVPEP entsprechend den Aminosäuren 299-315 ausgewählt. Diese Peptidsequenz wurde mit N-terminal angehängtem Cystein synthetisiert (CLHHEDNEETEETPVPEP) um eine kontrollierte spezifische Kopplung an das Trägerprotein KLH (keyhole limpet hemocyanine) zu ermöglichen. Mit dem Peptid-KLH-Konjugat wurden zwei Kaninchen nach einem optimierten Plan immunisiert. Die Peptidsynthese, die anschließende Kopplung an KLH sowie die Immunisierung von zwei Kaninchen wurden bei der Firma BioTrend Chemikalien GmbH in Auftrag gegeben. Vor der Erstimmunisierung wurde das Präimmunserum mehrerer Kaninchen in Western Blot Analysen von *mock*transfizierten Zellen und Gehirnextrakten auf ihre Kreuzreaktivität getestet. Zwei Kaninchen mit vernachlässigbarem Hintergrund erhielten 3 Wochen nach der Erstimmunisierung den ersten und weitere 4 Wochen später den zweiten Boost. Eine Woche später wurden den Kaninchen 20 ml Blut entnommen und die Seren in Western Blot Analysen und immuncytochemischen Färbungen von transient transfizierten Zellen (HEK293, CHO-dhfr-) getestet. Nach dem 3. bzw. 4. Boost wurden die Kaninchen ausgeblutet.

Die Seren beider Kaninchen waren positiv. Insbesondere das Antiserum AS4163 erkannte mit beiden Methoden sehr spezifisch in verschiedenen Zellen transient exprimiertes humanes ee3_1 Protein und konnte in Western Blot Analysen bis zu einer Verdünnung von 1:12000 eingesetzt werden. Das Antiserum AS4163 eignet sich auch zur Immunpräzipitation von ee3_1 Protein und kann daher zur Präzipitation von ee3_1 und damit interagierender Proteine aus transfizierten Zellen oder nativem Gewebe verwendet werden. Insbesondere erkennt das Antiserum AS4163 das entsprechende Epitop in der ee3_1 Sequenz der Maus, das sich von der humanen Sequenz nur durch eine Aminosäure, nämlich die Mutation von N304 zu Serin unterscheidet. Das Antiserum AS4163 eignet sich daher sehr gut zur immunhistochemischen Analyse der ee3_1 Proteinexpression in Wildtyp, transgenen und knock-out Mäusen, wie Figuren 22-24 zeigen.

### Ausführungsbeispiel 14

### ee3_1 wird von neuralen Stammzellen exprimiert

Neurale Stammiellen wurden aus dem Hippocampus von 4-6 Wochen alten männlichen Wistar Ratten isoliert wie vorbeschrieben (Ray et al., 1993). Die Protokolle genügen deutschem Recht. Die Tiere wurden mit 1 % (v/v) Isofluran, 70 % N20, 29 % Sauerstoff betäubt und durch Dekapitation getötet. Die Hirne wurden präpariert und gewaschen in 50 mL eiskaltem Dulbecco's Phosphate Buffered Saline (DPBS) mit 4.5 g/L glucose (DPBS/Glc). Der Hippocampus wurde aus 6 Tieren herauspräpariert, gewaschen in 10 mL DPBS/Glc und 5 min lang zentrifugiert bei 1600 x g bei 4 °C. Nach Abnahme des Überstandes wurde das Gewebe homogenisiert mit Schere und Skalpell. Die Gewebestücke wurden mit DPBS/Glc Medium gewaschen, 5 min bei 800 g zentrifugiert, und das Pellet resuspendiert in 0.01 % (w/v) Papain, 0.1 % (w/v) Dispase II (neutrale Protease), 0.01 % (w/v) DNase I, and 12.4 mM Mangansulfat in Hank's Balanced Salt Solution (HBSS). Das Gewebe wurde mit Pipettenspitzen trituriert, und 40 min bei Raumtemperatur inkubiert, unter gelegentlichem Mischen der Lösung (alle 10 min). Die Suspension wurde anschließend bei 800 x g 5 min lang bei 4 °C zentrifugiert, und das Pellet dreimal in 10 mL DMEM-Ham's F-12 Medium mit 2 mM L-Glutamin, 100 units/mL Penicillin und 100 units/mL Strepotomycin gewaschen. Danach wurden die Zellen in 1 mL Neurobasalmedium mit B27 (Invitrogen, Carlsbad, CA, USA), 2 mM L-Glutamin, 100 units/mL Penicillin und 100 units/mL Strepotomycin, 20 ng/mL EGF, 20 ng/mL FGF-2, und 2 µg/mL Heparin resuspendiert. Die Zellen wurden unter sterilen Bedingungen in 6-well Platten in einer Konzentration von 25,000-100,000 Zellen/mL ausgesät. Die Platten wurden bei 37 °C in 5 % CO2 inkubiert. Zellkulturmedium wurde einmal in der Woche gewechselt, wobei ungefähr nur 2/3 des Mediums ausgetauscht wurden. (Ref: Ray J, Peterson DA, Schinstine M, Gage FH (1993) Proliferation, differentiation, and long-terrn culture of primary hippocampal neurons. Proc Natl Acad Sci U S A 90: 3602-6.).

RNA wurde nach Standardprotokollen (RNeasy kit, Qiagen) aus hippocampalen Stammzellen isoliert, die 3 Wochen in Kultur gehalten wurden, nachdem sie aus gefrorenen Stocks aufgetaut waren. cDNA wurde unter Benutzung von oligodT Primern und Superscript II Reverser Transcriptase (Gibco) nach Standardprotokollen synthetisiert. PCR wurde mit den folgenden Reaktionsparametern durchgeführt: Denaturierung 94 °C 10 min, 30 Zyklen zu 94 °C 30 s, 55 °C 50 s, 72 °C 60 s; 72 °C 5 min 4 °c mit folgenden Primerpaaren: ee3_plus 5'-GGTGTGGGAGAAATGGCTTA-3' und ee3_minus 5'-ATACCAGCAGAGCCTGGAGA-3'.

Während der letzten Jahre wurde die Bedeutung der Neubildung von Nervenzellen (Neurogenese) im Verlauf neurologischer Erkrankungen erkannt. Im Gegensatz zu vielen anderen Geweben hat das reife Gehirn begrenzte regenerative Kapazitäten, und der ungewöhnlich hohe Grad an zellulärer Spezialisierung schränkt die Möglichkeiten ein, durch gesundes verbleibendes Gewebe die Funktion des zerstörten Gewebes zu übernehmen. Nervenzellen die aus Vorläuferzellen im erwachsenen Gehirn entstehen haben jedoch prinzipiell das Potential, solche Funktionen zu übernehmen.

Neurogenese kommt in diskreten Regionen des erwachsenen Hirns vor, (die rostrale subventricular Zone (SVZ) der Seitenventrikel und der Subgranularzone (SGZ) im Gyrus dentatus (DG). Es wurde von vielen Gruppen gezeigt, daß Neurogenese insbesondere durch neurologische Schädigungen induziert wird (z.B.. cerebral ischemia (Jin, et al. (2001), Proc. Natl. Acad. Sci. USA, 98, 4710-5, Jiang, et al. (2001), Stroke, 32, 1201-7, Kee, et al. (2001), Exp. Brain. Res., 136, 313-20, Perfilieva, et al. (2001), J. Cereb. Blood Flow Metab., 21, 211-7)). Neurogenese kommt auch beim Menschen vor (E-riksson, et al. (1998), Nat Med, 4, 1313-7.), und führt tatsächlich zu funktionellen Neuronen (van Praag, et al. (2002), Nature, 415, 1030-4). Insbesondere die Subgranularzone des Gyrus dentatus und der Hilus haben das Potential neue Neuronen während des Erwachsenenlebens zu generieren ( Gage, et al. (1998), J Neurobiol, 36, 249-66). Es ist auffällig, daß ee3 auf neuronalen Stammzellen aus dem Hippokampus zu detektieren ist (Fig. 25). Dies impliziert eine wichtige Bedeutung von ee3 für die Neurogenese. Diese Bedeutung in der Neurogenese bedeutet einen weiteren Beweis für die Nützlichkeit von ee3 für generell alle neurodegenerativen Erkrankungen.

Im Gegensatz zur Wirkung auf endogene Stammzellen im Hirn können Therapeutika, die mit ee3 interferieren für die in vitro Manipulation von Stammzellen (z.B. in vitro Differenzierung und Proliferation). Stammzellen werden im Moment für ihre Verwendbarkeit in einer Anzahl neurodegenerativer Erkrankungen exploriert, insbesondere die Parkinsonsche Erkrankung und Schlaganfall. Es ist z.B. wünschenswert, Zellen in vitro für die Transplantation bei Parkinson-Patienten zu differenzieren, und damit nach Injektion das dopaminerge Defizit auszugleichen ("replacement therapy")(Arenas (2002), Brain Res. Bull, 57, 795-808, Barker (2002), Mov. Disord., 17, 233-41). Eine andere Möglichkeit zum Einbringen von Stammzellen sind z.B. i.a. oder i.v. Injektionen bei Schlaganfall oder Hirntrauma (Mahmood, et al. (2001), Neurosurgery, 49, 1196-203; discussion 1203-4, Lu, et al. (2001), J Neurotrauma, 18, 813-9, Lu, et al. (2002), Cell Transplant, 11, 275-81, Li et al. (2002), Neurology, 59, 514-23). Eine andere Möglichkeit der Verwendung von ee3 in der Stammzelltherapie wäre die Herstellung von Zellen, die konstant einen Agonisten oder Antagonisten für ee3 sezernieren.

### Ausführungsbeispiel 15

### Klonierung zusätzlicher Verowandter der ee3-Rezeptorfamilie aus Xenopus laevis und Dario rerio

Es konnten zusätzlicher Mitglieder der ee3-Proteinfamilie aufgrund der erfindungsgemässen Homologiekriterien kloniert werden. Mit TBLASTN wuredn mit Proteinsequenzen aus dem humanen ee3_1 Protein EST-Datenbanken durchsucht. Dabei fanden sich ESTs aus X. laevis (Krallenfrosch)und aus D. rerio (Zebrafisch). Diese ESTs wurden mit Standardverfahren sequenziert, und es ergaben sich folgende Sequenzen:
Vollänge-Sequenz von xl_ee3 (Xaenopus laevis): 
CCCGGGCACGTTACCGTATTGATGTTACTAGTAGCGCACAGAAACATCCTGGTCTAAGCAGTTGCAGCAGGTACTGCGTT
GTAGTGGCGGTAGTTACGACTCTGTAGGTTAGAGCGGAGGCTTTGCTGGAGCAATGTCCGCCTAGTGAAGCTCGGAGAGG
TGCTCGCACCATGAATCTTAGGGGCCTCTTCCAGGATTTAACCCCAGTAAATTTCTCATCTACGCATGTTTGTTGCTCT
TTTCTGTTCTCCTTTCCCTGCGACTGGATAATATTATTCAGTGGAGTTACTGGCGGTGTTTGCTCCAATATGGTTGTGG
AAACTAATGGTTATTGTGGGAGCCTCAGTTGGTACAGGTGTATGGGCACGTAACCCTCAATACAGGGCAGAAGGTGAAAC
ATGTGTGGAGTTCAAGGCCATGCTAATTGCAGTGGGAATTCATTTGCTGCTTCTTATGTTTGAAGTTCTTGTTTGCGATC
GTATTGAAAGAGGAAACCACTACTTCTGGTTGCTAGTCTTTATGCCTTTATTCTTTGTGTCCCCAGTATCCGTTGCAGCT
TGCGTTTGGGGCTTTCGGCATGATCGATCATTGGAATTGGAAATCTTGTGCTCCGTCAATATTCTGCAGTTTATATTCAT
TGCCCTAAGACTTGACAGCATCATCACTTGGCCTTGGCTTGTGGTATGTGTCCCGCTGTGGATCCTTATGTCCTTCCTGT
GCCTAGTAGTTCTGTATTATATTGTGTGGTCAGTTCTGTTCCTGCGTTCAATGGATGTTATTGCAGAACAAAGGAGAACT
CATATTACTATGGCAGTCAGTTGGATGGCTATAGTTGTACCGCTTCTGACATTCGAGATATTACTTGTTCATCGACTTGA
TGGGCACAATCCATTATCGAATATCCCTATATTTGTTCCGCTTTGGCTTTCCTTAATAACGTTGATGGCAACAACCTTTG
GACAGAAAGGAGGCAATCACTGGTGGTTTGGGATTCGTAAAGACTTCTGCCAGTTTCTGTTGGAGATTTTCCCTTTTCTT
CGAGAATATGGCAATATCTCATATGATATTCATCATGAAGACAGTGAAGATGCTGAAGAAACACCTGTACCGGAGCCCCC
CAAAATCGCACCAATGTTTCGAAAGAAGACTGGCGTTGTCATTACCCAGAGCCCAGGGAAATATATTGTTCCTCCTGCTA
AACTTAACATCGACATGCCGGATTAAGGTGAAATTTGGTGGCTTGAGGGCACTTTTTTCTGTTTTAACTAATCCTGTTAG
TAGTACACTATCAGGTGTCATGGACTGAAGGGAAAAAAAGACTACTGACCTCATTCCTTTTTTGTATTCATTTGTAATTT
TTTTTGTTCCTGCAATGGTATGTGTTTTCCCATTCCTAATTCATGTCATCATGTTACTCAAGATCAGGGAAGCTTCTTAA
GGGCAAAGAATGCTGGAATTTGTAGTTTATAATTTGTGGATGACTATAAATTTTCACATCTGTTGTCTTGGTAATGACTG
CAGTCTTGCATTCTAGTTTCTAGTAACACAGAGATAGACCAGCTGTGGCCCTCCAGATACTGAGCTAACAAGCTTTGGGA
GACATCCTGGGAATCTTAGCAGCTCTGGGGCCACAGGTTGGACTTCTCAGCAGTAAAATTAAGTATAATGTTTATCTTAA
GTAAATGTCTTTGTGTGTGTTGTTATGCAATGCAGCTATTGTTTGATATCTTTAcagcagaacttgtgcatagaattgaa
ttcaagttgtgagctgttttataccactataaaaatacttttAAAAAAAAATCTGTTTAAGGGTCAAGCATTACCTTGGA
GAAGTGATATTTGAGCAGAGGGCTTATGGGATATATCTAATATACACCTTCCCTTAGGAGTTACTACTCCTTGCTCACTT
GTATAGTATTTATAAGAACATTTTATCAATGTAATATATTGTGTTCAAAATTATTCTTATGTACAGTATAAATGGATAAA
TACAAAGTATTTTTTTAAATAAAAGATGTAAAATACATATAAGTTGTCAAAATTTTGTTTGTAATTTACATTTTAAAATG
ATCTATGTGAATTCTACAATGAAAAAAGATCTATACAATTTCAAAAGCCAGTATGTCATTTTTATATACTGACCATGTAC
ATATTATGTAAGATGTAAAGCCAAACACCAATGACATGAATGTTAAGTTATTAGACTATGAATAAAACATTGATTTTATT
TTATGTTGTAAAAAAAAAAAAAAAAAAA
Das offenen Leseraster ("open reading frame") von xl_ee3: 
ATGAATCTTAGGGGCCTCTTCCAGGATTTTAACCCCAGTAAATTTCTCATCTACGCATGTTTGTTGCTCTTTTCTGTTCT
CCTTTCCCTGCGACTGGATAATATTATTCAGTGGAGTTACTGGGCGGTGTTTGCTCCAATATGGTTGTGGAAACTAATGG
TTATTGTGGGAGCCTCAGTTGGTACAGGTGTATGGGCACGTAACCCTCAATACAGGGCAGAAGGTGAAACATGTGTGGAG
TTCAAGGCCATGCTAATTGCAGTGGGAATTCATTTGCTGCTTCTTATGTTTGAAGTTCTTGTTTGCGATCGTATTGAAAG
AGGAAACCACTACTTCTGGTTGCTAGTCTTTATGCCTTTATTCTTTGTGTCCCCAGTATCCGTTGCAGCTTGCGTTTGGG
GCTTTCGGCATGATCGATCATTGGAATTGGAAATCTTGTGCTCCGTCAATATTCTGCAGTTTATATTCATTGCCCTAAGA
CTTGACAGCATCATCACTTGGCCTTGGCTTGTGGTATGTGTCCCGCTGTGGATCCTTATGTCCTTCCTGTGCCTAGTAGT
TCTGTATTATATTGTGTGGTCAGTTCTGTTCCTGCGTTCAATGGATGTTATTGCAGAACAAAGGAGAACTCATATTACTA
TGGCAGTCAGTTGGATGGCTATAGTTGTACCGCTTCTGACATTCGAGATATTACTTGTTCATCGACTTGATGGGCACAAT
CCATTATCGAATATCCCTATATTTGTTCCGCTTTGGCTTTCCTTAATAACGTTGATGGCAACAACCTTTGGACAGAAAGG
AGGCAATCACTGGTGGTTTGGGATTCGTAAAGACTTCTGCCAGTTTCTGTTGGAGATTTTCCCTTTTCTTCGAGAATATG
GCAATATCTCATATGATATTCATCATGAAGACAGTGAAGATGCTGAAGAAACACCTGTACCGGAGCCCCCCAAAATCGCA
CCAATGTTTCGAAAGAAGACTGGCGTTGTCATTACCCAGAGCCCAGGGAAATATATTGTTCCTCCTGCTAAACTTAACAT
CGACATGCCGGATTAA
und die Proteinsequenz von xl_ee3: 
MNLRGLFQDFNPSKFLIYACLLLFSVLLSLRLDNIIQWSYWAVFAPIWLWKLMVIVGASVGTGVWARNPQYRAEGETCVE
FKAMLIAVGIHLLLLMFEVLVCDRIERGNHYFWLLVFMPLFFVSPVSVAACVWGFRHDRSLELEILCSVNILQFIFIALR
LDSIITWPWLVVCVPLWILMSFLCLVVLYYIVWSVLFLRSMDVIAEQRRTHITMAVSWMAIVVPLLTFEILLVHRLDGHN
PLSNIPIFVPLWLSLITLMATTFGQKGGNHWWFGIRKDFCQFLLEIFPFLREYGNISYDIHHEDSEDAEETPVPEPPKIA
PMFRKKTGVVITQSPGKYIVPPAKLNIDMPD
Für D. rerio lauten diese Sequenzen (dr_ee3): 
CTCGAGCACTGTTGGCCTACTGGGATGTGAGTGCCAGTCAGCTAGCCAGCCTCTCCTTTTCAGTTCATGTAACTATGGTC
TGAAGAGGAAACCATGAATCTCCGAGGCGTTTTCCAAGATTTCAACCCCAGTAAGTTCCTGATCTACGCATGTCTGCTGC
TCTTCTCTGTGCTGCTGTCACTGAGGCTGGATGGCATCATCCAGTGGAGCTACTGGGCCGTGTTTGCGCCCATCTGGCTC
TGGAAGCTCATGGTCATCATCGGGGCGTCTGTGGGCACTGGAGTGTGGGCTCACAACCCGCAGTACAGGGCTGAAGGGGA
GACGTGTGTGGAGTTTAAGGCCATGCTGATCGCAGTGGGAATCCACCTGCTCCTGCTCACCTTCGAGGTGCTGGTCTGCG
AGCGCGTGGAACGGGCTTCGATCCCCTACTGGCTCCTGGTGTTCATGCCGCTCTTCTTCGTCTCTCCGGTGTCAGTGGCA
 
GCGTGTGTGTGGGGATTCAGACACGACCGCTCGCTGGAGCTGGAGATTCTGTGCTCTGTAAATATTCTTCAGTTTATCTT
CATCGCTCTGAAACTGGACGGATCATCAGCTGGCCGTGGCTGGTGGTGTGTGTGCCGCTCTGGATCCTCATGTCCTTCT
TGTGTCTGGTGGTcctctattatatcgtgtggtctgTGCTGTTTCTGCGCTCCATGGATGTGATCGCGGAGCAGCGGCGC
ACACACATCACCATGGCCATCAGCTGGATGACTATAGTCGTGCCCCTGCTCACTTTTGAGATTCTCCTCGTCCACAAGCT
gGATAATCATTATAGCCCCAACTACGTcCCGGTGTTTGTTCCTCTCTGGGTTTCTTTAGTGACTCTAATGGTGACCACAT
TTGGCCAGAAAGGAGGCAATCACTGGTGGTTTGGCATCCGTAAAGACTTCTGCCAGTTTCTgCTGGAGCTCTTCCCGTTC
CTCAGGGAATATGGCAACATCTACTATGACCTGCATcACGAGGACTCAGACATGTcCGAGGAGTTGCCCATTCACGAGGT
GCCCAAAATCCCTACCATGTTTAgCAAGAAGACGGGGGTGGTGATCACCCAAAGCCCTGGGAAATACTTTGTGCCCCCAC
CCAAACTGTGCATCGACATGCCAGACTAACATTGGAGCTCTCGTATACAGTATAGCACTATGCAATGGAATTCGCTTTGT
TACGTgCTGTTGAAGACGGcAACAACAATCCCATTAAACTCGGCTCTTGTTTCCTAAAAAAAATAGCTGCGCAAACGGAC
CTGTTGACATCA
Das offene Leseraster von dr_ee3 ("open reading frame"): 
ATGAATCTCCGAGGCGTTTTCCAAGATTTCAACCCCAGTAAGTTCCTGATCTACGCATGTCTGCTGCTCTTCTCTGTGCT
GCTGTCACTGAGGCTGGATGGCATCATCCAGTGGAGCTACTGGGCCGTGTTTGCGCCCATCTGGCTCTGGAAGCTCATGG
TCATCATCGGGGCGTCTGTGGGCACTGGAGTGTGGGCTCACAACCCGCAGTACAGGGCTGAAGGGGAGACGTGTGTGGAG
TTTAAGGCCATGCTGATCGCAGTGGGAATCCACCTGCTCCTGCTCACCTTCGAGGTGCTGGTCTGCGAGCGCGTGGAACG
GGCTTCGATCCCCTACTGGCTCCTGGTGTTCATGCCGCTCTTCTTCGTCTCTCCGGTGTCAGTGGCAGCGTGTGTGTGGG
GATTCAGACACGACCGCTCGCTGGAGCTGGAGATTCTGTGCTCTGTAAATATTCTTCAGTTTATCTTCATCGCTCTGAAA
CTGGACGGGATCATCAGCTGGCCGTGGCTGGTGGTGTGTGTGCCGCTCTGGATCCTCATGTCCTTCTTGTGTCTGGTGGT
cctctattatatcgtgtggtctgTGCTGTTTCTGCGCTCCATGGATGTGATCGCGGAGCAGCGGCGCACACACATCACCA
TGGCCATCAGCTGGATGACTATAGTCGTGCCCCTGCTCACTTTTGAGATTCTCCTCGTCCACAAGCTgGATAATCATTAT
AGCCCCAACTACGTcCCGGTGTTTGTTCCTCTCTGGGTTTCTTTAGTGACTCTAATGGTGACCACATTTGGCCAGAAAGG
AGGCAATCACTGGTGGTTTGGCATCCGTAAAGACTTCTGCCAGTTTCTgCTGGAGCTCTTCCCGTTCCTCAGGGAATATG
GCAACATCTACTATGACCTGCATcACGAGGACTCAGACATGTcCGAGGAGTTGCCCATTCACGAGGTGCCCAAAATCCCT
ACCATGTTTAgCAAGAAGACGGGGGTGGTGATCACCCAAAGCCCTGGGAAATACTTTGTGCCCCCACCCAAACTGTGCAT
CGACATGCCAGACTAA
und die Proteinsequenz von dr_ee3:

MNLRGVFQDFNPSKFLIYACLLLFSVLLSLRLDGIIQWSYWAVFAPIWLWKLMVIIGASVGTGVWAHNPQYRAEGETCVE
FKAMLIAVGIHLLLLTFEVLVCERVERASIPYWLLVFMPLFFVSPVSVAACVWGFRHDRSLELEILCSVNILQFIFIALK
LDGIISWPWLVVCVPLWILMSFLCLVVLYYIVWSVLFLRSMDVIAEQRRTHITMAISWMTIVVPLLTFEILLVHKLDNHY
SPNYVPVFVPLWVSLVTLMVTTFGQKGGNHWWFGIRKDFCQFLLELFPFLREYGNIYYDLHHEDSDMSEELPIHEVPKIP
TMFSKKTGVVITQSPGKYFVPPPKLCIDMPD

Figur 27 verdeutlicht noch einmal die hohe evolutionäre Konservierung der ee3 Familie unter Zuhilfenahme der beiden Proteine aus X. laevis und D. rerio.

## Patentansprüche

1. Nukleinsäure, **dadurch gekennzeichnet, dass** sie für ein Polypeptid gemäß einer der Figuren 13 oder 15A kodiert, oder für Varianten davon, die mindestens 90 % Identität auf Aminosäureebene zu einem Polypeptid gemäß einer der Figuren 13 oder 15A aufweisen, ausgenommen eine Nukleinsäure gemäß SEQ ID NO:75 aus der WO01/98353.

2. Nukleinsäure nach Anspruch 1, **dadurch gekennzeichnet, dass** sie eine Sequenz gemäß einer der Figuren 9A oder 11 A für den translatierten Bereich (in Großbuchstaben) enthält.

3. Expressionsvektor, **dadurch gekennzeichnet, dass** er eine DNA-Sequenz nach einem der Ansprüche 1 oder 2 enthält.

4. Wirtszelle, **dadurch gekennzeichnet, dass** sie mit einem Expressionsvektor nach Anspruch 3 transformiert ist.

5. Wirtszelle nach Anspruch 4, **dadurch gekennzeichnet, dass** sie eine Säugetierzelle, insbesondere eine humane Zelle, ist.

6. Aufgereinigtes Genprodukt, **dadurch gekennzeichnet, dass** es durch eine DNA-Sequenz nach einem der Ansprüche 1 oder 2 kodiert wird, ausgenommen ein Polypeptid gemäß der SEQ ID NO:31 aus der WO01/98353.

7. Aufgereinigtes Genprodukt nach Anspruch 6, **dadurch gekennzeichnet, dass** es ein Polypeptid ist.

8. Nicht-menschliches transgenes Tier, **dadurch gekennzeichnet, dass** ihm sein natives ee3-1-Polypeptid gemäß Anspruch 7 oder Teile davon fehlt.

9. Antikörper, **dadurch gekennzeichnet, dass** er ein Epitop auf einem Genprodukt nach einem der Ansprüche 6 oder 7 erkennt.

10. Antikörper nach Anspruch 9, **dadurch gekennzeichnet, dass** er monoklonal ist.

11. Antikörper nach einem der Ansprüche 9 oder 10, **dadurch gekennzeichnet, dass** er gegen einen Sequenzabschnitt auf der extrazellulären Domäne als Epitop gerichtet ist.

12. Verfahren zu Expression von Genprodukten nach einem der Ansprüche 6 oder 7, **dadurch gekennzeichnet, dass** Wirtszellen mit einem Expressionsvektor nach Anspruch 3 transformiert werden.

13. Verfahren zur Isolierung von Genprodukten nach einem der Ansprüche 6 oder 7, **dadurch gekennzeichnet, dass** Wirtszellen nach Anspruch 4 oder 5 unter geeigneten, die Expression fördernden Bedingungen kultiviert werden und das Genprodukt anschließend aus der Kultur aufgereinigt wird.

14. Nukleinsäure nach einem der Ansprüche 1 oder 2 oder ein Genprodukt nach einem der Ansprüche 6 oder 7 als Arzneimittel.

15. Nukleinsäure nach einem der Ansprüche 1 oder 2 oder Genprodukt nach einem der Ansprüche 6 oder 7 zur Behandlung von neurologischen Erkrankungen.

16. Verfahren zur Identifizierung eines zellulären Interaktionspartners eines Polypeptids, das von einer Nukleinsäure nach einem der Ansprüche 1 oder 2 kodiert wird, wobei ein sogenanntes "yeast-two-hybrid"-System eingesetzt wird.

## Claims

1. A nucleic acid that codes for a polypeptide according to figure 13 or figure 15A, or variants thereof which at amino acid level have an identity of at least 90 % with respect to a polypeptide according to figure 13 or figure 15A, except for the nucleic acid of Seq. ID No: 75 of WO 01/98353.

2. The nucleic acid as claimed in claim 1, which comprises a sequence according to figure 9A or figure 11A for the translated region (in capital letters).

3. An expression vector, which comprises a DNA sequence as claimed in claim 1 or 2.

4. A host cell, which is transformed with an expression vector as claimed in claim 3.

5. The host cell as claimed in claim 4, which is a mammalian cell, particularly a human cell.

6. A purified gene product, which is encoded by a DNA sequence as claimed in claim 1 or 2, except for the polypeptide of Seq. ID No: 31 of WO 01/98353.

7. The purified gene product as claimed in claim 6, which is a polypeptide.

8. A non-human transgenic animal, which is lacking its native ee3-1 polypeptide as claimed in claim 7 or parts thereof.

9. An antibody, which recognizes an epitope on a gene product as claimed in claim 6 or 7.

10. The antibody as claimed in claim 9, which is monoclonal.

11. The antibody as claimed in claim 9 or 10, which is directed against a sequence section on the extracellular domain as epitope.

12. A method for expression gene products as claimed in claim 6 or 7, wherein host cells are transformed with an expression vector as claimed in claim 3.

13. A method for isolating gene products as claimed in claim 6 or 7, wherein host cells as claimed in claim 4 or 5 are cultured under suitable, expression-promoting conditions and the gene product is subsequently purified from the culture.

14. A nucleic acid as claimed in claim 1 or 2 or a gene product as claimed in claim 6 or 7 as a remedy.

15. A nucleic acid as claimed in claim 1 or 2 or a gene product as claimed in claim 6 or 7 for treating neurological disorders.

16. A method for identifying a cellular interaction partner of a polypeptide encoded by a nucleic acid as claimed in claim 1 or 2, using a so-called "yeast-two-hybrid" system.

## Revendications

1. Acide nucléique, **caractérisé en ce qu'**il code pour un polypeptide correspondant à l'une des figures 13 ou 15A ou pour des variantes de celui-ci qui ont une séquence d'acides aminés présentant au moins 90 % d'identité avec un polypeptide correspondant à l'une des figures 13 ou 15A, à l'exception d'un acide nucléique correspondant à la SÉQ ID N° 75 du WO 01/98353.

2. Acide nucléique selon la revendication 1,
**caractérisé en ce qu'**il contient une séquence correspondant à l'une des figures 9A ou 11A pour le domaine traduit (en lettres capitales).

3. Vecteur d'expression, **caractérisé en ce qu'**il contient une séquence d'ADN selon l'une des revendications 1 ou 2.

4. Cellule hôte, **caractérisée en ce qu'**elle est transformée avec un vecteur d'expression selon la revendication 3.

5. Cellule hôte selon la revendication 4, **caractérisée en ce qu'**il s'agit d'une cellule de mammifère, en particulier d'une cellule humaine.

6. Produit génique purifié, **caractérisé en ce qu'**il est codé par une séquence d'ADN selon l'une des revendications 1 ou 2, à l'exception d'un polypeptide correspondant à la SÉQ ID N° 31 du WO 01/98353.

7. Produit génique purifié selon la revendication 6, **caractérisé en ce qu'**il s'agit d'un polypeptide.

8. Animal transgénique non humain, **caractérisé en ce qu'**il est dépourvu de son polypeptide ee3-1 natif selon la revendication 7 ou de parties de celui-ci.

9. Anticorps, **caractérisé en ce qu'**il reconnaît un épitope d'un produit génique selon l'une des revendications 6 ou 7.

10. Anticorps selon la revendication 9, **caractérisé en ce qu'**il est monoclonal.

11. Anticorps selon l'une des revendications 9 ou 10, **caractérisé en ce qu'**il est dirigé contre un segment de séquence du domaine extracellulaire servant d'épitope.

12. Procédé d'expression de produits géniques selon l'une des revendications 6 ou 7, **caractérisé en ce que** des cellules hôtes sont transformées avec un vecteur d'expression selon la revendication 3.

13. Procédé d'isolement de produits géniques selon l'une des revendications 6 ou 7, **caractérisé en ce que** des cellules hôtes selon l'une des revendications 4 ou 5 sont cultivées dans des conditions adéquates favorisant l'expression et **en ce que** le produit génique est ensuite purifié à partir de la culture.

14. Acide nucléique selon l'une des revendications 1 ou 2 ou produit génique selon l'une des revendications 6 ou 7 en tant que médicament.

15. Acide nucléique selon l'une des revendications 1 ou 2 ou produit génique selon l'une des revendications 6 ou 7 pour le traitement de maladies neurologiques.

16. Procédé d'identification d'un partenaire d'interaction cellulaire d'un polypeptide codé par un acide nucléique selon l'une des revendications 1 ou 2 en utilisant un système dit "double hybride en levure".s
